# EUROPEAN PATENT APPLICATION

(11) **EP 4 847 528 A1**
(43) Date of publication of application: **02.12.2026**
(21) Application number: 25744677.3
(22) Date of filing: 23.01.2025
(51) Int. Cl.: C07K 16/28, C12N 15/13, C12N 5/10, C12N 15/85, A61K 39/395, A61K 38/00, A61P 35/00, G01N 33/574, A61K 47/68

(54) **ANTI-FGFR2B ANTIBODY AND ANTIBODY-DRUG CONJUGATE**

(30) Priority: 26.01.2024 CN 202410115964
(71) Applicant: Shanghai Allink Biotherapeutics Co., Ltd., Shanghai 200240 (CN)
(72) Inventor: PAN, Jiajia, Shanghai 201208 (CN); HE, Hongwang, Shanghai 201208 (CN); LI, Li, Shanghai 201208 (CN); FENG, Hui, Shanghai 201208 (CN)
(74) Representative: Bandpay & Greuter
(86) International application number: PCT/CN2025/074271
(87) International publication number: WO 2025/157214

(57) **Abstract**

The present invention provides an anti-FGFR2b antibody and an antibody-drug conjugate. The antibody of the present invention or an antigen-binding fragment thereof comprises HCDR1 having an amino acid sequence as shown in SEQ ID NO: 25, 1, 9, or 17; HCDR2 having an amino acid sequence as shown in SEQ ID NO: 45, 2, 10, 18, 26, 33, 43, or 44; HCDR3 having an amino acid sequence as shown in SEQ ID NO: 27, 3, 11, or 19; LCDR1 having an amino acid sequence as shown in SEQ ID NO: 51, 5, 13, 21, 29, 37, or 38; LCDR2 having an amino acid sequence as shown in SEQ ID NO: 53, 6, 14, 22, 30, 39, or 52; and LCDR3 having an amino acid sequence as shown in SEQ ID NO: 31, 7, 15, or 23. The antibody-drug conjugate of the present invention has a good effect of inhibiting tumor cell proliferation.

## Description

### TECHNICAL FIELD

The present invention provides antibodies or antigen-binding fragments thereof that specifically bind to FGFR2b, and antibody-drug conjugates thereof.

### BACKGROUND

Gastric cancer is the second most common malignant tumor in China. Gastric cancer, including gastroesophageal junction cancer, ranks fifth in global incidence and third in global mortality. Advanced gastric cancer is treated with sequential chemotherapy, with a platinum-fluoropyrimidine doublet as the first line, but the median survival of patients is less than 1 year. Approved targeted therapies for the treatment of gastric cancer include trastuzumab (for HER2 positive patients, first line), ramucirumab (anti-angiogenic drug, second line), and nivolumab or pembrolizumab (anti-PD-1 antibody, third line). In addition, gastric cancer has high heterogeneity, necessitating precise treatment strategies based on different gastric cancer subtypes.

Fibroblast growth factors (FGFs) and their receptors (FGFRs) play important regulatory roles in embryonic development, tissue homeostasis, and metabolism. FGF family members only bind to four known tyrosine kinase receptors, fibroblast growth factor receptors 1-4 (FGFR1-4) and their isoforms, with various FGFs binding to different FGFRs to varying degrees.

FGFR2, a member of the FGFR family, undergoes alternative splicing at the mRNA level to generate two typical splice isoforms, designated FGFR2-IIIb (FGFR2b) and FGFR2-IIIc (FGFR2c). The immunoglobulin-like domains and intracellular tyrosine kinase domains of FGFR2b and FGFR2c are nearly identical, differing primarily in the latter half of the third immunoglobulin-like domain. Furthermore, they have distinct expression distribution and ligand-binding specificities. Among them, FGFR2b is mainly expressed on the surface of epithelial cells and specifically interacts with FGF-7, FGF-10, and FGF-22, while FGFR2c is mainly expressed in mesenchymal cells and can bind to a variety of FGF ligands, including FGF1, FGF2, FGF4, FGF6, FGF9, FGF17, and FGF18, among others. FGFR2 is hypothesized to play a crucial role in epithelial-mesenchymal transition.

However, underlying FGFR2 overexpression, missense mutation activation, or abnormal protein fusion have been reported in a variety of cancer types, including endometrial cancer, ovarian cancer, breast cancer, lung cancer, gastric cancer, esophageal cancer, bladder cancer, and bile duct cancer, among others. For example, FGFR2b is gene amplified or overexpressed in gastric cancer cells. FGFR2b overexpression accounts for approximately 30% of current gastric cancer/gastroesophageal junction cancer patients, who usually have a poor prognosis and very limited treatment options after progression on first-line chemotherapy. Furthermore, a significant unmet medical need remains for improved anti-cancer drugs effective in FGFR2b-expressing cancers.

Antibody-drug conjugates (ADCs) are a class of targeted therapeutic agents that conjugate cytotoxic small molecules to monoclonal antibodies via chemical linkers. Exploiting the targeting ability of antibodies, they deliver therapeutic agents to antigen-positive cancer cells. FGFR2b is also a potential target for ADCs, but currently, no FGFR2b ADCs are available in this field.

In summary, FGFR2b may be a potential target for tumor treatment. This invention aims to develop novel monoclonal antibody drugs or antibody-drug conjugates targeting FGFR2b, blocking the FGF/FGFR2b signaling pathway for the treatment of cancer patients with FGFR2b overexpression (such as advanced gastric cancer/gastroesophageal junction cancer, etc.), effectively improving the quality of life of cancer patients and prolonging their survival.

### SUMMARY

The purpose of the present application is to provide an antibody capable of specifically binding to FGFR2b (i.e., an anti-FGFR2b antibody) or an antigen-binding fragment thereof of the present application, a polynucleotide molecule encoding the antibody or antigen-binding fragment thereof of the present application, an expression vector and a host cell for expressing the antibody or antigen-binding fragment thereof of the present application, and uses of the antibody or antigen-binding fragment thereof of the present application.

In one aspect, the present invention provides an antibody or an antigen-binding fragment thereof that specifically binds to FGFR2b comprising HCDR1 comprising an amino acid sequence as shown in SEQ ID NO: 1, 9, 17 or 25; HCDR2 comprising an amino acid sequence as shown in SEQ ID NO: 2, 10, 18, 26, 33, 43, 44 or 45; HCDR3 comprising an amino acid sequence as shown in SEQ ID NO: 3, 11, 19 or 27; LCDR1 comprising an amino acid sequence as shown in SEQ ID NO: 5, 13, 21, 29, 37, 38 or 51; LCDR2 comprising an amino acid sequence as shown in SEQ ID NO: 6, 14, 22, 30, 39, 52 or 53; and LCDR3 comprising an amino acid sequence as shown in SEQ ID NO: 7, 15, 23 or 31.

In one aspect, the present invention provides an anti-FGFR2b antibody or an antigen-binding fragment thereof, comprising a heavy chain variable region and/or a light chain variable region.

In another aspect, the present invention provides an isolated anti-FGFR2b antibody or antigen-binding fragment thereof having one or more of the following characteristics:
(I) it binds to an epitope on human FGFR2b protein that is the same as, completely overlapping with, or partially overlapping with the epitope bound by the antibody or antigen-binding fragment described herein;
(II) it competes with the antibody or antigen-binding fragment thereof described herein for binding to an epitope on human FGFR2b protein.

In some embodiments, the antibody described herein is a monoclonal antibody.

In another aspect, the present invention provides a polynucleotide molecule encoding an anti-FGFR2b antibody or antigen-binding fragment thereof described herein.

In another aspect, the present invention provides an expression vector comprising the polynucleotide molecule described herein, preferably, the vector is a eukaryotic expression vector.

In another aspect, the present invention provides a host cell comprising the polynucleotide molecule described herein or the expression vector described herein, preferably, the host cell is a eukaryotic cell, more preferably a mammalian cell.

In another aspect, the present invention provides a method for preparing the anti-FGFR2b antibody or antigen-binding fragment thereof described herein, the method comprises expressing the antibody or antigen-binding fragment thereof in a host cell described herein under conditions suitable for the expression of the antibody or antigen-binding fragment thereof, and recovering the expressed antibody or antigen-binding fragment thereof from the host cell.

The present invention also provides an immunoconjugate comprising the antibody or antigen-binding fragment thereof according to any embodiment of the present invention and a conjugation moiety.

The present invention also provides a pharmaceutical composition comprising the antibody or antigen-binding fragment thereof, polynucleotide molecule, expression vector, host cell or immunoconjugate described in any embodiment herein, and a pharmaceutically acceptable carrier and/or excipient.

In another aspect, the present invention provides use of the antibody or antigen-binding fragments thereof, polynucleotide molecule, expression vector, host cell, immunoconjugate, or pharmaceutical composition described herein in the manufacture of a medicament for treating and/or preventing an FGFR2b-mediated disease or disorder, preferably the disease or disorder is cancer; more preferably, the cancer is selected from endometrial cancer, ovarian cancer, breast cancer, lung cancer, gastric cancer, esophageal cancer, bladder cancer, and bile duct cancer.

The present invention also provides an article comprising a first container, the first container containing a composition comprising the antibody or antigen-binding fragment thereof, nucleic acid molecule, immunoconjugate, or pharmaceutical composition described in any embodiment herein.

In another aspect, the present invention provides a method for detecting the presence of FGFR2b in a sample using the antibody or antigen-binding fragment thereof described herein, or a detection composition containing the antibody or antigen-binding fragment thereof.

Other aspects of the present disclosure will be apparent to those skilled in the art based on the disclosure herein.

### DESCRIPTION OF FIGURES

Figure 1: ELISA results of the anti-FGFR2b chimeric antibody binding to FGFR1c protein (upper panel) and FGFR3c protein (lower panel), respectively.
Figure 2: ELISA results of the anti-FGFR2b chimeric antibody binding to Cyno FGFR2b protein.
Figure 3: ELISA results of the anti-FGFR2b chimeric antibody binding to mouse FGFR2b protein.
Figure 4: Results of blocking activity assay of the anti-FGFR2b chimeric antibody (FGF7/FGFR2b).
Figure 5: Results of blocking activity assay of the anti-FGFR2b chimeric antibody (FGF10/FGFR2b).
Figure 6: Results of ADCC activity assay of anti-FGFR2b chimeric antibody.
Figure 7: Results of cell proliferation inhibition activity assay of the anti-FGFR2b chimeric antibody.
Figure 8: Results of ERK phosphorylation inhibition activity assay of the anti-FGFR2b chimeric antibody.
Figure 9: Results of cell binding activity assay of the anti-FGFR2b humanized antibody. Upper panel: hu-032; lower panel: hu-042.
Figure 10: Results of ADCC activity assay of the anti-FGFR2b humanized antibody. Upper panel: hu-032; lower panel: hu-042.
Figure 11: Results of cell proliferation inhibition activity assay of the anti-FGFR2b humanized antibody. Upper panel: hu-032; lower panel: hu-042.
Figure 12: Results of ERK phosphorylation inhibition activity assay of the anti-FGFR2b humanized antibody.
Figure 13: Results of internalization activity assay of anti-FGFR2b ADC.
Figure 14: Results of in vitro killing activity assay of anti-FGFR2b ADC.
Figure 15: Results of cell killing assay of different linker-toxin conjugate.
Figure 16: Results of cell killing assay of hu-042-016 ADC.
Figure 17: Results of bystander killing activity of anti-FGFR2b ADC.
Figure 18: Results of in vivo efficacy assay of anti-FGFR2b ADC with different linkers.
Figure 19: Results of in vivo efficacy assay of anti-FGFR2b ADC (DAR4).
Figure 20: Results of in vivo efficacy study of anti-FGFR2b antibody-drug conjugate on a human gastric cancer subcutaneous xenograft.
Figure 21: Results of in vivo efficacy study of anti-FGFR2b antibody-drug conjugate on a human breast cancer subcutaneous xenograft.
Figure 22: Results of in vivo efficacy study of anti-FGFR2b antibody-drug conjugate on a human lung cancer subcutaneous xenograft.

### DETAILED DESCRIPTION

The present invention provides an antibody or functional fragment thereof that specifically binds to FGFR2b, an antibody-drug conjugate (ADC) or a pharmaceutical composition thereof, the use of the antibody, functional fragment, pharmaceutical composition, or ADC in the treatment and/or prevention of an FGFR2b-mediated disease, and a method for treating or preventing an FGFR2b-mediated disease using the antibody, functional fragment, pharmaceutical composition, or ADC.

It should be understood that within the scope of the present invention, the above-mentioned technical features of the present invention and the technical features specifically described below (such as the Examples) can be combined with each other to form a new technical solution.

### Definitions

The practice of the present description will employ, unless otherwise indicated, conventional techniques of molecular biology (including recombinant techniques), microbiology, cell biology, biochemistry and immunology, which are within the skill of the art.

To facilitate a better understanding of the present invention, certain scientific and technical terms are specifically defined below. Unless otherwise clearly defined elsewhere herein, scientific and technical terms used herein have the meanings commonly understood by those of ordinary skill in the art to which this invention belongs. For definitions and terms in the art, professionals may refer specifically to Current Protocols in Molecular Biology (Ausubel). The abbreviations for amino acid residues are standard 3-letter and/or 1-letter codes used in the art to refer to one of the 20 commonly used L-amino acids. The singular forms used herein (including the claims) include their corresponding plural forms, unless the context clearly dictates otherwise.

The term "about" when used in conjunction with a numerical value refers to encompass the numerical value within a range having a lower limit of 5% less than the specified numerical value and an upper limit of 5% greater than the specified numerical value, including but not limited to ±5%, ±2%, ±1%, and ±0.1%, as such variations are appropriate for performing the disclosed methods.

The term "and/or" should be understood to mean any one of the alternatives or a combination of any two or more of the alternatives.

As used herein, the term "or" should be understood to have the same meaning as "and/or" as defined above. For example, when separating items in a list, "or" or "and/or" should be interpreted as inclusive, i.e., including at least one of the numbers or elements in the list, but also including more than one, and optionally, additional unlisted items. Only when explicitly stated to the contrary, such as "only one" or "exactly one" or when used in a claim, "consisting of..." will refer to only one of the listed numbers or one of the elements of the list.

Unless expressly stated otherwise, as used herein, the words "a" and "an" should be understood to mean "at least one."

"FGFR2IIIb" or "FGFR2b" are used interchangeably to refer to the splice form of fibroblast growth factor receptor 2IIIb. Exemplary FGFR2b sequences include the Homo sapiens (human) FGFR2b protein (e.g., precursor sequence with signal peptide, Genbank Accession No.: NP_075259.4); Rattus norvegicus (rat) FGFR2b protein (e.g., full sequence, Genbank accession number: NP_001103363.1); and Mus musculus (mouse) FGFR2b protein (e.g., full sequence, Genbank accession number: NP_963895.2).

"FGFR2IIIc" or "FGFR2c" are used interchangeably to refer to the splice form of fibroblast growth factor receptor 2IIIc. Exemplary FGFR2c sequences include human FGFR2c protein (e.g., precursor sequence, Genbank accession number: NP_000132.3); Rattus norvegicus (rat) FGFR2c protein (full sequence, Genbank accession number: NP_001103362.1); and Mus musculus (mouse) FGFR2c protein (full sequence, Genbank accession number: NP_034337.2).

The term "percent (%) amino acid sequence identity," or simply "identity," is defined as the percentage of amino acid residues in a candidate amino acid sequence that are identical to the amino acid residues in a reference amino acid sequence, after aligning the amino acid sequences (and introducing gaps, if necessary) to achieve maximum percent sequence identity, and not considering any conservative substitutions as part of the sequence identity. Various methods in the art can be used for sequence alignment to determine percent amino acid sequence identity, for example, using publicly available computer software such as BLAST, BLAST-2, ALIGN, or MEGALIGN (DNASTAR) software. Those skilled in the art can determine appropriate parameters for measuring alignment, including any algorithms needed to achieve maximal alignment over the full length of the sequences being compared.

The term "immune response" refers to the actions of, for example, lymphocytes, antigen-presenting cells, phagocytes, granulocytes, and soluble macromolecules (including antibodies, cytokines, and complement) produced by the above cells or the liver, which result in the selective damage, destruction, or elimination from the body of invading pathogens, cells or tissues infected with pathogens, cancer cells, or, in the case of autoimmunity or pathological inflammation, normal human cells or tissues.

The term "signal transduction pathway" or "signal transduction activity" refers to a biochemical causality, typically initiated by protein-protein interactions such as the binding of a growth factor to a receptor, that results in the transmission of a signal from one part of a cell to another part. Generally, transmission involves the specific phosphorylation of one or more tyrosine, serine, or threonine residues on one or more proteins in a series of reactions that lead to signal transduction. The penultimate process typically includes nuclear events, leading to changes in gene expression.

The term "activity" or "biological activity", or the term "biological property" or "biological characteristic" is used interchangeably herein and includes but is not limited to epitope/antigen affinity and specificity, the ability to neutralize or antagonize FGFR2b activity in vivo or in vitro, IC50 , the in vivo stability of the antibody and the immunogenic properties of the antibody. Other identifiable biological properties or characteristics of antibodies known in the art include, for example, cross-reactivity (i.e., generally with non-human homologs of the target peptide, or with other proteins or tissues), and the ability to maintain high expression levels of a protein in mammalian cells. The aforementioned properties or characteristics are observed, measured, or assessed using techniques known in the art, including but not limited to ELISA, FACS, or BIACORE surface plasmon resonance analysis, unrestricted in vitro or in vivo neutralization assays, receptor binding, the generation and/or secretion of cytokines or growth factors, signal transduction, and immunohistochemistry of tissue sections from different sources (including human, primate, or any other source).

The term "antibody" refers to any form of antibody having the desired biological activity. Therefore, it is used in the broadest sense and specifically includes, but is not limited to, a monoclonal antibody (including a full-length monoclonal antibody), a polyclonal antibody, a multispecific antibody (e.g., a bispecific antibody), a humanized antibody, a fully human antibody, a chimeric antibody, and a camelized single domain antibody.

The term "isolated antibody" refers to the purified state of the binding compound, and in this case means that the molecule is substantially free of other biomolecules, such as nucleic acids, proteins, lipids, sugars, or other substances such as cell debris and growth medium. The term "isolated" does not imply the complete absence of such substances or the absence of water, buffers, or salts unless they are present in amounts that significantly interfere with experimental or therapeutic applications of the binding compounds described herein.

The term "monoclonal antibody" refers to an antibody obtained from substantially homogeneous antibody populations, i.e., the individual antibodies comprising the population are identical except for possible naturally occurring mutations that may be present in minor amounts. Monoclonal antibodies are highly specific, being directed against a single antigenic epitope. In contrast, conventional (polyclonal) antibody preparations typically include a large number of antibodies directed against (or specific for) different epitopes. The modifier "monoclonal" indicates the character of the antibody as being obtained from substantially homogeneous antibody populations, and is not to be construed as requiring production of the antibody by any particular method.

The term "full-length antibody" refers to an immunoglobulin molecule that, when naturally occurring, comprises at least four peptide chains: two heavy (H) chains and two light (L) chains interconnected by disulfide bonds. Each heavy chain is composed of a heavy chain variable region (abbreviated herein as VH) and a heavy chain constant region (abbreviated herein as CH). The heavy chain constant region is composed of three domains, CH1, CH2, and CH3. Each light chain is composed of a light chain variable region (abbreviated herein as VL) and a light chain constant region. The light chain constant region is composed of one domain: CL. The VH and VL regions can be further subdivided into complementarity determining regions (CDRs) with high variability, and, interspersed with the CDRs, more conserved framework regions (FRs). Each VH or VL region is composed of three CDRs and four FRs, arranged from amino-terminus to carboxyl-terminus in the following order: FR1, CDR1, FR2, CDR2, FR3, CDR3, FR4. The variable regions of the heavy and light chains contain the binding domain that interacts with the antigen. The constant region of an antibody may mediate the binding of the immunoglobulin to host tissues or factors, including various cells of the immune system (e.g., effector cells) and the first component (Clq) of the classical complement system.

The term "antigen-binding fragment" of an antibody ("parent antibody") includes fragments or derivatives of an antibody, typically including at least one fragment of the antigen-binding region or variable region (e.g., one or more CDRs) of the parent antibody, which retains at least some of the binding specificity of the parent antibody. Examples of an antibody-binding fragment include, but are not limited to, Fab, Fab', F(ab')2, and Fv fragments; diabodies; linear antibodies; single chain antibodies such as sc-Fv; nanobodies; and multispecific antibodies formed from antibody fragments. When the antigen-binding activity is expressed on a molar basis, the binding fragment or derivative typically retains at least 10% of its antigen-binding activity. Preferably, the binding fragment or derivative retains at least 20%, 50%, 70%, 80%, 90%, 95%, or 100% or more of the antigen-binding affinity of the parent antibody. It is also contemplated that the antigen-binding fragment of an antibody may include conservative or non-conservative amino acid substitutions that do not significantly change its biological activity (referred to as "conservative variants" or "functional conservative variants" of the antibody). The term "binding compound" refers to both an antibody and a binding fragment thereof.

The term "single-chain Fv" or "scFv" antibody refers to an antibody fragment comprising the VH and VL domains of an antibody, wherein these domains are present in a single polypeptide chain. The Fv polypeptide generally further comprises a polypeptide linker between the VH and VL domains that enables the scFv to form the desired structure for antigen binding.

The term "domain antibody" refers to an immunologically functional immunoglobulin fragment containing only a heavy chain variable region or a light chain variable region. In some cases, two or more VH regions are covalently linked with a peptide linker to form a bivalent domain antibody. The two VH regions of a bivalent domain antibody can target the same or different antigens.

The term "bivalent antibody" comprises two antigen-binding sites. In some cases, the two binding sites have the same antigen specificity. However, a bivalent antibody can be bispecific.

The term "diabody" refers to a small antibody fragment having two antigen-binding sites, the fragment comprising a heavy chain variable domain (VH) linked to a light chain variable domain (VL) in the same polypeptide chain (VH-VL or VL-VH). By using a linker that is too short to allow pairing between the two domains on the same chain, the domains are forced to pair with the complementary domains of another chain and generate two antigen-binding sites.

The term "murine antibody" or "hybridoma antibody" as used herein refers to a monoclonal antibody against human FGFR2b prepared according to the knowledge and skills in the art. During preparation, the test subject is injected with a FGFR2b antigen, and then the hybridoma expressed with the desired sequence or functional properties is separated.

The term "chimeric antibody" refers to an antibody having the variable domain of a first antibody and the constant domain of a second antibody, wherein the first antibody and the second antibody are from different species. Typically, the variable domain is obtained from an antibody of a rodent or the like (the "parent antibody"), while the constant domain sequence is obtained from a human antibody, such that the resulting chimeric antibody is less likely to induce an adverse immune response in a human subject compared to the parent rodent antibody.

The term "humanized antibody" refers to a form of antibody that contains sequences from both human and non-human (e.g., mouse, rat) antibodies. Generally, a humanized antibody comprises substantially all of at least one, and usually two, variable domains, in which all or substantially all of the hypervariable loops correspond to those of a non-human immunoglobulin, and all or substantially all of the framework (FR) regions are those of a human immunoglobulin sequence. The humanized antibody optionally may comprise at least a portion of a human immunoglobulin constant region (Fc).

The term "fully human antibody" refers to an antibody that contains only the human immunoglobulin protein sequence. If produced in a mouse, in mouse cells, or in a hybridoma derived from mouse cells, the fully human antibody may contain murine sugar glycans. Similarly, "mouse antibody" refers to an antibody that comprises only mouse immunoglobulin sequences. Alternatively, if produced in a rat, in rat cells, or in a hybridoma derived from rat cells, the fully human antibody may contain rat sugar glycans. Similarly, "rat antibody" refers to an antibody that comprises only rat immunoglobulin sequences.

An "isotype" antibody refers to an antibody class provided by the heavy chain constant region genes (e.g., IgM, IgE, IgG such as IgG1, IgG2, or IgG4). An isotype also includes a modified form of one of these classes, where modifications have been made to alter Fc function, for example, to enhance or diminish effector function or binding to Fc receptors.

The term "Fc region" as used herein is used to define the C-terminal region of an immunoglobulin heavy chain that comprises at least a portion of the constant region. The term includes a native sequence Fc region and a variant Fc region. In some embodiments, a human IgG heavy chain Fc region extends from Cys226 or Pro230 to the carboxyl-terminus of the heavy chain. However, the C-terminal lysine (Lys447) of the Fc region may or may not be present (numbering in this paragraph is according to the EU numbering system, also known as the EU index, as described in Kabat et al., Sequences of Proteins of Immunological Interest, 5th Ed. Public Health Service, National Institutes of Health, Bethesda, MD, 1991).

The term "epitope" refers to a protein determinant capable of specifically binding to an antibody. Epitopes usually consist of chemically active surface molecules such as amino acids or sugar side chains, and usually have specific three-dimensional structural characteristics, as well as specific charge characteristics. Conformational and non-conformational epitopes are distinguished in that binding to the former but not the latter is lost in the presence of denaturing solvents.

The term "cross-reactivity" as described herein refers to binding to antigenic fragments of the same target molecule from human, monkey, and/or murine (mouse or rat). Thus, "cross-reactivity" should be understood as an interspecies reaction with the same molecule X expressed in different species. The cross-reactivity specificity of a monoclonal antibody that recognizes human FGFR2b, monkey, and/or mouse FGFR2b (mouse or rat) can be determined by FACS analysis.

"Affinity" or "binding affinity" refers to the intrinsic binding affinity that reflects the interaction between members of a binding pair. The affinity of molecule X for its partner Y can generally be represented by the equilibrium dissociation constant (K_{D}), which is the ratio of the dissociation rate constant and the association rate constant (k_{dis} and kₒₙ, respectively). Affinity can be measured by common methods known in the art. One specific method for measuring affinity is the ForteBio kinetic binding assay herein.

The term "not binding" to a protein or cell means not binding to the protein or cell, or not binding to the protein or cell with high affinity, that is, the K_{D} for binding to the protein or cell is 1.0×10⁻⁶ M or higher, more preferably 1.0×10⁻⁵ M or higher, even more preferably 1.0×10⁻⁴ M or higher, 1.0×10⁻³ M or higher, more preferably 1.0×10⁻² M or higher.

The term "high affinity" for an IgG antibody refers to a K_{D} for the antigen of 1.0×10⁻⁶ M or lower, preferably 5.0×10⁻⁸ M or lower, more preferably 1.0×10⁻⁸ M or lower, 5.0×10⁻⁹ M or lower, even more preferably 1.0×10⁻⁹ M or lower. For other antibody subtypes, "high affinity" binding may vary. For example, "high affinity" binding for an IgM subtype refers to a K_{D} of 10⁻⁶ M or lower, preferably 10⁻⁷ M or lower, more preferably 10⁻⁸ M or lower.

"Competitive binding" ability refers to the ability of an antibody or antigen-binding fragment to inhibit the binding interaction between two molecules (e.g., human FGFR2b and an anti-FGFR2b antibody) to any detectable degree (e.g., inhibition of at least 85%, or at least 90%, or at least 95%).

The term "antibody-dependent cellular cytotoxicity", "antibody-dependent cell-mediated cytotoxicity", or "ADCC" refers to a cell-mediated immune defense in which effector cells of the immune system actively bind antibodies to cell membrane surface antigens.

The term "complement-dependent cytotoxicity" or "CDC" refers to the effector function of IgG and IgM antibodies that, upon binding to a surface antigen, trigger the classical complement pathway, including the formation of the membrane attack complex and lysis of the target cell.

The terms "nucleic acid", "polynucleotide", "nucleic acid molecule", and "polynucleotide molecule" refer to deoxyribonucleic acid (DNA) or ribonucleic acid (RNA) and polymers thereof in either single-stranded or double-stranded form. Unless expressly limited, the terms include nucleic acids containing known analogs of natural nucleotides that have similar binding properties as the reference nucleic acid and are metabolized in a manner similar to naturally occurring nucleotides (see, e.g., U.S. Patent No. 8,278,036 to Kariko et al., which discloses mRNA molecules in which uridine is replaced by pseudouridine, methods for synthesizing the mRNA molecules, and methods for delivering therapeutic proteins in vivo). Unless otherwise indicated, a particular nucleic acid sequence also implicitly includes conservatively modified variants thereof (e.g., degenerate codon substitutions), alleles, orthologs, SNPs, and complementary sequences, as well as the sequences explicitly indicated. Specifically, degenerate codon substitutions may be achieved by generating sequences in which the third position of one or more selected (or all) codons is substituted with mixed bases and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

"Construct" refers to any recombinant polynucleotide molecule (such as a plasmid, cosmid, virus, autonomously replicating polynucleotide molecule, bacteriophage, or linear or circular single-stranded or double-stranded DNA or RNA polynucleotide molecule), derived from any source, capable of integrating with a genome or autonomously replicating, constituting a polynucleotide molecule in which one or more polynucleotide molecules have been linked (i.e., operably linked) in a functionally operable manner. A recombinant construct will typically contain a polynucleotide of the invention operably linked to a transcription initiation regulatory sequence, which directs transcription of the polynucleotide in a host cell. Both heterologous and non-heterologous (i.e., endogenous) promoters can be used to direct expression of the nucleic acids of the invention.

"Vector" refers to any recombinant polynucleotide construct that can be used for the purpose of transformation (i.e., introducing heterologous DNA into a host cell). One type of vector is a "plasmid", which refers to a circular double-stranded DNA loop into which additional DNA segments can be connected. Another type of vector is a viral vector, wherein additional DNA segments can be connected into the viral genome. Certain vectors are capable of autonomous replication in the host cell into which they are introduced (e.g., bacterial vectors and episomal mammalian vectors with a bacterial origin of replication). After being introduced into the host cell, other vectors (e.g., a non-episomal mammalian vector) are integrated into the genome of the host cell upon introduction into the host cell, and thereby are replicated along with the host genome. Moreover, certain vectors are capable of directing the expression of genes to which they are operably linked. Such vectors are referred to herein as "expression vectors".

As used herein, the term "expression vector" refers to a nucleic acid molecule capable of replicating and expressing a gene of interest when transformed, transfected, or transduced into a host cell. An expression vector contains one or more phenotypic selection markers and an origin of replication to ensure maintenance of the vector and, if desired, to provide amplification within the host.

The term "immunoconjugate" refers to an antibody that is connected to a portion having a desired activity through a linker or directly. The connection can be a covalent connection or a noncovalent connection. The desired activity can be, for example, cell killing activity or detection (such as luminescence) activity. The portion having the desired activity can be an anti-tumor agent, an immunomodulator, a cytotoxic drug, a fluorescent substance, a luminescent substance, an enzyme, a small nucleic acid molecule, and any combination thereof. Suitable small nucleic acids may include siRNA or antisense oligonucleotides (ASOs), etc. In some embodiments, the immunoconjugate is an antibody-drug conjugate (ADC) as described in any embodiment herein, or an antibody or antigen-binding fragment thereof labeled with a radioactive atom or spin label as described in any embodiment herein.

The terms "antibody-drug conjugate", "antibody-drug conjugate", and "ADC" are used interchangeably and generally refer to one or more therapeutic compounds (e.g., Dxd) linked to one or more antibodies or antigen-binding fragments and are defined by the following general formula: Ab-(L-D)(s), wherein Ab = antibody or antigen-binding fragment, L = linker, D = drug unit, and s = number of drug moieties per antibody or antigen-binding fragment, which can be any number from 1 to 10. In some embodiments, the linker L may include a cleavable moiety between the antibody or antigen-binding fragment and the drug unit.

The term "drug" in the present invention generally refers to any compound having a desired biological activity and having a reactive functional group for preparing the conjugates described herein. The desired biological activity includes the diagnosis, cure, alleviation, treatment, or prevention of human or other animal diseases. As new drugs are continuously discovered and developed, these new drugs should also be included in the drugs described herein. Specifically, the drugs include but are not limited to a cytotoxic drug, a cell differentiation factor, a stem cell trophic factor, a steroid, a drug for treating an autoimmune disease, an anti-inflammatory drug, or a drug for an infectious disease. More specifically, the drugs include but are not limited to a microtubule inhibitor or a DNA/RNA damaging agent.

The term "cytotoxic drug" or "cytotoxin" refers to a substance that inhibits cellular function and/or causes cell death or destruction. In principle, a cytotoxic drug can kill tumor cells at sufficiently high concentrations, but due to lack of selectivity, it also causes apoptosis of normal cells while killing tumor cells, leading to severe side effects. Cytotoxic drugs include toxins, such as small molecule toxins or enzymatically active toxins of bacterial, fungal, plant, or animal origin, radioisotopes, chemotherapeutic drugs, antibiotics, and nucleolytic enzymes, among others.

A "microtubule inhibitor" refers to a class of compounds that exert an anti-tumor effect by interfering with the mitotic process of a cell, either by inhibiting tubulin polymerization or promoting tubulin assembly. Non-limiting examples thereof include: maytansinoids, calicheamicin, taxanes, vincristine, colchicine, dolastatin/auristatin/monomethyl auristatin E (MMAE)/monomethyl auristatin F (MMAF).

In some embodiments, the microtubule inhibitors include but are not limited to dolastatins and auristatins, and maytansines; the DNA damaging agents include but are not limited to calicheamicins, duocarmycins, the anthramycin derivative PBD (pyrrolobenzodiazepine), the camptothecin derivative SN-38, and topoisomerase I inhibitors.

In some embodiments, the auristatin drugs include but are not limited to: monomethyl auristatin E (MMAE), monomethyl auristatin F (MMAF), and auristatin F (AF) or derivatives thereof; the maytansine drugs include but are not limited to DM1, DM3, DM4 or derivatives thereof ("Progress in warhead research of antibody-drug conjugates", Hu Xinyue et al., China Medical Biotechnology, December 2017, Vol. 12, No. 6; "Research progress of maytansinoid antibody-drug conjugates", Zhou Lei et al., Chinese Journal of New Drugs, 2016, Vol. 25, No. 22, pp. 2521-2530); the topoisomerase I inhibitors include but are not limited to exatecan, topotecan, irinotecan, and 9-nitrocamptothecin.

In the present invention, the term "linker" or "L" generally refers to any chemical moiety capable of covalently joining a compound (usually a drug unit) to another moiety, such as an antibody or antigen-binding fragment. The linker may be susceptible to or substantially resistant to acid-induced cleavage, peptidase-induced cleavage, light-based cleavage, esterase-induced cleavage, and/or disulfide bond cleavage under conditions that maintain the activity of the compound or antibody.

According to the properties, the linkers suitable for the present invention can be linkers that can be cleaved by tissue proteases, such as valine-citrulline (val-cit) linkers, cBu-Cit linkers and CX linkers; non-cleavable linkers such as SMCC linkers or MD linkers; MC-GGFG linkers, TRX linkers, galactoside-containing linkers, etc.

In specific embodiments, a linker includes but is not limited to maleimidocaproyl-valine-citrulline-p-aminobenzyloxy (mc-vc-PAB), acetyl-lysine-valine-citrulline-p-aminobenzyloxycarbonyl (AcLys-VC-PABC), aminoPEG6-propionyl, and maleimidocaproic acid (MC), maleimidopropionyl (MP), valine-citrulline (val-cit, vc), alanine-phenylalanine (ala-phe), p-aminobenzyloxycarbonyl (PAB), N-succinimidyl 4-(2-pyridylthio)pentanoate (SPP), N-succinimidyl 4-(N-maleimidomethyl)-cyclohexane-1-carboxylate (SMCC), N-succinimidyl (4-iodo-acetyl)aminobenzoate (SIAB), N-succinimidyl-4-(2-pyridyldithio)butyrate (SPDB), and N-succinimidyl 3-(pyridin-2-yldithio)-propionate (SPDP).

The term "load" or "drug load" or "payload" refers to the average number of payloads per antibody within an ADC molecule (herein, "payload" is interchangeable with "therapeutically active substance or active pharmaceutical ingredient"). The drug load can range from 1 to 20 therapeutically active substances or active pharmaceutical ingredients per antibody.

The term "drug/antibody ratio" or "DAR" refers to the ratio of therapeutically active substance or active pharmaceutical ingredient (D) conjugated to the antibody relative to the antibody. The ADCs described herein typically have a DAR of 1-10, and in certain specific embodiments have a DAR of 1-8, 2-8, 2-6, 2-5, 3-8, 4-6, and 2-4. Representative DAR values are 2, 3, 4, 5, 6, 7, 8, 9, 10, often expressed as a combination of the letter D and a number, where the number indicates the DAR value, e.g., D2 indicates a drug/antibody ratio with a DAR value of 2. In some embodiments, DAR is the average DAR, i.e., characterized by a detection method (e.g., by conventional methods such as UV/visible spectroscopy, mass spectrometry, ELISA, and HPLC). Quantitative DAR values can also be determined. The DAR may be limited by the number of attachment sites on the antibody. For example, when the attachment site is a cysteine thiol, the antibody may have only one or a few cysteine thiol groups or may have only one or a few thiol groups that are sufficiently reactive (through which the linking unit can be attached).

"Activation", "stimulation", and "treatment" as applied to a cell or receptor may have the same meaning, e.g., a cell or receptor is activated, stimulated, or treated with a ligand, unless the context dictates otherwise or is expressly specified otherwise. "Ligand" includes natural and synthetic ligands, e.g., cytokines, cytokine variants, analogs, muteins, and binding compounds derived from antibodies. "Ligand" also includes small molecules, e.g., peptide mimetics of cytokines and peptide mimetics of antibodies. "Activation" can refer to cell activation regulated by internal mechanisms as well as external or environmental factors. "Response", e.g., a response of a cell, tissue, organ, or organism, includes a change in biochemical or physiological behavior (e.g., concentration, density, adhesion, or migration within a biological compartment, rate of gene expression, or differentiation state), wherein the change is associated with activation, stimulation, or treatment, or with internal mechanisms such as genetic programming.

As used herein, the term "treatment" or "treating" of any disease or disorder in one embodiment refers to ameliorating the disease or disorder (i.e., slowing or arresting or reducing the progression of the disease or at least one of its clinical symptoms). In another embodiment, "treatment" or "treating" refers to alleviating or ameliorating at least one physical parameter, including those physical parameters that may not be discernible by the patient. In another embodiment, "treatment" or "therapy" refers to modulating the disease or disorder either physically (e.g., stabilization of a discernible symptom), physiologically (e.g., stabilization of a physical parameter), or both. Unless expressly described herein, methods for assessing the treatment and/or prevention of a disease are generally known in the art.

Subject" includes any human or non-human animal. The term "non-human animal" includes all vertebrates, e.g., mammals and non-mammals, such as non-human primates, sheep, dogs, cats, horses, cows, chickens, amphibians, reptiles, etc. As used herein, the term "cyno" or "cynomolgus monkey" refers to a cynomolgus monkey.

Administration "in combination with" one or more other therapeutic agents includes simultaneous (concurrent) administration and sequential administration in any order.

"Therapeutically effective amount," "therapeutically effective dose," and "effective amount" refer to an amount of the FGFR2b antibody or antigen-binding fragment thereof of the present invention, when administered alone or in combination with other therapeutic agents to a cell, tissue, or subject, that is effective to prevent or ameliorate the symptoms of one or more diseases or conditions or the progression of the disease or condition. A therapeutically effective dose also refers to an amount of an antibody or antigen-binding fragment thereof sufficient to result in an improvement in symptoms, e.g., an amount to treat, cure, prevent, or ameliorate a related medical condition or to increase the rate of treatment, cure, prevention, or amelioration of such a condition. When a single active ingredient is administered to an individual, a therapeutically effective dose refers only to that ingredient. When administered in combination, a therapeutically effective dose refers to the combined amount of the active ingredients that results in a therapeutic effect, whether administered in combination, sequentially, or simultaneously. An effective amount of a therapeutic agent will result in an improvement in a diagnostic criterion or parameter by at least 10%, typically by at least 20%, preferably by at least about 30%, more preferably by at least 40%, and most preferably by at least 50%.

"Pharmaceutically acceptable carrier" refers to a component of a pharmaceutical preparation or composition other than the active ingredient that is non-toxic to the subject. Pharmaceutically acceptable carriers include, but are not limited to, buffers, excipients, stabilizers, or preservatives. Pharmaceutically acceptable carriers comprise, but are not limited to, buffers, excipients, stabilizers or preservatives.

The term "cancer" is used herein to refer to a group of cells that exhibit an abnormally high level of proliferation and growth. Cancer may be benign (also known as a benign tumor), pre-malignant, or malignant. Cancer cells may be solid cancer cells or leukemia cancer cells. The term "tumor" as used herein refers to one or more cells comprising a cancer. The term "tumor growth" is used herein to refer to the proliferation or growth of one or more cells comprising a cancer, which results in a corresponding increase in the size or extent of the cancer.

### Anti-FGFR2b Antibody

In one aspect, the present invention provides an antibody or an antigen-binding fragment thereof that specifically binds to FGFR2b. The terms "anti-FGFR2b antibody", "anti-FGFR2b", "FGFR2b antibody", or "antibody that binds FGFR2b" refer to an antibody that is capable of binding to FGFR2b protein or a fragment thereof with sufficient affinity so that the antibody can be used as a diagnostic agent and/or a therapeutic agent targeting FGFR2b.

In some embodiments, the present invention provides an antibody that binds to FGFR2b protein. In some embodiments, the present invention provides an antibody that blocks the FGF/FGFR2b signaling pathway.

In some embodiments, an antibody of the present invention binds to human or cynomolgus monkey FGFR2b protein. In some embodiments, an antibody of the present invention binds to human FGFR2b and blocks the interaction between human FGFR2b and FGF protein.

In some embodiments, the amino acid sequence of HCDR1 comprised by the antibody or antigen-binding fragment thereof that specifically binds to FGFR2b of the present invention is selected from the amino acid sequence shown in SEQ ID NO: 1, 9, 17 or 25, or has 1, 2 or 3 amino acid differences from the amino acid sequence shown in SEQ ID NO: 1, 9, 17 or 25. In some embodiments, the amino acid sequence of HCDR1 comprised by the antibody or antigen-binding fragment thereof that specifically binds to FGFR2b of the present invention is selected from the amino acid sequence shown in SEQ ID NO: 17 or 25, or has 1, 2 or 3 amino acid differences from the amino acid sequence shown in SEQ ID NO: 17 or 25.

In some embodiments, the amino acid sequence of HCDR2 comprised by the antibody or antigen-binding fragment thereof that specifically binds to FGFR2b of the present invention is selected from the amino acid sequence shown in SEQ ID NO: 2, 10, 18, 26, 33, 43, 44 or 45, or has 1, 2 or 3 amino acid differences from the amino acid sequence shown in SEQ ID NO: 2, 10, 18, 26, 33, 43, 44 or 45. In some embodiments, the amino acid sequence of HCDR2 comprised by the antibody or antigen-binding fragment thereof that specifically binds to FGFR2b of the present invention is selected from the amino acid sequence shown in SEQ ID NO: 18, 26, 33, 43, 44 or 45, or has 1, 2 or 3 amino acid differences from the amino acid sequence shown in SEQ ID NO: 18, 26, 33, 43, 44 or 45.

In some embodiments, the amino acid sequence of HCDR3 comprised by the antibody or antigen-binding fragment thereof that specifically binds to FGFR2b of the present invention is selected from the amino acid sequence shown in SEQ ID NO: 3, 11, 19 or 27, or has 1, 2 or 3 amino acid differences from the amino acid sequence shown in SEQ ID NO: 3, 11, 19 or 27. In some embodiments, the amino acid sequence of HCDR3 comprised by the antibody or antigen-binding fragment thereof that specifically binds to FGFR2b of the present invention is selected from the amino acid sequence shown in SEQ ID NO: 19 or 27, or has 1, 2 or 3 amino acid differences from the amino acid sequence shown in SEQ ID NO: 19 or 27.

In some embodiments, the amino acid sequence of LCDR1 comprised by the antibody or antigen-binding fragment thereof that specifically binds to FGFR2b of the present invention is selected from the amino acid sequence shown in SEQ ID NO: 5, 13, 21, 29, 37, 38 or 51, or has 1, 2 or 3 amino acid differences from the amino acid sequence shown in SEQ ID NO: 5, 13, 21, 29, 37, 38 or 51. In some embodiments, the amino acid sequence of LCDR1 comprised by the antibody or antigen-binding fragment thereof that specifically binds to FGFR2b of the present invention is selected from the amino acid sequence shown in SEQ ID NO: 21, 29, 37, 38 or 51, or has 1, 2 or 3 amino acid differences from the amino acid sequence shown in SEQ ID NO: 21, 29, 37, 38 or 51.

In some embodiments, the amino acid sequence of LCDR2 comprised by the antibody or antigen-binding fragment thereof that specifically binds to FGFR2b of the present invention is selected from the amino acid sequence shown in SEQ ID NO: 6, 14, 22, 30, 39, 52, or 53, or has 1, 2 or 3 amino acid differences from the amino acid sequence shown in SEQ ID NO: 6, 14, 22, 30, 39, 52, or 53. In some embodiments, the amino acid sequence of LCDR2 comprised by the antibody or antigen-binding fragment thereof that specifically binds to FGFR2b of the present invention is selected from the amino acid sequence shown in SEQ ID NO: 39, 52, or 53, or has 1, 2 or 3 amino acid differences from the amino acid sequence shown in SEQ ID NO: 39, 52, or 53.

In some embodiments, the amino acid sequence of LCDR3 comprised by the antibody or antigen-binding fragment thereof that specifically binds to FGFR2b of the present invention is selected from the amino acid sequence shown in SEQ ID NO: 7, 15, 23 or 31, or has 1, 2 or 3 amino acid differences from the amino acid sequence shown in SEQ ID NO: 7, 15, 23 or 31. In some embodiments, the amino acid sequence of LCDR3 comprised by the antibody or antigen-binding fragment thereof that specifically binds to FGFR2b of the present invention is selected from the amino acid sequence shown in SEQ ID NO: 23 or 31, or has 1, 2 or 3 amino acid differences from the amino acid sequence shown in SEQ ID NO: 23 or 31.

In some embodiments, the antibody or antigen-binding fragment thereof that specifically binds to FGFR2b of the present invention comprises: HCDR1 having an amino acid sequence as shown in SEQ ID NO: 1, 9, 17 or 25, or having 1, 2, or 3 amino acid differences from the amino acid sequence shown in SEQ ID NO: 1, 9, 17 or 25; HCDR2 having an amino acid sequence as shown in SEQ ID NO: 2, 10, 18, 26, 33, 43, 44 or 45, or having 1, 2, or 3 amino acid differences from the amino acid sequence shown in SEQ ID NO: 2, 10, 18, 26, 33, 43, 44 or 45; HCDR3 having an amino acid sequence as shown in SEQ ID NO: 3, 11, 19 or 27, or having 1, 2, or 3 amino acid differences from the amino acid sequence shown in SEQ ID NO: 3, 11, 19 or 27; LCDR1 having an amino acid sequence as shown in SEQ ID NO: 5, 13, 21, 29, 37, 38 or 51, or having 1, 2, or 3 amino acid differences from the amino acid sequence shown in SEQ ID NO: 5, 13, 21, 29, 37, 38 or 51; LCDR2 having an amino acid sequence as shown in SEQ ID NO: 6, 14, 22, 30, 39, 52 or 53, or having 1, 2, or 3 amino acid differences from the amino acid sequence shown in SEQ ID NO: 6, 14, 22, 30, 39, 52 or 53; and LCDR3 having an amino acid sequence as shown in SEQ ID NO: 7, 15, 23 or 31, or having 1, 2, or 3 amino acid differences from the amino acid sequence shown in SEQ ID NO: 7, 15, 23 or 31.

In some embodiments, the antibody or antigen-binding fragment thereof that specifically binds to FGFR2b of the present invention comprises: HCDR1 having an amino acid sequence as shown in SEQ ID NO: 17 or 25, or having 1, 2, or 3 amino acid differences from the amino acid sequence shown in SEQ ID NO: 17 or 25; HCDR2 having an amino acid sequence as shown in SEQ ID NO: 18, 26, 33, 43, 44, or 45, or having 1, 2, or 3 amino acid differences from the amino acid sequence shown in SEQ ID NO: 18, 26, 33, 43, 44, or 45; HCDR3 having an amino acid sequence as shown in SEQ ID NO: 19 or 27, or having 1, 2, or 3 amino acid differences from the amino acid sequence shown in SEQ ID NO: 19 or 27; LCDR1 having an amino acid sequence as shown in SEQ ID NO: 21, 29, 37, 38 or 51, or having 1, 2, or 3 amino acid differences from the amino acid sequence shown in SEQ ID NO: 21, 29, 37, 38 or 51; LCDR2 having an amino acid sequence as shown in SEQ ID NO: 39, 52 or 53, or having 1, 2, or 3 amino acid differences from the amino acid sequence shown in SEQ ID NO: 39, 52 or 53; and LCDR3 having an amino acid sequence as shown in SEQ ID NO: 23 or 31, or having 1, 2, or 3 amino acid differences from the amino acid sequence shown in SEQ ID NO: 23 or 31.

In some embodiments, the heavy chain variable region of the anti-FGFR2b antibody or antigen-binding fragment thereof of the present invention comprises:
HCDR1, HCDR2 and HCDR3 having amino acid sequences as shown in SEQ ID NO: 1, SEQ ID NO: 2 and SEQ ID NO: 3, respectively; or HCDR1, HCDR2 and HCDR3 having 1, 2 or 3 amino acid differences from the amino acid sequences as shown in SEQ ID NO: 1, SEQ ID NO: 2 and SEQ ID NO: 3; or
HCDR1, HCDR2 and HCDR3 having amino acid sequences as shown in SEQ ID NO: 9, SEQ ID NO: 10 and SEQ ID NO: 11, respectively; or HCDR1, HCDR2 and HCDR3 having 1, 2 or 3 amino acid differences from the amino acid sequences as shown in SEQ ID NO: 9, SEQ ID NO: 10 and SEQ ID NO: 11; or
HCDR1, HCDR2 and HCDR3 having amino acid sequences as shown in SEQ ID NO: 17, SEQ ID NO: 18 and SEQ ID NO: 19, respectively; or HCDR1, HCDR2 and HCDR3 having 1, 2 or 3 amino acid differences from the amino acid sequences as shown in SEQ ID NO: 17, SEQ ID NO: 18 and SEQ ID NO: 19; or
HCDR1, HCDR2 and HCDR3 having amino acid sequences as shown in SEQ ID NO: 25, SEQ ID NO: 26 and SEQ ID NO: 27, respectively; or HCDR1, HCDR2 and HCDR3 having 1, 2 or 3 amino acid differences from the amino acid sequences as shown in SEQ ID NO: 25, SEQ ID NO: 26 and SEQ ID NO: 27; or
HCDR1, HCDR2 and HCDR3 having amino acid sequences as shown in SEQ ID NO: 17, SEQ ID NO: 33 and SEQ ID NO: 19, respectively; or HCDR1, HCDR2 and HCDR3 having 1, 2 or 3 amino acid differences from the amino acid sequences as shown in SEQ ID NO: 17, SEQ ID NO: 33 and SEQ ID NO: 19; or
HCDR1, HCDR2 and HCDR3 having amino acid sequences as shown in SEQ ID NO: 25, SEQ ID NO: 43 and SEQ ID NO: 27, respectively; or HCDR1, HCDR2 and HCDR3 having 1, 2 or 3 amino acid differences from the amino acid sequences as shown in SEQ ID NO: 25, SEQ ID NO: 43 and SEQ ID NO: 27; or
HCDR1, HCDR2 and HCDR3 having amino acid sequences as shown in SEQ ID NO: 25, SEQ ID NO: 44 and SEQ ID NO: 27, respectively; or HCDR1, HCDR2 and HCDR3 having 1, 2 or 3 amino acid differences from the amino acid sequences as shown in SEQ ID NO: 25, SEQ ID NO: 44 and SEQ ID NO: 27; or
HCDR1, HCDR2 and HCDR3 having amino acid sequences as shown in SEQ ID NO: 25, SEQ ID NO: 45 and SEQ ID NO: 27, respectively; or HCDR1, HCDR2 and HCDR3 having 1, 2 or 3 amino acid differences from the amino acid sequences as shown in SEQ ID NO: 25, SEQ ID NO: 45 and SEQ ID NO: 27.

In some embodiments, the light chain variable region of the anti-FGFR2b antibody or antigen-binding fragment thereof of the present invention comprises:
LCDR1, LCDR2 and LCDR3 having amino acid sequences as shown in SEQ ID NO: 5, SEQ ID NO: 6 and SEQ ID NO: 7, respectively; or LCDR1, LCDR2 and LCDR3 having 1, 2 or 3 amino acid differences from the amino acid sequences as shown in SEQ ID NO: 5, SEQ ID NO: 6 and SEQ ID NO: 7; or
LCDR1, LCDR2 and LCDR3 having amino acid sequences as shown in SEQ ID NO: 13, SEQ ID NO: 14 and SEQ ID NO: 15, respectively; or LCDR1, LCDR2 and LCDR3 having 1, 2 or 3 amino acid differences from the amino acid sequences as shown in SEQ ID NO: 13, SEQ ID NO: 14 and SEQ ID NO: 15; or
LCDR1, LCDR2 and LCDR3 having amino acid sequences as shown in SEQ ID NO: 21, SEQ ID NO: 22 and SEQ ID NO: 23, respectively; or LCDR1, LCDR2 and LCDR3 having 1, 2 or 3 amino acid differences from the amino acid sequences as shown in SEQ ID NO: 21, SEQ ID NO: 22 and SEQ ID NO: 23; or
LCDR1, LCDR2 and LCDR3 having amino acid sequences as shown in SEQ ID NO: 29, SEQ ID NO: 30 and SEQ ID NO: 31, respectively; or LCDR1, LCDR2 and LCDR3 having 1, 2 or 3 amino acid differences from the amino acid sequences as shown in SEQ ID NO: 29, SEQ ID NO: 30 and SEQ ID NO: 31; or
LCDR1, LCDR2 and LCDR3 having amino acid sequences as shown in SEQ ID NO: 21, SEQ ID NO: 39 and SEQ ID NO: 23, respectively; or LCDR1, LCDR2 and LCDR3 having 1, 2 or 3 amino acid differences from the amino acid sequences as shown in SEQ ID NO: 21, SEQ ID NO: 39 and SEQ ID NO: 23; or
LCDR1, LCDR2 and LCDR3 having amino acid sequences as shown in SEQ ID NO: 37, SEQ ID NO: 39 and SEQ ID NO: 23, respectively; or LCDR1, LCDR2 and LCDR3 having 1, 2 or 3 amino acid differences from the amino acid sequences as shown in SEQ ID NO: 37, SEQ ID NO: 39 and SEQ ID NO: 23; or
LCDR1, LCDR2 and LCDR3 having amino acid sequences as shown in SEQ ID NO: 38, SEQ ID NO: 39 and SEQ ID NO: 23, respectively; or LCDR1, LCDR2 and LCDR3 having 1, 2 or 3 amino acid differences from the amino acid sequences as shown in SEQ ID NO: 38, SEQ ID NO: 39 and SEQ ID NO: 23; or
LCDR1, LCDR2 and LCDR3 having amino acid sequences as shown in SEQ ID NO: 51, SEQ ID NO: 52 and SEQ ID NO: 31, respectively; or LCDR1, LCDR2 and LCDR3 having 1, 2 or 3 amino acid differences from the amino acid sequences as shown in SEQ ID NO: 51, SEQ ID NO: 52 and SEQ ID NO: 31; or
LCDR1, LCDR2 and LCDR3 having amino acid sequences as shown in SEQ ID NO: 51, SEQ ID NO: 53 and SEQ ID NO: 31, respectively; or LCDR1, LCDR2 and LCDR3 having 1, 2 or 3 amino acid differences from the amino acid sequences as shown in SEQ ID NO: 51, SEQ ID NO: 53 and SEQ ID NO: 31.

In some embodiments, the antibody of the present invention comprises:
a heavy chain variable region comprising HCDR1, HCDR2, and HCDR3 as shown in SEQ ID NO: 1, SEQ ID NO: 2, and SEQ ID NO: 3, respectively; and a light chain variable region comprising LCDR1, LCDR2, and LCDR3 as shown in SEQ ID NO: 5, SEQ ID NO: 6, and SEQ ID NO: 7, respectively; or
a heavy chain variable region comprising HCDR1, HCDR2, and HCDR3 as shown in SEQ ID NO: 9, SEQ ID NO: 10, and SEQ ID NO: 11, respectively; and a light chain variable region comprising LCDR1, LCDR2, and LCDR3 as shown in SEQ ID NO: 13, SEQ ID NO: 14, and SEQ ID NO: 15, respectively; or
a heavy chain variable region comprising HCDR1, HCDR2, and HCDR3 as shown in SEQ ID NO: 17, SEQ ID NO: 18, and SEQ ID NO: 19, respectively; and a light chain variable region comprising LCDR1, LCDR2, and LCDR3 as shown in SEQ ID NO: 21, SEQ ID NO: 22, and SEQ ID NO: 23, respectively; or
a heavy chain variable region comprising HCDR1, HCDR2, and HCDR3 as shown in SEQ ID NO: 25, SEQ ID NO: 26, and SEQ ID NO: 27, respectively; and a light chain variable region comprising LCDR1, LCDR2, and LCDR3 as shown in SEQ ID NO: 29, SEQ ID NO: 30, and SEQ ID NO: 31, respectively; or
a heavy chain variable region comprising HCDR1, HCDR2, and HCDR3 as shown in SEQ ID NO: 17, SEQ ID NO: 33, and SEQ ID NO: 19, respectively; and a light chain variable region comprising LCDR1, LCDR2, and LCDR3 as shown in SEQ ID NO: 37, SEQ ID NO: 39, and SEQ ID NO: 23, respectively; or
a heavy chain variable region comprising HCDR1, HCDR2, and HCDR3 as shown in SEQ ID NO: 17, SEQ ID NO: 18, and SEQ ID NO: 19, respectively; and a light chain variable region comprising LCDR1, LCDR2, and LCDR3 as shown in SEQ ID NO: 37, SEQ ID NO: 39, and SEQ ID NO: 23, respectively; or
a heavy chain variable region comprising HCDR1, HCDR2, and HCDR3 as shown in SEQ ID NO: 17, SEQ ID NO: 33, and SEQ ID NO: 19, respectively; and a light chain variable region comprising LCDR1, LCDR2, and LCDR3 as shown in SEQ ID NO: 38, SEQ ID NO: 39, and SEQ ID NO: 23, respectively; or
a heavy chain variable region comprising HCDR1, HCDR2, and HCDR3 as shown in SEQ ID NO: 17, SEQ ID NO: 18, and SEQ ID NO: 19, respectively; and a light chain variable region comprising LCDR1, LCDR2, and LCDR3 as shown in SEQ ID NO: 38, SEQ ID NO: 39, and SEQ ID NO: 23, respectively; or
a heavy chain variable region comprising HCDR1, HCDR2, and HCDR3 as shown in SEQ ID NO: 17, SEQ ID NO: 33, and SEQ ID NO: 19, respectively; and a light chain variable region comprising LCDR1, LCDR2, and LCDR3 as shown in SEQ ID NO: 21, SEQ ID NO: 39, and SEQ ID NO: 23, respectively; or
a heavy chain variable region comprising HCDR1, HCDR2, and HCDR3 as shown in SEQ ID NO: 17, SEQ ID NO: 18, and SEQ ID NO: 19, respectively; and a light chain variable region comprising LCDR1, LCDR2, and LCDR3 as shown in SEQ ID NO: 21, SEQ ID NO: 39, and SEQ ID NO: 23, respectively; or
a heavy chain variable region comprising HCDR1, HCDR2, and HCDR3 as shown in SEQ ID NO: 25, SEQ ID NO: 43, and SEQ ID NO: 27, respectively; and a light chain variable region comprising LCDR1, LCDR2, and LCDR3 as shown in SEQ ID NO: 51, SEQ ID NO: 52, and SEQ ID NO: 31, respectively; or
a heavy chain variable region comprising HCDR1, HCDR2, and HCDR3 as shown in SEQ ID NO: 25, SEQ ID NO: 44, and SEQ ID NO: 27, respectively; and a light chain variable region comprising LCDR1, LCDR2, and LCDR3 as shown in SEQ ID NO: 51, SEQ ID NO: 52, and SEQ ID NO: 31, respectively; or
a heavy chain variable region comprising HCDR1, HCDR2, and HCDR3 as shown in SEQ ID NO: 25, SEQ ID NO: 44, and SEQ ID NO: 27, respectively; and a light chain variable region comprising LCDR1, LCDR2, and LCDR3 as shown in SEQ ID NO: 51, SEQ ID NO: 53, and SEQ ID NO: 31, respectively; or
a heavy chain variable region comprising HCDR1, HCDR2, and HCDR3 as shown in SEQ ID NO: 25, SEQ ID NO: 45, and SEQ ID NO: 27, respectively; and a light chain variable region comprising LCDR1, LCDR2, and LCDR3 as shown in SEQ ID NO: 51, SEQ ID NO: 52, and SEQ ID NO: 31, respectively; or
a heavy chain variable region comprising HCDR1, HCDR2, and HCDR3 as shown in SEQ ID NO: 25, SEQ ID NO: 45, and SEQ ID NO: 27, respectively; and a light chain variable region comprising LCDR1, LCDR2, and LCDR3 as shown in SEQ ID NO: 51, SEQ ID NO: 53, and SEQ ID NO: 31, respectively; or
a heavy chain variable region comprising HCDR1, HCDR2, and HCDR3 as shown in SEQ ID NO: 25, SEQ ID NO: 43, and SEQ ID NO: 27, respectively; and a light chain variable region comprising LCDR1, LCDR2, and LCDR3 as shown in SEQ ID NO: 51, SEQ ID NO: 53, and SEQ ID NO: 31, respectively.

In some embodiments, the antibody or antigen-binding fragment thereof of the present invention comprises a heavy chain variable region and a light chain variable region:
the heavy chain variable region comprises the amino acid sequence as shown in SEQ ID NO:4, or an amino acid sequence that has at least 95%, 96%, 97%, 98% or 99% sequence identity to the amino acid sequence as shown in SEQ ID NO:4; and the light chain variable region comprises the amino acid sequence as shown in SEQ ID NO:8, or an amino acid sequence that has at least 95%, 96%, 97%, 98% or 99% sequence identity to the amino acid sequence as shown in SEQ ID NO:8; or
the heavy chain variable region comprises the amino acid sequence as shown in SEQ ID NO:12, or an amino acid sequence that has at least 95%, 96%, 97%, 98% or 99% sequence identity to the amino acid sequence as shown in SEQ ID NO:12; and the light chain variable region comprises the amino acid sequence as shown in SEQ ID NO:16, or an amino acid sequence that has at least 95%, 96%, 97%, 98% or 99% sequence identity to the amino acid sequence as shown in SEQ ID NO:16; or
the heavy chain variable region comprises the amino acid sequence as shown in SEQ ID NO:20, or an amino acid sequence that has at least 95%, 96%, 97%, 98% or 99% sequence identity to the amino acid sequence as shown in SEQ ID NO:20; and the light chain variable region comprises the amino acid sequence as shown in SEQ ID NO:24, or an amino acid sequence that has at least 95%, 96%, 97%, 98% or 99% sequence identity to the amino acid sequence as shown in SEQ ID NO:24; or
the heavy chain variable region comprises the amino acid sequence as shown in SEQ ID NO:28, or an amino acid sequence that has at least 95%, 96%, 97%, 98% or 99% sequence identity to the amino acid sequence as shown in SEQ ID NO:28; and the light chain variable region comprises the amino acid sequence as shown in SEQ ID NO:32, or an amino acid sequence that has at least 95%, 96%, 97%, 98% or 99% sequence identity to the amino acid sequence as shown in SEQ ID NO:32.

In some embodiments, the antibody or antigen-binding fragment thereof of the present invention comprises:
the heavy chain variable region comprising the amino acid sequence shown in any one of SEQ ID NOs: 34, 35, 36, 46, 47, 48, 49, or 50; and
the light chain variable region comprising the amino acid sequence shown in any one of SEQ ID NO: 40, 41, 42, 54, 55, 56, 57, 58, 58, 60, 61 or 62.

In some embodiments, the antibody or antigen-binding fragment thereof of the present invention comprises:
the heavy chain variable region comprising an amino acid sequence comprising at least 95%, 96%, 97%, 98% or 99% sequence identity to the amino acid sequence shown in any one of SEQ ID NOs: 34, 35, 36, 46, 47, 48, 49 or 50; and
the light chain variable region comprising an amino acid sequence comprising at least 95%, 96%, 97%, 98% or 99% sequence identity to the amino acid sequence shown in any one of SEQ ID NOs: 40, 41, 42, 54, 55, 56, 57, 58, 58, 60, 61 or 62.

In some embodiments, the antibody or antigen-binding fragment thereof of the present invention comprises:
the heavy chain variable region having the amino acid sequence shown in SEQ ID NO: 34 and the light chain variable region having the amino acid sequence shown in SEQ ID NO: 40, 41 or 42; or
the heavy chain variable region having the amino acid sequence shown in SEQ ID NO: 35 and the light chain variable region having the amino acid sequence shown in SEQ ID NO: 40, 41 or 42; or
the heavy chain variable region having the amino acid sequence shown in SEQ ID NO: 36 and the light chain variable region having the amino acid sequence shown in SEQ ID NO: 40, 41 or 42; or
the heavy chain variable region having the amino acid sequence shown in SEQ ID NO: 46, 47, or 48 and the light chain variable region having the amino acid sequence shown in SEQ ID NO: 54; or
the heavy chain variable region having the amino acid sequence shown in SEQ ID NO: 46, 47, or 48 and the light chain variable region having the amino acid sequence shown in SEQ ID NO: 55; or
the heavy chain variable region having the amino acid sequence shown in SEQ ID NO: 48 or 49 and the light chain variable region having the amino acid sequence shown in SEQ ID NO: 56; or
the heavy chain variable region having the amino acid sequence shown in SEQ ID NO: 48 or 49 and the light chain variable region having the amino acid sequence shown in SEQ ID NO: 57; or
the heavy chain variable region having the amino acid sequence shown in SEQ ID NO: 48 or 49 and the light chain variable region having the amino acid sequence shown in SEQ ID NO: 58; or
the heavy chain variable region having the amino acid sequence shown in SEQ ID NO: 49 or 50 and the light chain variable region having the amino acid sequence shown in SEQ ID NO: 59; or
the heavy chain variable region having the amino acid sequence shown in SEQ ID NO: 49 or 50 and the light chain variable region having the amino acid sequence shown in SEQ ID NO: 60; or
the heavy chain variable region having the amino acid sequence shown in SEQ ID NO: 49 or 50 and the light chain variable region having the amino acid sequence shown in SEQ ID NO: 61; or
the heavy chain variable region having the amino acid sequence shown in SEQ ID NO: 49 or 50 and the light chain variable region having the amino acid sequence shown in SEQ ID NO: 62.

In some embodiments, the antibody or antigen-binding fragment thereof of the present invention comprises:
the heavy chain comprising the amino acid sequence shown in any one of SEQ ID NO: 63, and
the light chain comprising the amino acid sequence shown in any one of SEQ ID NO: 64, 65, or 66.

In some embodiments, the antibody or antigen-binding fragment thereof of the present invention comprises:
the heavy chain comprising an amino acid sequence comprising at least 95%, 96%, 97%, 98% or 99% sequence identity to the amino acid sequence shown in any one of SEQ ID NOs: 63; and
the light chain comprising an amino acid sequence comprising at least 95%, 96%, 97%, 98% or 99% sequence identity to the amino acid sequence shown in any one of SEQ ID NOs: 64, 65, or 66.

In some embodiments, the antibody or antigen-binding fragment thereof of the present invention comprises:
the heavy chain variable region having the amino acid sequence shown in SEQ ID NO: 63 and the light chain variable region having the amino acid sequence shown in SEQ ID NO: 64, or
the heavy chain variable region having the amino acid sequence shown in SEQ ID NO: 63 and the light chain variable region having the amino acid sequence shown in SEQ ID NO: 65, or
the heavy chain variable region having the amino acid sequence shown in SEQ ID NO: 63 and the light chain variable region having the amino acid sequence shown in SEQ ID NO: 66.

In some embodiments, the antibody or antigen-binding fragment thereof described in the present invention is a murine antibody, a chimeric antibody, a humanized antibody, or a fully human antibody.

Any suitable method for generating an antibody may be used to produce the antibody of the present invention. Any suitable form of FGFR2b may be used as an immunogen (antigen) to generate the antibody. By way of example and not limitation, any FGFR2b variant or fragment thereof may be used as an immunogen. In some embodiments, a hybridoma cell that produces a murine monoclonal anti-human FGFR2b antibody can be generated by methods well known in the art.

Antibodies derived from rodents (e.g., mice) when used as therapeutic agents in vivo may induce unwanted antibody immunogenicity, with repeated use leading to an immune response in humans against the therapeutic antibody. Such an immune response results in at least loss of therapeutic efficacy, and in severe cases, a potentially fatal allergic reaction. One approach to reduce the immunogenicity of rodent antibodies involves the generation of chimeric antibodies, in which the mouse variable regions are fused to human constant regions (Liu et al. (1987) Proc. Natl. Acad. Sci. USA 84:3439-43). However, the retention of intact rodent variable regions in chimeric antibodies may still elicit deleterious immunogenicity in patients. Transplanting the complementarity determining region (CDR) loops of rodent variable domains onto human frameworks (i.e., humanization) has been used to further minimize rodent sequences (Jones et al. (1986) NER24 Nature 321: 522; Verhoeyen et al. (1988) Science 239: 1534).

In some embodiments, the chimeric or humanized antibody of the present invention can be prepared based on the sequence of the murine monoclonal hybridoma antibody prepared as described above. DNA encoding the heavy and light chain immunoglobulins can be obtained from the desired murine hybridoma and engineered using standard molecular biology techniques to incorporate non-murine (e.g., human) immunoglobulin sequences.

In some embodiments, the chimeric FGFR2b antibody of the present invention can be prepared by operably linking the hybridoma-derived immunoglobulin heavy and light chain variable regions to human IgG constant regions using methods known in the art (see, e.g., U.S. Patent No. 4,816,567 to Cabilly et al.) to obtain a chimeric heavy chain and a chimeric light chain. In some embodiments, the chimeric antibody of the present invention comprises a constant region selected from any human IgG subtype, such as IgG1, IgG2, IgG3, IgG4, preferably IgG1.

In some embodiments, the chimeric FGFR2b antibody of the present invention can be obtained by "mixing and matching" chimeric light chain and chimeric heavy chain expression plasmids to transfect expression cells, and the FGFR2b binding of such "mixed and matched" antibodies can be tested using the binding assays described above and other conventional binding assays (e.g., ELISA).

The precise amino acid sequence boundaries of the CDRs in the variable region of the antibody of the present invention can be determined using any of a number of well-known schemes, including Chothia based on the three-dimensional structure of the antibody and the topology of CDR loops (Chothia et al. (1989) Nature 342:877-883; Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), the international ImMunoGeneTics database (IMGT) (1999 Nucleic Acids Research, 27, 209-212), and the North CDR definition based on affinity propagation clustering using a large number of crystal structures.

Unless otherwise indicated, the CDRs of the antibody of the present invention can have their boundaries determined by those skilled in the art according to any scheme in the art (e.g., different assignment systems or combinations thereof).

It should be noted that the boundaries of the CDRs of the variable region of the same antibody obtained based on different assignment systems may be different. That is, the CDR sequences of variable regions of the same antibody defined under different assignment systems may be different. Therefore, when an antibody is defined with reference to the specific CDR sequences defined herein, the scope of the antibody also covers an antibody whose variable region sequence comprises the specific CDR sequences, but whose claimed CDR boundaries differ from the specific CDR boundaries defined herein due to the application of a different scheme (e.g., a different assignment system or combination).

Antibodies with different specificities (i.e., different binding sites for different antigens) have different CDRs. However, although CDR is different between antibodies, there are only a limited number of amino acid positions in the CDR that directly participate in antigen binding. Using at least two of the Kabat, Chothia, AbM, Contact and North methods, the minimum overlapping region can be determined, thereby providing a "minimum binding unit" for antigen binding. The minimum binding unit can be a sub-portion of a CDR. As those skilled in the art will appreciate, by the structure of the antibody and protein folding, the residues of the CDR sequence remainder can be determined. Therefore, the present invention also contemplates the variants of any CDR provided herein. For example, in a variant of a CDR, the amino acid residues of the minimum binding unit can remain unchanged, and the remaining CDR residues defined according to Kabat or Chothia can be replaced by conservative amino acid residues.

### >Chi-006-VH

HCDR1: SYNIH (SEQ ID NO:1)
HCDR2: YIYPDNGDTNYNQKFKG (SEQ ID NO:2)
HCDR3: DWTY (SEQ ID NO:3)

QAYLQQSGAELVRSGASVKMSCKASGYTFTSYNIHWVKQTPGQGLEWIGYIYPDNG
DTNYNQKFKGKAALTADTSSSTAYMQISSLTSEDSAVYFCARDWTYWGQGTLVTVSA
(SEQ ID NO:4)

### >Chi-006-VL

LCDR1: SATSIVNHMY (SEQ ID NO:5)
LCDR2: FTSNLAS (SEQ ID NO:6)
LCDR3: QQWSSTPFT (SEQ ID NO:7)

QIVLTQSPALMSASPGEKVTMTCSATSIVNHMYWYQQKPGSSPKPWIYFTSNLASGV
PARFSGSGSGTSYSLTISSMEVEDAATYYCQQWSSTPFTFGSGTKLEIK (SEQ ID NO:8)

### >Chi-018-VH

HCDR1: DYYIH (SEQ ID NO:9)
HCDR2: WIYHENGDTEFAPKFQG (SEQ ID NO:10)
HCDR3: KITY (SEQ ID NO:11)

EVQLQQSGAELVRSGASVKLSCTASGFNIKDYYIHWMKQRPEQGLEWIGWIYHENG
DTEFAPKFQGKATMTADTSSNTAYLQLNSLTSEDSGVYYCNAKITYWGQGTLVTVSA
(SEQ ID NO:12)

### >Chi-018-VL

LCDR1: KASQNVGTNVA (SEQ ID NO:13)
LCDR2: SASYRFS (SEQ ID NO:14)
LCDR3: QQYYSSPYT (SEQ ID NO:15)

DIMMTQSQKFMSTSVGDRVSVTCKASQNVGTNVAWYQQIPGQSPKALIYSASYRFS
GVPDRFTGSGSGTDFTLTISNVQSEDLADYFCQQYYSSPYTFGGGTKLEIK (SEQ ID NO:16)

### >Chi-032-VH

HCDR1: SYNMH (SEQ ID NO:17)
HCDR2: YIYPENGDTNYNQRFKG (SEQ ID NO:18)
HCDR3: ADY (SEQ ID NO:19)

QAYLQQSGAELVRSGASVKMSCKASGYTFTSYNMHWIKQTPGQGLEWIGYIYPENG
DTNYNQRFKGKATLTADTSSRTAYMQISSLTSEDSAVYFCATADYWGQGTSVTVSS (SEQ
ID NO:20)

### >Chi-032-VL

LCDR1: SASSSVNHMY (SEQ ID NO:21)
LCDR2: LTSNLAS (SEQ ID NO:22)
LCDR3: QQWSSNPFT (SEQ ID NO:23)

EIVLTQSPALMSASPGERVTMTCSASSSVNHMYWYQQKPRSSPKPWIYLTSNLASGV
PARFSGSGSGTSYSLTISSMEAEDAATYYCQQWSSNPFTFGSGTKLEIK (SEQ ID NO:24)

### >Chi-042-VH

HCDR1: DYNMH (SEQ ID NO:25)
HCDR2: YIYPNNGDTSYNQKFKG (SEQ ID NO:26)
HCDR3: GLLD (SEQ ID NO:27)

EVQLQQSGPELVKPGASVKMSCKASGYTFTDYNMHWVKQSHGKSLEWIGYIYPNN
GDTSYNQKFKGKATLTVNKSSSTAYMELRSLTSEDSAVYYCARGLLDWGQGTLVTVSA
(SEQ ID NO:28)

### >Chi-042-VL

LCDR1: GASENIYGALN (SEQ ID NO:29)
LCDR2: GATNLAD (SEQ ID NO:30)
LCDR3: QNVLSTPYT (SEQ ID NO:31)

DIQMTQSPASLSASVGETVTITCGASENIYGALNWYQRKQGKSPQLLIYGATNLADG

MSSRFSGSGSGRQYSLKISSLHPDDVATYYCQNVLSTPYTFGGGTKLEIK (SEQ ID NO:32)

The humanized antibody of the present invention can be prepared by inserting murine CDR regions into human germline framework regions using methods known in the art. See, U.S. Patent No. 5,225,539 to Winter et al. and U.S. Patent Nos. 5,530,101; 5,585,089; 5,693,762; and 6,180,370 to Queen et al.

The amino acid sequences of the light chain and heavy chain variable regions and CDRs of the anti-human FGFR2b humanized antibody of the present invention are as follows:
The HCDR sequences of hu-032-HC are as follows:
HCDR1: SYNMH (SEQ ID NO:17)
HCDR2: YIYPENGDTNYNQKFQG (SEQ ID NO:18) or YIYPENGDTNYAQKFQG (SEQ ID NO:33)
HCDR3: ADY (SEQ ID NO:19)
hu-032-HC-1 
QVQLVQSGAEVKKPGASVKVSCKASGYTFTSYNMHWVRQAPGQGLEWMGYIYPEN
GDTNYAQKFQGRVTMTRDTSISTAYMELSRLRSDDTAVYYCATADYWGQGTLVTVSS
(SEQ ID NO:34)
hu-032-HC-2 
QVQLVQSGAEVKKPGASVKVSCKASGYTFTSYNMHWVRQAPGQGLEWMGYIYPEN
GDTNYNQKFQGRVTMTRDTSISTAYMELSRLRSDDTAVYYCATADYWGQGTLVTVSS
(SEQ ID NO:35)
hu-032-HC-3

QVQLVQSGAEVKKPGASVKVSCKASGYTFTSYNMHWVRQAPGQGLEWMGYIYPEN
GDTNYNQKFQGRVTMTRDTSIRTAYMELSRLRSDDTAVYYCATADYWGQGTLVTVSS
(SEQ ID NO:36)

The LCDR sequences of hu-032-LC are as follows:
LCDR1
   RASSSVNHMY (SEQ ID NO:21) or RASSSVNHLA (SEQ ID NO:37) or RASSSVNHMA (SEQ ID NO:38)
LCDR2: LTSNRAT (SEQ ID NO:39)
LCDR3: QQWSSNPFT (SEQ ID NO:23)
hu-032-LC-1 
EIVLTQSPATLSLSPGERATLSCRASSSVNHLAWYQQKPGQAPRLLIYLTSNRATGIPA
RFSGSGSGTDFTLTISSLEPEDFAVYYCQQWSSNPFTFGQGTKLEIK (SEQ ID NO:40)
hu-032-LC-2 
EIVLTQSPATLSLSPGERATLSCRASSSVNHMAWYQQKPGQAPRPLIYLTSNRATGIP
ARFSGSGSGTDFTLTISSLEPEDFAVYYCQQWSSNPFTFGQGTKLEIK (SEQ ID NO:41)
hu-032-LC-3

EIVLTQSPATLSLSPGERATLSCRASSSVNHMYWYQQKPGQSPRPLIYLTSNRATGIPA
RFSGSGSGTDFTLTISSLEPEDFAVYYCQQWSSNPFTFGQGTKLEIK (SEQ ID NO:42)

The HCDR sequences of hu-042-HC are as follows:
HCDR1: DYNMH (SEQ ID NO:25)
HCDR2: IIYPNNGDTSYAQKFQG (SEQ ID NO:43) or YIYPNNGDTSYNQKFQG (SEQ ID NO:44) or YIYPNNGDTSYAQKFQG (SEQ ID NO:45)
HCDR3: GLLD (SEQ ID NO:27)
hu-042-HC-1 
QVQLVQSGAEVKKPGASVKVSCKASGYTFTDYNMHWVRQAPGQGLEWMGIIYPNN
GDTSYAQKFQGRVTMTRDTSTSTVYMELSSLRSEDTAVYYCARGLLDWGQGTLVTVSS
(SEQ ID NO:46)
hu-042-HC-2 
QVQLVQSGAEVKKPGASVKVSCKASGYTFTDYNMHWVRQAPGQGLEWMGYIYPN
NGDTSYNQKFQGRVTMTRDTSTSTVYMELSSLRSEDTAVYYCARGLLDWGQGTLVTVSS
(SEQ ID NO:47)
hu-042-HC-3 
QVQLVQSGAEVKKPGASVKVSCKASGYTFTDYNMHWVRQAPGQGLEWMGYIYPN
NGDTSYNQKFQGRVTMTVDKSTSTVYMELSSLRSEDTAVYYCARGLLDWGQGTLVTVS
S (SEQ ID NO:48)
hu-042-HC-4 
QVQLVQSGAEVKKPGASVKVSCKASGYTFTDYNMHWVRQAPGQGLEWMGYIYPN
NGDTSYAQKFQGRVTMTVDKSTSTVYMELSSLRSEDTAVYYCARGLLDWGQGTLVTVS
S (SEQ ID NO:49)
hu-042-HC-5

QVQLVQSGAEVKKPGASVKVSCKASGYTFTDYNMHWVRQAPGQGLEWMGIIYPNN
GDTSYAQKFQGRVTMTVDKSTSTVYMELSSLRSEDTAVYYCARGLLDWGQGTLVTVSS
(SEQ ID NO:50)

The LCDR sequences of hu-042-LC are as follows:
LCDR1: RASENIYGALN (SEQ ID NO:51)
LCDR2: GATSLQS (SEQ ID NO:52) or GATSLAS (SEQ ID NO:53)
LCDR3: QNVLSTPYT (SEQ ID NO:31)
hu-042-LC-1 
DIQMTQSPSSLSASVGDRVTITCRASENIYGALNWYQQKPGKAPKLLIYGATSLQSGV
PSRFSGSGSGTDFTLTISSLQPEDFATYYCQNVLSTPYTFGQGTKLEIK (SEQ ID NO:54)
hu-042-LC-2 
DIQMTQSPSSLSASVGDRVTITCRASENIYGALNWYQQKPGKSPKLLIYGATSLQSGV
PSRFSGSGSGTDFTLTISSLQPEDFATYYCQNVLSTPYTFGQGTKLEIK (SEQ ID NO:55)
hu-042-LC-3 
DIQMTQSPSSLSASVGDRVTITCRASENIYGALNWYQQKPGKAPKLLIYGATSLQSGV
PSRFSGSGSGRDFTLTISSLQPEDFATYYCQNVLSTPYTFGQGTKLEIK (SEQ ID NO:56)
hu-042-LC-4 
DIQMTQSPSSLSASVGDRVTITCRASENIYGALNWYQRKPGKAPKLLIYGATSLQSGV
PSRFSGSGSGRDFTLTISSLQPEDFATYYCQNVLSTPYTFGQGTKLEIK (SEQ ID NO:57)
hu-042-LC-5 
DIQMTQSPSSLSASVGDRVTITCRASENIYGALNWYQRKPGKAPKLLIYGATSLASGV
PSRFSGSGSGRDFTLTISSLQPEDFATYYCQNVLSTPYTFGQGTKLEIK (SEQ ID NO:58)
hu-042-LC-6 
DIQMTQSPSSLSASVGDRVTITCRASENIYGALNWYQRKPGKSPKLLIYGATSLASGV
PSRFSGSGSGRDFTLTISSLQPEDFATYYCQNVLSTPYTFGQGTKLEIK (SEQ ID NO:59)
hu-042-LC-7 
DIQMTQSPSSLSASVGDRVTITCRASENIYGALNWYQRKPGKSPKLLIYGATSLASGV
PSRFSGSGSGTDFTLTISSLQPEDFATYYCQNVLSTPYTFGQGTKLEIK (SEQ ID NO:60)
hu-042-LC-8 
DIQMTQSPSSLSASVGDRVTITCRASENIYGALNWYQQKPGKSPKLLIYGATSLASGV
PSRFSGSGSGTDFTLTISSLQPEDFATYYCQNVLSTPYTFGQGTKLEIK (SEQ ID NO:61)
hu-042-LC-9

DIQMTQSPSSLSASVGDRVTITCRASENIYGALNWYQQKPGKAPKLLIYGATSLASGV
PSRFSGSGSGTDFTLTISSLQPEDFATYYCQNVLSTPYTFGQGTKLEIK (SEQ ID NO:62)

The numbers of the humanized anti-FGFR2b antibodies of the present invention and the sources of their heavy chain and light chain variable regions are shown in Tables 1 and 2.

**Table 1: Numbering of humanized anti-FGFR2b antibody 032 and sources of heavy and light chain variable regions thereof**

| Serial number | hu-032-HC-1 | hu-032-HC-2 | hu-032-HC-3 |
|---|---|---|---|
| hu-032-LC-1 | hu-032-003 | hu-032-004 | hu-032-005 |
| hu-032-LC-2 | hu-032-006 | hu-032-007 | hu-032-008 |
| hu-032-LC-3 | hu-032-009 | hu-032-010 | hu-032-011 |

**Table 2: Numbering of humanized anti-FGFR2b antibody 042 and sources of heavy and light chain variable regions thereof**

| Serial number | hu-042-HC-1 | hu-042-HC-2 | hu-042-HC-3 | hu-042-HC-4 | hu-042-HC-5 |
|---|---|---|---|---|---|
| hu-042-LC-1 | hu-042-001 | hu-042-002 | hu-042-003 | - | - |
| hu-042-LC-2 | hu-042-004 | hu-042-005 | hu-042-006 | - | - |
| hu-042-LC-3 | - | - | hu-042-007 | hu-042-010 | - |
| hu-042-LC-4 | - | - | hu-042-008 | hu-042-011 | - |
| hu-042-LC-5 | - | - | hu-042-009 | hu-042-012 | - |
| hu-042-LC-6 | - | - | - | hu-042-013 | hu-042-017 |
| hu-042-LC-7 | - | - | - | hu-042-014 | hu-042-018 |
| hu-042-LC-8 | - | - | - | hu-042-015 | hu-042-019 |
| hu-042-LC-9 | - | - | - | hu-042-016 | hu-042-020 |

The light and heavy chain amino acid sequences of antibodies hu-042-012, hu-042-015, and hu-042-016 are shown below.
>hu-042-012-HC, hu-042-015-HC, hu-042-016-HC 
QVQLVQSGAEVKKPGASVKVSCKASGYTFTDYNMHWVRQAPGQGLEWMGYIYPN
NGDTSYAQKFQGRVTMTVDKSTSTVYMELSSLRSEDTAVYYCARGLLDWGQGTLVTVS
SASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPELLGGP
SVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSREEM
TKNQVSLTCLVKGFYPSDIAVEWESNGQPENNYKTTPPVLDSDGSFFLYSKLTVDKSRWQ
QGNVFSCSVMHEALHNHYTQKSLSLSPGK*(SEQ ID NO:63)
>hu-042-012-LC 
DIQMTQSPSSLSASVGDRVTITCRASENIYGALNWYQRKPGKAPKLLIYGATSLASGV
PSRFSGSGSGRDFTLTISSLQPEDFATYYCQNVLSTPYTFGQGTKLEIKRTVAAPSVFIFPPS
DEQLKSGTASVVCLLNNFYPREAKVQWKVDNALQSGNSQESVTEQDSKDSTYSLSSTLTL
SKADYEKHKVYACEVTHQGLSSPVTKSFNRGEC*(SEQ ID NO:64)
>hu-042-015-LC 
DIQMTQSPSSLSASVGDRVTITCRASENIYGALNWYQQKPGKSPKLLIYGATSLASGV
PSRFSGSGSGTDFTLTISSLQPEDFATYYCQNVLSTPYTFGQGTKLEIKRTVAAPSVFIFPPS
DEQLKSGTASVVCLLNNFYPREAKVQWKVDNALQSGNSQESVTEQDSKDSTYSLSSTLTL
SKADYEKHKVYACEVTHQGLSSPVTKSFNRGEC* (SEQ ID NO:65)
>hu-042-016-LC

DIQMTQSPSSLSASVGDRVTITCRASENIYGALNWYQQKPGKAPKLLIYGATSLASGV
PSRFSGSGSGTDFTLTISSLQPEDFATYYCQNVLSTPYTFGQGTKLEIKRTVAAPSVFIFPPS
DEQLKSGTASVVCLLNNFYPREAKVQWKVDNALQSGNSQESVTEQDSKDSTYSLSSTLTL
SKADYEKHKVYACEVTHQGLSSPVTKSFNRGEC* (SEQ ID NO:66)

In some embodiments, the amino acid changes include amino acid deletions, insertions, or substitutions. In some embodiments, the anti-FGFR2b antibody or antigen-binding fragment thereof of the present invention includes an antibody having an amino acid sequence that has been mutated by amino acid deletion, insertion, or substitution, but still has at least about 90%, 91%, 92%, 93%, 94%, 95%, 96%, 97%, 98%, 99%, or 100% identity to the above-mentioned antibody (particularly in the CDR regions depicted in the above sequences). In some embodiments, the antibody of the present invention has no more than 1, 2, 3, 4, or 5 amino acid mutations (by deletion, insertion, or substitution) in the CDR regions when compared to the CDR regions depicted in the specific sequences. In some embodiments, the antibody of the present invention has no more than 1, 2, 3, 4, or 5 amino acid mutations (by deletion, insertion, or substitution) in the CDR regions when compared to the CDR regions depicted in the specific sequences.

In some embodiments, a polynucleotide molecule encoding an antibody of the present invention includes a polynucleotide molecule that has been mutated by nucleotide deletion, insertion, or substitution, but still has at least about 60%, 70%, 80%, 90%, 95%, or 100% identity to the coding regions corresponding to the CDRs depicted in the sequences described above.

In some embodiments, the FGFR2b antibody is an IgG antibody, such as an IgG1, IgG2, IgG3, or IgG4 antibody, or a modified form thereof, as described in the following sections.

In some embodiments, one or more amino acid modifications can be introduced into the Fc region of an antibody provided herein to generate an Fc region variant. The Fc region variant can comprise a human Fc region sequence (e.g., a human IgG1, IgG2, IgG3, or IgG4 Fc region) comprising an amino acid modification (e.g., substitution) at one or more amino acid positions.

In some embodiments, it may be desirable to generate a cysteine-engineered antibody, e.g., a "thioMAb", in which one or more residues of the antibody are replaced with cysteine residues.

In some embodiments, an antibody provided herein may be further modified to contain other non-proteinaceous moieties that are known in the art and readily available. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymers, carboxymethyl cellulose, dextran, polyvinyl alcohol, polyvinyl pyrrolidone, poly-1,3-dioxane, poly-1,3,6-trioxane, ethylene/maleic anhydride copolymers, polyamino acids (homopolymers or random copolymers), and dextran or poly (n-vinyl pyrrolidone) polyethylene glycol, propylene glycol homopolymers, polypropylene oxide/ethylene oxide copolymers, polyoxyethylated polyols (e.g., glycerol), polyvinyl alcohol, and mixtures thereof.

### Antibody Expression

In another aspect, the present invention provides a polynucleotide molecule encoding an anti-FGFR2b antibody or antigen-binding fragment thereof described herein. The polynucleotide molecule may comprise a polynucleotide molecule encoding an amino acid sequence of the light chain variable region and/or the heavy chain variable region of the antibody, or a polynucleotide molecule encoding an amino acid sequence of the light chain and/or the heavy chain of the antibody.

In another aspect, the present invention provides an expression vector comprising the polynucleotide molecule described herein, preferably, the vector is a eukaryotic expression vector. In some embodiments, the polynucleotide molecule as described herein is comprised in one or more expression vectors.

In another aspect, the present invention provides a host cell comprising the polynucleotide molecule described herein or the expression vector described herein, preferably, the host cell is a eukaryotic cell, more preferably a mammalian cell.

In another aspect, the present invention provides a method for preparing the anti-FGFR2b antibody or antigen-binding fragment thereof described herein, the method comprising expressing the antibody or antigen-binding fragment thereof in a host cell described herein under conditions suitable for the expression of the antibody or antigen-binding fragment thereof, and recovering the expressed antibody or antigen-binding fragment thereof from the host cell.

The present invention provides mammalian host cells for expressing the recombinant antibody of the present invention, including many immortalized cell lines available from the American Type Culture Collection (ATCC). These especially include Chinese hamster ovary (CHO) cells, NSO, SP2/0 cells, HeLa cells, baby hamster kidney (BHK) cells, monkey kidney cells (COS), human hepatocellular carcinoma cells, A549 cells, 293T cells and many other cell lines. Mammalian host cells include humans, mice, rats, dogs, monkeys, pigs, goats, cattle, horses and hamster cells. Particularly preferred cell lines are selected by determining which cell line has high expression levels.

In one embodiment, the present invention provides a method for preparing an anti-FGFR2b antibody, wherein the method comprises introducing an expression vector into a mammalian host cell and then culturing the host cell for a period of time sufficient to allow expression of the antibody in the host cell, or more preferably, secretion of the antibody into the culture medium in which the host cell is grown, to produce the antibody. The antibody can be recovered from the culture medium using standard protein purification methods.

It is likely that antibodies expressed by different cell lines or in transgenic animals will have different glycosylation from one another. However, all antibodies encoded by the nucleic acid molecules provided herein or comprising the amino acid sequences provided herein are part of the present invention, regardless of the glycosylation of the antibodies. Likewise, in certain embodiments, non-fucosylated antibodies are advantageous because they generally have more potent efficacy in vitro and in vivo than their fucosylated counterparts and are less likely to be immunogenic because their carbohydrate structures are normal components of natural human serum IgG.

### Antibody-Drug Conjugate (ADC)

The present invention also provides an antibody-drug conjugate (ADC) comprising the antibody or antigen-binding fragment thereof that specifically binds to FGFR2b according to any embodiment of the present invention and a coupling moiety.

In some embodiments, the conjugation moiety comprises an anti-tumor agent, an immunomodulator, a cytotoxic agent, a fluorescent substance, a luminescent substance, an enzyme, a small nucleic acid molecule (including siRNA or antisense oligonucleotide (ASO)), or any combination thereof.

In some embodiments, the antibody-drug conjugate comprises a plurality of conjugation moieties, which may be a combination of different therapeutically active substances or active pharmaceutical ingredients, or a combination of the same therapeutically active substance or active pharmaceutical ingredient.

In some embodiments, the conjugation moiety is covalently linked to the anti-FGFR2b antibody or antigen-binding fragment thereof via a linker in a non-site-specific manner or a site-specific manner.

Based on the obtained antibodies that specifically bind to human FGFR2b, the inventors prepared a series of antibody-drug conjugates (ADCs) using different linkers and cytotoxic drug components, and verified their activities. It was found that the ADCs of the present invention have good inhibitory effects on the proliferation of cells overexpressing human FGFR2b, and have good tumor growth inhibitory efficacy in transplanted tumor models.

In some embodiments, the present invention provides an antibody-drug conjugate having the structure Ab-(L-D)m, wherein Ab is an antibody or antigen-binding fragment thereof that specifically binds to FGFR2b according to any of the embodiments of the present invention; L is a linker; D is a therapeutically active substance or active pharmaceutical ingredient; m represents the average number of L-D units conjugated to Ab, and m ranges from 1 to 8, preferably m is 4, more preferably about 3.0, 3.5, 3.6, 3.7, 3.8, 4.0, 7.2, 7.3, 7.4, 7.5, 7.6, 7.8, or 8.0.

In some embodiments, the amino acid sequence of LCDR1 of the antibody or antigen-binding fragment thereof that specifically binds to FGFR2b is selected from SEQ ID NO: 37 or 51, the amino acid sequence of LCDR2 is selected from SEQ ID NO: 39 or 53, the amino acid sequence of LCDR3 is selected from SEQ ID NO: 23 or 31, the amino acid sequence of HCDR1 is selected from SEQ ID NO: 17 or 25, the amino acid sequence of HCDR2 is selected from SEQ ID NO: 18 or 45, and the amino acid sequence of HCDR3 is selected from SEQ ID NO: 19 or 27.

In some embodiments, the heavy chain variable region of the antibody that specifically binds to FGFR2b comprises HCDR1, HCDR2, and HCDR3 having amino acid sequences as shown in SEQ ID NO: 17, SEQ ID NO: 18, and SEQ ID NO: 19, respectively, and the light chain variable region comprises LCDR1, LCDR2, and LCDR3 having amino acid sequences as shown in SEQ ID NO: 37, SEQ ID NO: 39, and SEQ ID NO: 23, respectively; or the heavy chain variable region comprises HCDR1, HCDR2, and HCDR3 having amino acid sequences as shown in SEQ ID NO: 25, SEQ ID NO: 45, and SEQ ID NO: 27, respectively, and the light chain variable region comprises LCDR1, LCDR2, and LCDR3 having amino acid sequences as shown in SEQ ID NO: 51, SEQ ID NO: 52, and SEQ ID NO: 31, respectively.

In some embodiments, the antibody that specifically binds to FGFR2b is hu-042-012, hu-042-015, and hu-042-016 described herein.

In some embodiments, the antibody can be conjugated to the drug directly or via a linker.

In some embodiments, the drug moiety D is covalently linked to the anti-FGFR2b antibody or antigen-binding fragment thereof via a linker L in a non-site-specific manner or a site-specific manner.

The linker may comprise one or more linker components. In some embodiments, the structure of L is as shown in formula (A): 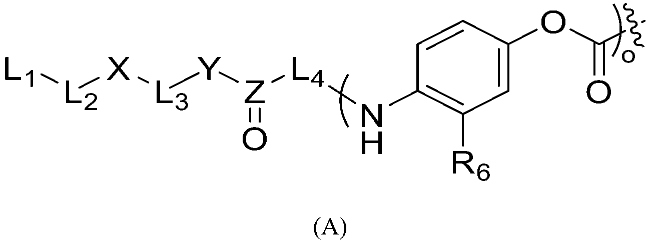 wherein:
L₁ represents a group or bond formed by the reaction of a functional group capable of reacting with a thiol group and a thiol group;
L₂ and Y are each independently selected from -(CH₂)ₚ-, -(OCH₂CH₂)ₚ-, and -(CH₂CH₂O)ₚ-;
L₃ is a hydrophilic group;
L₄ is an amino acid group;
R₆ is selected from H, C₁-C₆ alkoxy, -(OCH₂CH₂)ₚO-C₁-C₆ alkyl, and -(CH₂CH₂O)ₚ-C₁-C₆ alkyl;
X is selected from -NH-, -NH(CH₂)ₙC(=O)-, -C(=O)(CH₂)ₙNH-, and -(CH₂)ₙC(=O)-;
Z is selected from C and S;
n is selected from 0, 1 and 2;
p is an integer selected from 1 to 10.

In some embodiments, the functional group capable of reacting with a thiol group in L₁ is selected from maleimide, halogen, halogen-substituted functional groups (such as halogen-substituted aldehyde groups, halogen-substituted -S(O)₂ -), aldehyde groups, alkenyl groups, alkynyl groups, alkanoyl groups, sulfonyl groups, silane groups, isocyanate groups and norbornenyl groups.

In some embodiments, the functional group capable of reacting with a thiol group in L₁ is selected from the group consisting of: 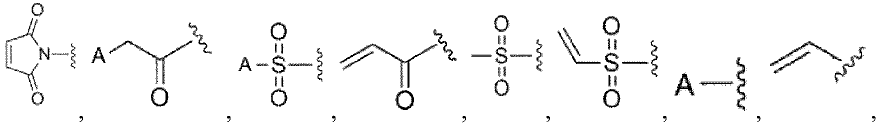 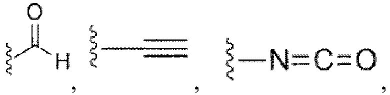 and their derivatives;
wherein: A is a halogen; the wavy line indicates the position where L₁ and L₂ are connected. Preferably, the functional group described in L₁ is:

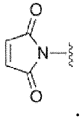

In some embodiments, L₂ is -(CH₂)ₚ-, and p is an integer from 1 to 4; preferably, L₂ is -CH₂CH₂- or -CH₂CH₂CH₂ -.

In some embodiments, X is -NH- or -NH(CH₂)ₙC(=O)-; preferably, X is -NH- or -NHC(=O)-.

In some embodiments, Y is -(CH₂)ₚ-, and p is 1, 2, or 3; preferably, Y is methylene.

In some embodiments, Z is C.

In some embodiments, L₃ is a divalent hydrophilic group with two monovalent radical centers generated by removing two hydrogen atoms from a compound selected from a monosaccharide, a disaccharide, a five- or six-membered saturated heterocyclic ring containing 1-2 hydrogen atoms and a derivative thereof; preferably, the monosaccharide is selected from trioses, tetroses, pentoses, hexoses, and heptoses; preferably, the disaccharide is selected from maltose, sucrose, and lactose; preferably, the five- or six-membered saturated heterocyclic ring containing 1-2 nitrogen atoms or derivative thereof is selected from piperidinyl and pyrrolidinyl.

In some embodiments, the antibody-drug conjugate according to the present invention is characterized in that L₃ is a divalent hydrophilic group with two monovalent radical centers generated by removing two hydrogen atoms from a compound selected from glucose, galactose, mannose, glucuronic acid, galactonic acid, mannuronic acid, N-acetylglucosamine, N-acetylgalactosamine, N-acetylmannosamine and N-acetylmuramic acid.

In some embodiments, the antibody drug conjugate according to the present invention is characterized in that L₃ is a structure represented by the formula L₃-1: 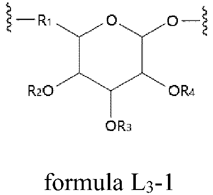 wherein:
R₁ is selected from -O(CH₂)ₚ- and -C(=O)-, p is an integer from 1 to 6, preferably 1, 2, 3 or 4;
R₂, R₃ and R₄ are each independently selected from H, C₁-C₆ alkyl, -(CH₂CH₂O)ₜ-C₁-C₆ alkyl, sulfonic acid group and phosphoric acid group;
t is an integer from 1 to 20;
wavy lines indicate the position where L₃ connects to X and Y.

In some embodiments, L₄ is selected from the following amino acid residues: valine, citrulline, alanine, glycine, phenylalanine, asparagine, glutamic acid, lysine, serine, threonine, cysteine and tyrosine; the amino acid group is optionally substituted by one or more -OR₂₀, wherein R₂₀ is selected from H, C₁-C₆ alkyl, -(CH₂CH₂O)ₜ-C₁-C₆ alkyl, sulfonic acid group and phosphate group, wherein t is an integer from 1 to 20.

In some embodiments, L₄ is selected from the following peptide segments: valine-citrulline (VC), valine-alanine (VA), glycine-glycine-phenylalanine-glycine (GGFG), glycine-glycine-phenylalanine-glycine-glycine, glycine-glycine-phenylalanine-glycine-glycine-glycine, citrulline-valine, alanine-valine, alanine-alanine, citrulline-alanine, asparagine-citrulline, citrulline-asparagine, citrulline-citrulline, phenylalanine-lysine and lysine-phenylalanine, preferably selected from the following peptide segments: valine-citrulline (VC), valine-alanine (VA) and glycine-Glycine-phenylalanine-glycine (GGFG); each peptide segment is optionally substituted by one or more substituents selected from the following group: -OR₂₀ and -(OCH₂CH₂)ₚO-C₁-C₆ alkyl, wherein R₂₀ is selected from H, C₁-C₆ alkyl, -(CH₂CH₂O)ₜ-C₁-C₆ alkyl, sulfonic acid group and phosphate group, t is an integer from 1 to 20, and p is an integer from 1 to 6.

In some embodiments, L₄ is selected from: 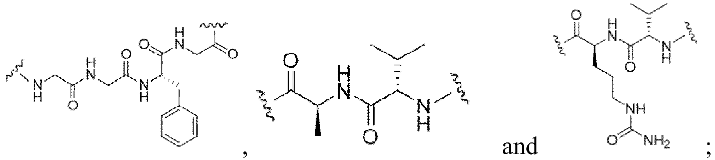 wherein the wavy line indicates the position of attachment of L₄ and rest of formula (A).

In some embodiments, R₆ is H, C₁-C₃ alkoxy or -(OCH₂CH₂)ₚO-C₁-C₃ alkyl, wherein p is an integer from 1 to 6; preferably, R₆ is H, methoxy or -(OCH₂CH₂)ₚOCH₃, wherein p is an integer from 1 to 6 or from 1 to 4.

In some embodiments, the structure of the linker L is as described in any of the following structures:

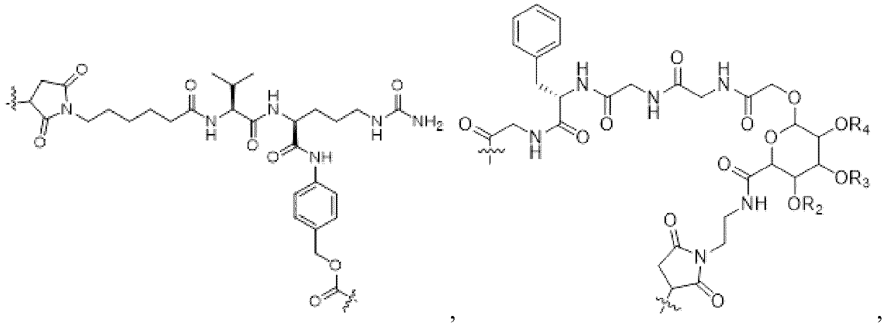

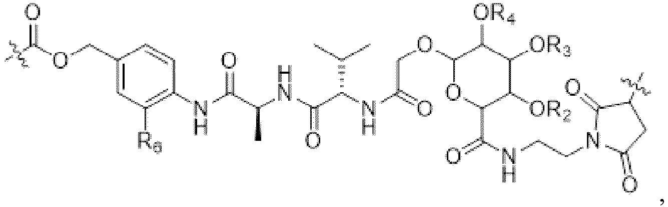

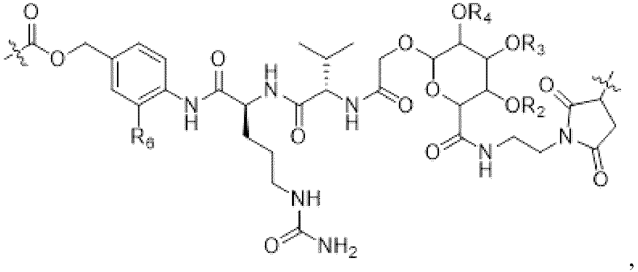

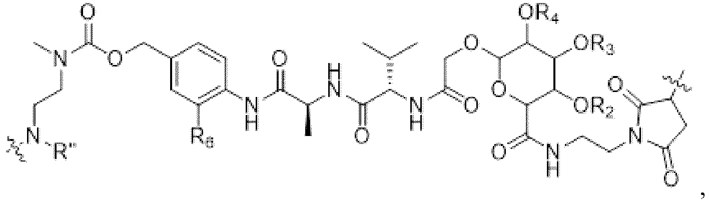

In each formula, R₂, R₃, R₄ and R₆ are as described in any embodiment herein, R" is H or C₁-C₆ alkyl; the wavy line indicates the position where L is attached to the rest of the ADC.

In some embodiments, the linker is selected from maleimidocaproyl-valine-citrulline-p-aminobenzyloxy (mc-vc-PAB), acetyl-lysine-valine-citrulline-p-aminobenzyloxycarbonyl (AcLys-VC-PABC), aminoPEG6-propionyl, and maleimidocaproic acid (MC), maleimidopropionyl (MP), valine-citrulline (val-cit, vc), alanine-phenylalanine (ala-phe), p-aminobenzyloxycarbonyl (PAB), N-succinimidyl 4-(2-pyridylthio)pentanoate (SPP), N-succinimidyl 4-(N-maleimidomethyl)-cyclohexane-1-carboxylate (SMCC), N-succinimidyl (4-iodo-acetyl)aminobenzoate (SIAB), N-succinimidyl-4-(2-pyridyldithio)butyrate (SPDB), and N-succinimidyl 3-(pyridin-2-yldithio)-propionate (SPDP).

In some embodiments, the drug moiety D comprises a cytotoxin, a plant toxin, a small molecule toxin, a radioisotope, etc. In some embodiments, the drug moiety D comprises a tubulin inhibitor, a DNA damaging agent.

In some preferred embodiments, the microtubule inhibitors include dolastatins and auristatins, and maytansines; the DNA damaging agents include calicheamicins, duocarmycins, anthramycin derivative PBD (pyrrolobenzodiazepine), and topoisomerase I inhibitors.

In some preferred embodiments, the auristatin drugs include monomethyl auristatin E (MMAE), monomethyl auristatin F (MMAF), and auristatin F (AF) or derivatives thereof; the maytansine drugs include but are not limited to DM1, DM3, DM4 or derivatives thereof; the topoisomerase I inhibitors include but are not limited to exatecan, topotecan, irinotecan, 9-nitrocamptothecin, and the camptothecin derivative SN-38.

In some embodiments, the drug moiety D may also be a vitamin A precursor, folic acid, etc. The drug moiety D is not limited to the above categories, but also includes all drugs that can be used in ADC.

In some embodiments, the linker-drug (L-D) moiety is selected from the structures represented by the following formulas: VCMMAE, Deruxtecan, CPD2, CPD4, CPD5, CPD6, or SN-38Comp5:

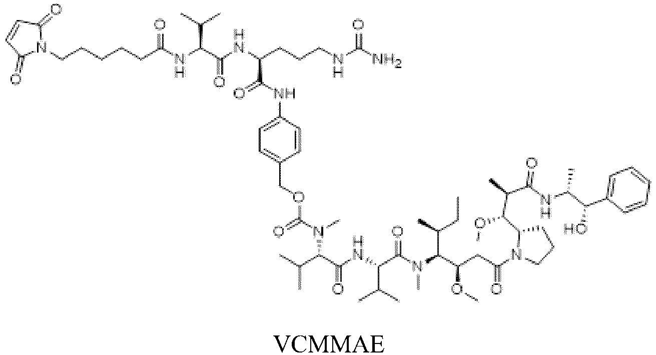

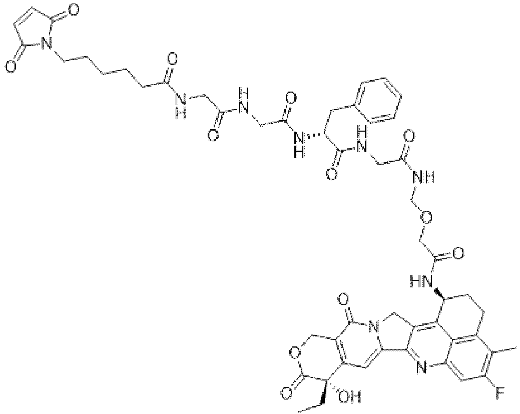

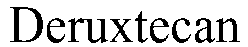

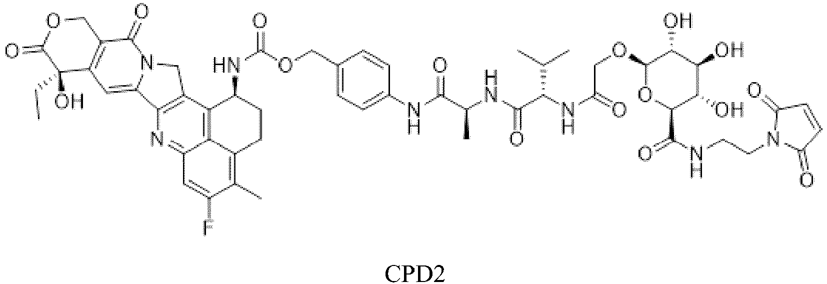

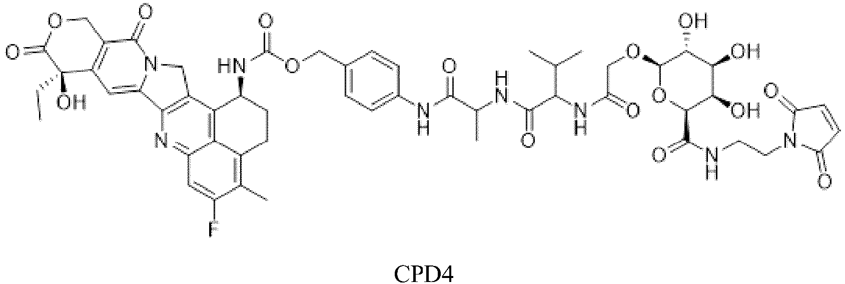

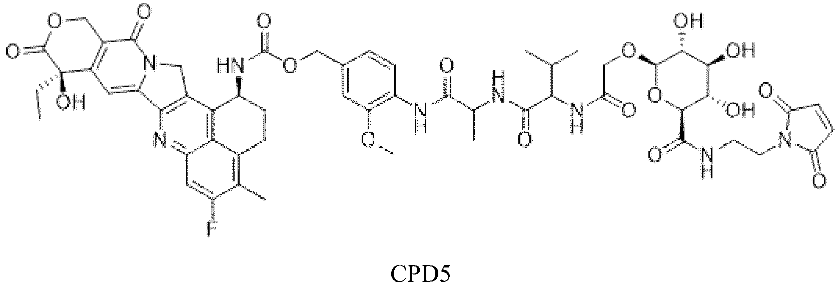

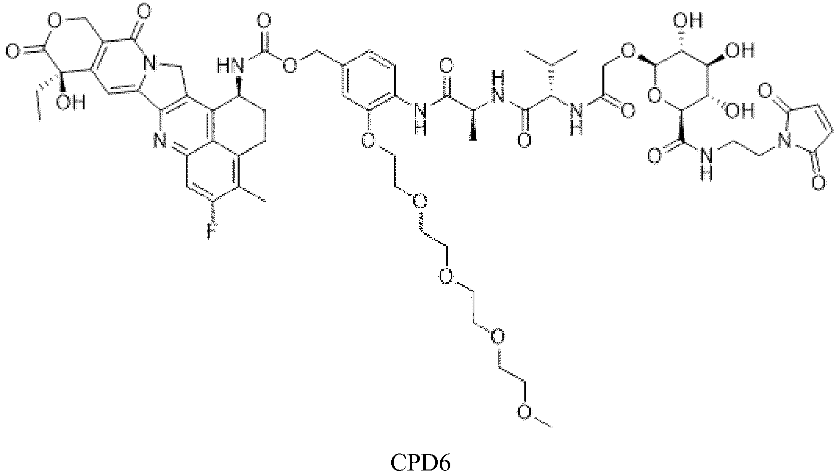

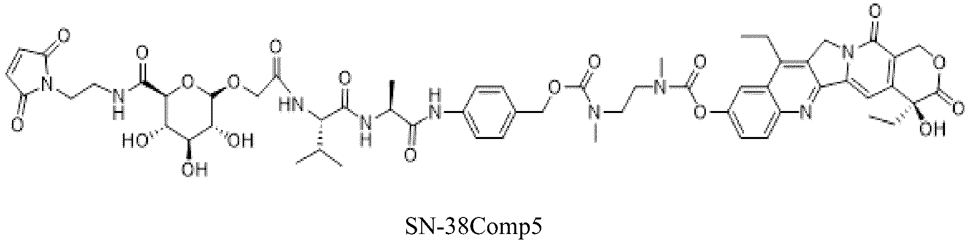

It should be understood that the structures shown above for MMAE, Deruxtecan, CPD2, CPD4, CPD5, CPD6, or SN-38Comp5 are conjugated to Ab via reaction of their maleimide group with a sulfhydryl group of the antibody.

In some embodiments, the antibody-drug conjugate is selected from the structures represented by formulas ADC-I, ADC-II, ADC-III, ADC-IV, ADC-V, ADC-VI, or ADC-VII: 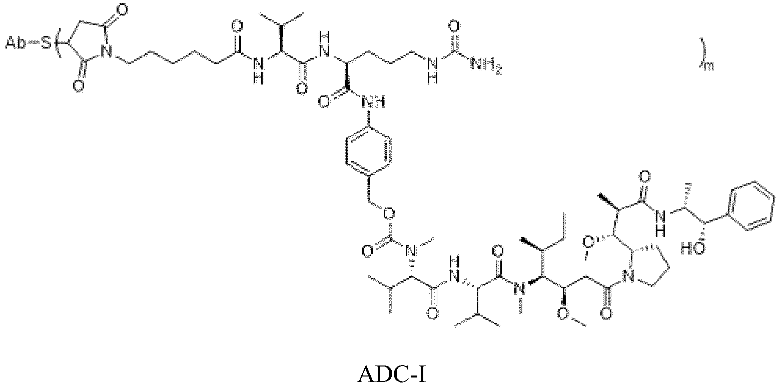 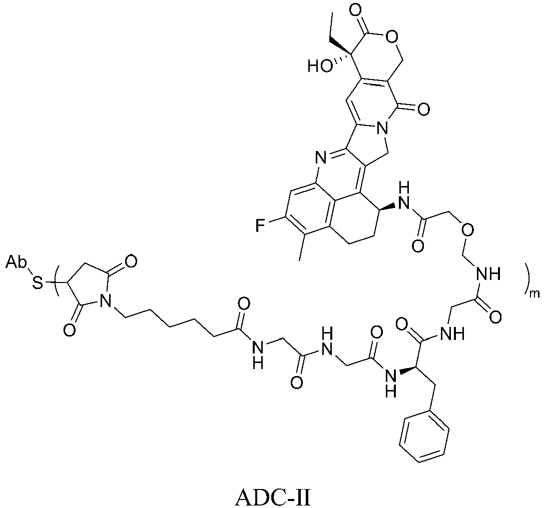 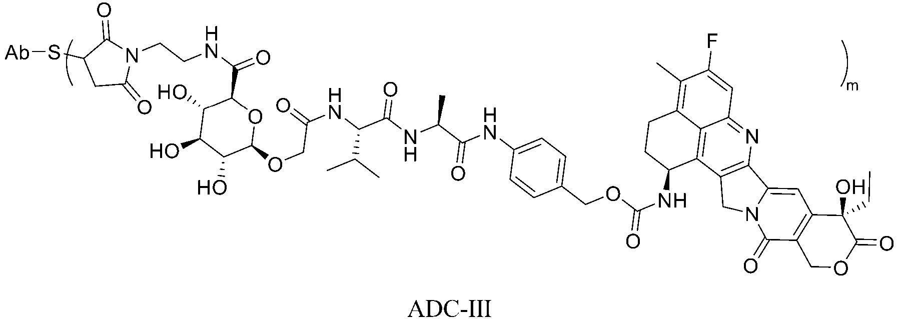 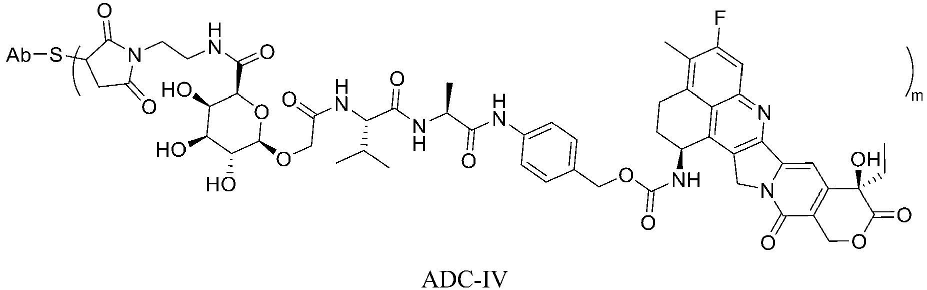 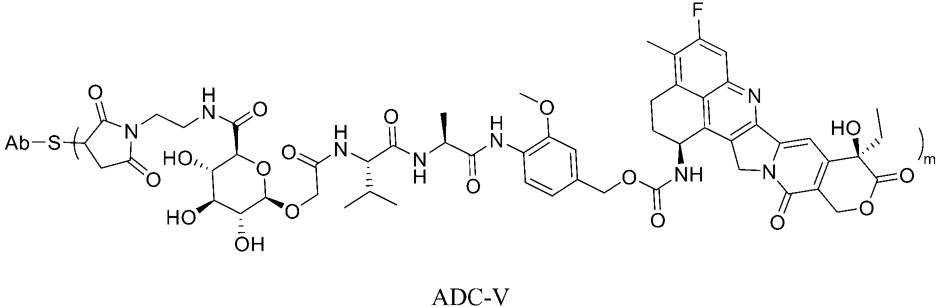 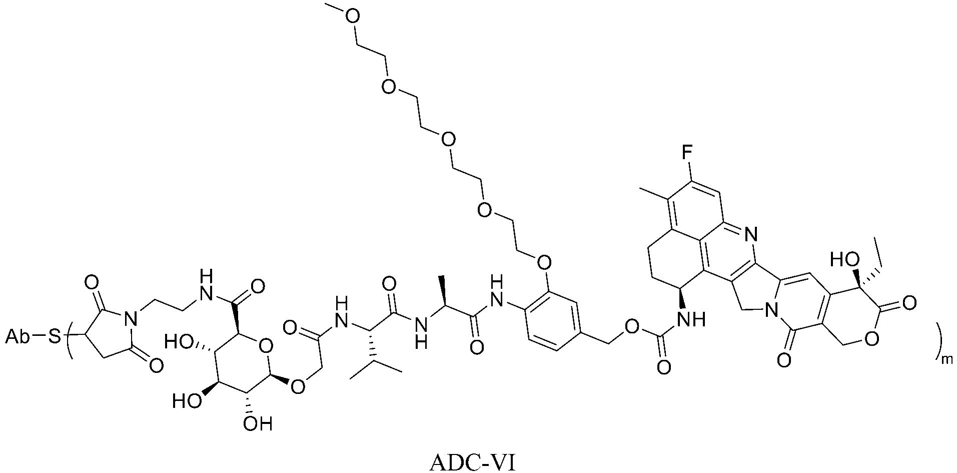 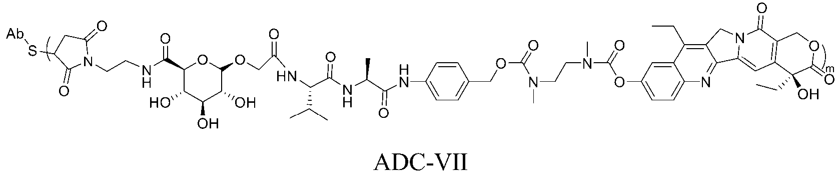 wherein m represents the average number of linker-drug (L-D) units conjugated to Ab, and m ranges from 1 to 8, preferably m is 4; preferably, the amino acid sequence of LCDR1 of the Ab is selected from SEQ ID NO: 37 or 51, the amino acid sequence of LCDR2 is selected from SEQ ID NO: 39 or 53, the amino acid sequence of LCDR3 is selected from SEQ ID NO: 23 or 31, the amino acid sequence of HCDR1 is selected from SEQ ID NO: 17 or 25, the amino acid sequence of HCDR2 is selected from SEQ ID NO: 18 or 45, and the amino acid sequence of HCDR3 is selected from SEQ ID NO: 19 or 27. In some embodiments, the heavy chain variable region of the Ab comprises HCDR1, HCDR2, and HCDR3 having amino acid sequences as shown in SEQ ID NO: 17, SEQ ID NO: 18, and SEQ ID NO: 19, respectively, and the light chain variable region comprises LCDR1, LCDR2, and LCDR3 having amino acid sequences as shown in SEQ ID NO: 37, SEQ ID NO: 39, and SEQ ID NO: 23, respectively; or the heavy chain variable region comprises HCDR1, HCDR2, and HCDR3 having amino acid sequences as shown in SEQ ID NO: 25, SEQ ID NO: 45, and SEQ ID NO: 27, respectively, and the light chain variable region comprises LCDR1, LCDR2, and LCDR3 having amino acid sequences as shown in SEQ ID NO: 51, SEQ ID NO: 52, and SEQ ID NO: 31, respectively. More preferably, the Ab is hu-042-012, hu-042-015, and hu-042-016 as described herein.

It should be understood that in the antibody-drug conjugate of the present invention, the attachment position of the drug moiety D to the remainder of the linker-drug conjugate generally does not affect the biological activity of the drug D itself. Those skilled in the art can readily determine the active site of drug D based on existing techniques and covalently link it to the remainder of the linker-drug conjugate at a position distal to the active site. Covalent linkage methods are well known in the art and include acylamino groups (-NRCO-, where R is H or C1-C4 alkyl), ester bonds (-COO-), and -S-.

In some embodiments, the ADC described herein is selected from ADC-02, ADC-03, ADC-04, ADC-05, ADC-07, ADC-08, ADC-09, ADC-10, ADC-12, ADC-14, ADC-17, ADC-18, ADC-19, ADC-20, and ADC-21 described herein.

The ADCs described herein can be prepared by several routes using organic chemical reactions, conditions, and reagents known to those skilled in the art, including: (1) reacting a nucleophilic group of the antibody with a divalent linker reagent to form an antibody-linker via a covalent bond, followed by reaction with the drug; and (2) reacting a nucleophilic group of the drug moiety with a divalent linker reagent to form a drug-linker via a covalent bond, followed by reaction with a nucleophilic group of the antibody.

### Pharmaceutical composition and pharmaceutical formulation

In another aspect, the present invention provides a pharmaceutical composition comprising an anti-FGFR2b antibody or antigen-binding fragment thereof as described herein, a polynucleotide molecule as described herein, an expression vector as described herein, a host cell as described herein, or an immunoconjugate as described herein, and a pharmaceutically acceptable carrier or excipient. It should be understood that the anti-FGFR2b antibody or pharmaceutical composition thereof provided by the present invention can be incorporated into a formulation with suitable carriers, excipients, and other reagents for combined administration, thereby providing improved transfer, delivery, tolerance, etc.

The term "pharmaceutical composition" refers to a preparation that permits the active ingredient contained therein to exist in biologically effective form, and that contains no additional ingredients that are unacceptably toxic to a subject to which the preparation would be administered.

A pharmaceutical formulation comprising an anti-FGFR2b antibody described herein can be prepared by mixing the anti-FGFR2b antibody of the present invention having the desired purity with one or more optional pharmaceutical excipients (Remington's Pharmaceutical Sciences, 16th edition, Osol, A. ed. (1980)), preferably in the form of an aqueous solution or a lyophilized formulation.

In another aspect, the present invention provides a pharmaceutical combination comprising an antibody or antigen-binding fragment thereof as described herein, a polynucleotide molecule as described herein, an expression vector as described herein, a host cell as described herein, or an immunoconjugate as described herein, and one or more additional therapeutic agents.

### Medical use and treatment method

Any anti-FGFR2b antibody provided herein can be used in therapeutic methods. It should also be understood that when discussing "antibody", compositions comprising the antibody are also included. The anti-FGFR2b antibody of the present invention can be used in therapeutic or prophylactic methods described in any embodiment of the present invention in a therapeutically effective amount or a prophylactically effective amount.

In another aspect, the present invention provides use of the antibody or antigen-binding fragment thereof described herein, polynucleotide molecule described herein, expression vector described herein, host cell described herein, immunoconjugate described herein, or pharmaceutical composition described herein in the manufacture of a medicament for treating and/or preventing an FGFR2b-mediated disease or disorder, preferably the disease or disorder is cancer; more preferably, the cancer is selected from endometrial cancer, ovarian cancer, breast cancer, lung cancer, gastric cancer, esophageal cancer, bladder cancer, and bile duct cancer.

In another aspect, the present invention provides use of the antibody or antigen-binding fragment thereof described herein, polynucleotide molecule described herein, expression vector described herein, host cell described herein, immunoconjugate described herein, or pharmaceutical composition described herein for use in treating and/or preventing an FGFR2b-mediated disease or disorder, preferably the disease or disorder is cancer; more preferably, the cancer is selected from endometrial cancer, ovarian cancer, breast cancer, lung cancer, gastric cancer, esophageal cancer, bladder cancer, and bile duct cancer.

In another aspect, a method for treating and/or preventing an FGFR2b-mediated disease or disorder, comprising administering to a subject in need thereof the antibody or antigen-binding fragment thereof described herein, polynucleotide molecule described herein, expression vector described herein, host cell described herein, immunoconjugate described herein, or pharmaceutical composition described herein in the manufacture of a medicament for treating and/or preventing an FGFR2b-mediated disease or disorder, preferably the disease or disorder is cancer; more preferably, the cancer is selected from endometrial cancer, ovarian cancer, breast cancer, lung cancer, gastric cancer, esophageal cancer, bladder cancer, and bile duct cancer. In some embodiments, the present invention provides a method for treating a disease and/or disorder that requires blocking of FGFR2b, comprising administering to a subject in need thereof an anti-FGFR2b antibody described herein.

In some embodiments, the cancer or tumor described herein can be selected from endometrial cancer, ovarian cancer, breast cancer, lung cancer, gastric cancer, esophageal cancer, bladder cancer, and bile duct cancer.

In some embodiments, the administration of the present invention includes, but is not limited to, oral, intravenous, subcutaneous, intramuscular, intraarterial, intraarticular (e.g., in arthritic joints), by inhalation, aerosol delivery, or intratumoral administration.

### Methods for diagnosis and detection

In another aspect, the present invention provides a method for detecting the presence of FGFR2b in a sample using the antibodies or antigen-binding fragments thereof described herein. The term "detection" as used herein includes quantitative or qualitative detection. In some embodiments, the sample is a biological sample. In certain embodiments, the biological sample is blood, serum, or other liquid samples of biological origin. In certain embodiments, the biological sample comprises cells or tissues. The method includes the steps of contacting the sample with an antibody or antigen-binding fragment thereof described herein or a detection composition containing the antibody or antigen-binding fragment thereof, and detecting the presence of a conjugate or binding signal generated by the binding of the antibody or antigen-binding fragment thereof to FGFR2b. When used for detection purposes, the antibody or antigen-binding fragment thereof described herein may be labeled to indicate whether the conjugate has been formed.

The present invention includes all combinations of the specific embodiments described. Further embodiments of the present invention and the full scope of its applicability will become apparent from the detailed description provided below. However, it should be understood that although the detailed description and specific examples indicate preferred embodiments of the present invention, these descriptions and examples are provided by way of illustration only, as various changes and modifications within the spirit and scope of the present invention will become apparent to those skilled in the art from this detailed description. All publications, patents, and patent applications cited herein, including citations, are incorporated herein by reference in their entirety for all purposes.

The compounds of the present invention can be prepared by a variety of synthetic methods well known to those skilled in the art, including the specific embodiments listed below, embodiments formed by combining them with other methods, and equivalent alternatives known to those skilled in the art. Preferred embodiments include but are not limited to the examples of the present invention.

### EXAMPLE

The present invention is illustrated by the following examples, but is not intended to be limiting thereof. The present invention has been described in detail herein, and specific embodiments thereof are disclosed. It will be apparent to those skilled in the art that various changes and modifications can be made to the specific embodiments of the present invention without departing from the spirit and scope of the invention.

### Example 1: Preparation of recombinant FGFR2b extracellular domain protein

The recombinant protein Human FGFR2b-His used for mouse immunization or antibody activity detection was purchased from Kactus Biosciences (Cat# Kactus-FGR-HM1BB), and the protein comprises Arg152-Glu378 (Accession # P21802-3). The recombinant protein Cyno FGFR2b-His used for antibody cross-species binding activity detection was purchased from Kactus Biosciences (Cat# Kactus-FGF-CM1BB), and this protein comprises Pro154-Lys368 (Accession # A0A2K5TL84-1); the recombinant protein mouse FGFR2b-His used for antibody cross-species binding activity detection was purchased from Kactus Biosciences (Cat# Kactus-FGF-MM1BB), and this protein comprises Pro39-Glu263 (Accession # P21803-2); the recombinant protein human FGFR2c-His used for antibody specificity detection was purchased from Kactus Biosciences (Cat# Kactus-FGR-HM1BC), and this protein comprises Arg152-Glu377 (Accession # P21802-1).

### Example 2: Preparation of mouse hybridoma cells

### 2.1 Mouse Immunization

Balb/c, A/J, or C57 mice were immunized with the purchased human FGFR2b-His recombinant protein as an antigen. After the primary immunization (intraperitoneal injection after emulsification with Freund's complete adjuvant, 50 µg/mouse), booster immunizations (intraperitoneal injection after emulsification with Freund's incomplete adjuvant, 25 µg/mouse ) were performed every other week. Blood was collected after the second booster immunization, and serum antibody titers and specificity against the immunogen were determined using ELISA and FACS.

### 2.2 Cell fusion

On the fourth day after the final booster immunization (without emulsification, intraperitoneal injection, 50 µg/mouse),, mouse spleens were were removed and triturated in DPBS. The spleen trituration solution was collected in a 50 ml centrifuge tube (Corning), and the erythrocytes were lysed to obtain a splenocyte suspension. Splenocytes were fused with mouse myeloma Sp2/0 cells using a conventional electrofusion method. The fusion product was adjusted to an appropriate density and cultured in DMEM (Gibco) medium containing 20% fetal bovine serum (Gibco) and 1× HAT (hypoxanthine, methotrexate, and thymidine) in an incubator at 5% CO₂, 130°C. A half-medium change was performed on the seventh day of culture. On the tenth day, hybridoma supernatants were screened by ELISA to identify clones that positively bound to human FGFR2b-His and cyno FGFR2b-His, but negatively bound to human FGFR2c-His. After obtaining the target clones, flow cytometry was performed to select clones that were positive for binding to CHO-K1-huFGFR2b cells (prepared by the inventors, Met1-Thr822, Accession # P21802-3) and negative for binding to CHO-K1-huFGFR2c cells (prepared by the inventors, Met1-Thr821, Accession # P21802-1). In addition, flow cytometry was used to screen clones with FGF10 (Cat# Kactus-FGF-HE010B)/FGFR2b blocking activity. Next, the positive clones that met the above screening conditions were subcloned by limiting dilution and cultured in DMEM medium containing 10% fetal bovine serum (Gibco). ELISA was used on the 10th day after subcloning, and the positive clones were selected and expanded to 24-well plates for further culture. After 3 days, cell binding activity was verified by FACS. The best clone was selected based on the test results, cultured in DMEM (Gibco) medium containing 10% fetal bovine serum, 5% CO₂ , 37°C incubator, and frozen in liquid nitrogen for subsequent sequencing.

### Example 3: Screening of mouse hybridoma cells

Based on the titer detection of blood samples collected after mouse immunization, mice with high titers were selected for 7 rounds of fusion. Among 33,600 different polyclonal hybridoma cells, following the method and screening criteria described in Example 2.2, 58 hybridoma cells that specifically bound to FGFR2b and had ligand blocking activity were selected for sequencing of the antibody heavy and light chain variable regions.

### Example 4: Determination of variable region sequences of murine antibodies (according to Kabat or IMGT)

Positive clones were transferred from 24-well plates (Corning) to T75 culture flasks (Corning). After the cells expanded to 70-90% confluence, they were frozen and then stored on dry ice and sent to GenScript for mouse antibody variable region sequencing. For NGS sequencing, RNA was extracted and purified, reverse transcribed into cDNA, and the cDNA was used as a template for two rounds of PCR to amplify the positive bands of heavy chain/light chain. After sequencing, the paired heavy chain and light chain sequencing results were analyzed, and the murine antibody variable region sequences were finally obtained.

### Example 5: Construction of chimeric antibodies

After sequencing the mouse antibody light and heavy chain variable regions, the gene fragments were synthesized in GenScript. The heavy and light chain variable region fragments were respectively cloned into plasmids containing the human heavy chain constant region Fc or light chain constant region κ sequence, and correct clones were confirmed by sequencing. Transient transfection and purification of the antibodies were performed by GenScript, yielding chimeric antibodies with purity greater than 90% as determined by SDS-PAGE and SEC, which were used for subsequent activity assays.

### Example 6: Screening of chimeric antibodies

### 6.1 Binding activity of chimeric antibodies to CHO-K1-huFGFR2b/huFGFR2c cells

CHO-K1-huFGFR2b (CHO-K1 cells overexpressing human FGFR2b, constructed by the inventors) or CHO-K1-huFGFR2c (CHO-K1 cells overexpressing human FGFR2c, constructed by the inventors) cells in T75 culture flasks (Corning) were digested and counted, and the cell density was adjusted to 1E6/mL, and 100 µL per well of cell suspension was added to a 96-well U-bottom plate (Corning). The anti-FGFR2b chimeric antibodies constructed in Example 5, positive controls (Bemarituzumab/Bema and Aprutumab/Apru), and negative control (anti-KLH IgG1) were diluted in FACS Buffer (2% FBS/PBS) (starting from 20 µg/mL or 10 µg/mL, 3-fold serial dilution, total of 12 or 8 dilutions). Cells were resuspended in 100 µL per well of the dilution and incubated at 4°C for 1 hour. After washing three times with FACS buffer, the secondary antibody Alexa fluor 488 F(ab')₂ Fragment Goat Anti-Human IgG, Fcγ fragment specific (Cat# Jackson immunoresearch-109-546-098) was diluted 1:1000, and cells were resuspended in 100 µL per well of the dilution and incubated at 4°C in the dark for 30 min. After washing twice with FACS buffer, cells were resuspended in 50 µL per well of FACS buffer , and the median fluorescence intensity (MFI) was measured using an Intellicyt iQue3 (Satorius). Curve fitting analysis of cell binding activity was performed using GraphPad Prism 9 software. The EC50 and upper plateau values are shown in Table 3 (CHO-K1-huFGFR2b) and Table 4 (CHO-K1-huFGFR2c). Among them, Bemarituzumab showed high affinity and specific binding to FGFR2b, while Aprutumab bound to both FGFR2b and FGFR2c with weaker binding to FGFR2b. The results in Table 3 indicated that Chi-004, Chi-013, Chi-019, Chi-038, Chi-044, Chi-045, Chi-046, Chi-047, Chi-048, Chi-049, Chi-051, Chi-052, Chi-053, Chi-054, Chi-055, Chi-056, Chi-057, and Chi-058 showed relatively weak binding to CHO-K1-huFGFR2b; the results in Table 4 showed that Chi-027 binds weakly to CHO-K1-huFGFR2c and would not be further studied.

**Table 3: Binding activity of chimeric antibodies to CHO-Kl-huFGFR2b cells**

| CHO-K1-huFGFR2b FACS binding | Relative EC50 (µg/ml) | Plateau (MFI) | CHO-K1-huFGFR2b FACS binding | Relative EC50 (µg/ml) | Plateau (MFI) |
|---|---|---|---|---|---|
| Chi-001 | 0.14 | 140339 | Chi-010 | 0.16 | 144398 |
| Chi-002 | 0.10 | 142392 | Chi-0 11 | 0.06 | 137819 |
| Chi-003 | 0.04 | 154885 | Chi-012 | 0.06 | 151993 |
| Chi-004 | 0.88 | 144259 | Chi-013 | 9.00E+40 | 91843 |
| Chi-005 | 0.11 | 156758 | Chi-014 | 0.03 | 154835 |
| Chi-006 | 0.29 | 152742 | Chi-015 | 0.13 | 153352 |
| Chi-008 | 0.17 | 149997 | Chi-016 | 0.08 | 146929 |
| Chi-009 | 0.08 | 145694 | Chi-017 | 0.04 | 153949 |
| N/A | N/A | N/A | Chi-018 | 0.05 | 154584 |
| *Aprutumab* | *0.23* | *52762* | *Aprutumab* | *0.24* | *64441* |
| *Bemarituzumab* | *0.08* | *144716* | *Bemarituzumab* | *0.06* | *151173* |
| *anti-KLH IgG1* | *152647.00* | *10779* | *anti-KLH IgG1* | *1880.00* | *11550* |
| Chi-019 | 0.41 | 148760 | Chi-032 | 0.07 | 150650 |
| Chi-020 | 0.12 | 140769 | Chi-033 | 0.01 | 153482 |
| Chi-021 | 0.08 | 149097 | Chi-034 | 0.01 | 146877 |
| Chi-022 | 0.08 | 145586 | Chi-035 | 0.01 | 142101 |
| Chi-023 | 0.07 | 137115 | Chi-036 | 0.07 | 138371 |
| Chi-024 | 0.09 | 145691 | Chi-037 | 0.03 | 135699 |
| Chi-025 | 0.17 | 147529 | Chi-038 | 3145.00 | 25533 |
| Chi-026 | 0.08 | 146003 | Chi-039 | 0.15 | 135064 |
| Chi-027 | 0.11 | 147298 | Chi-040 | 0.00 | 149447 |
| *Aprutumab* | *0.12* | *49876* | *Aprutumab* | *0.14* | *44106* |
| *Bemarituzumab* | *0.02* | *165131* | *Bemarituzumab* | *0.03* | *150000* |
| *anti-KLH IgG1* | *2.40* | *10665* | *anti-KLH IgG1* | *732021.00* | *9302* |
| Chi-028 | 0.10 | 144921 | Chi-041 | 0.02 | 147040 |
| Chi-029 | 0.15 | 145237 | Chi-042 | 0.02 | 137260 |
| Chi-030 | 0.11 | 142087 | Chi-043 | 0.04 | 150564 |
| Chi-031 | 0.18 | 147554 | Chi-044 | 328.50 | 43841 |
| *Aprutumab* | *0.23* | *46971* | Chi-045 | 0.43 | 129411 |
| *Bemarituzumab* | *0.09* | *150148* | *Aprutumab* | *0.21* | *41588* |
| *anti-KLH IgG1* | *11.13* | *10299* | *Bemarituzumab* | *0.06* | *152638* |
| Chi-051 | 0.66 | 1004765 | *anti-KLH IgG1* | *0.03* | *8937* |
| Chi-052 | 0.62 | 978981 | Chi-046 | 0.13 | 152962 |
| Chi-053 | 0.58 | 987786 | Chi-047 | 0.73 | 144719 |
| Chi-054 | 0.31 | 945174 | Chi-048 | 1.31 | 78876 |
| Chi-055 | 0.40 | 872372 | Chi-049 | 1.84 | 103176 |
| Chi-056 | 0.80 | 773930 | *Aprutumab* | *0.02* | *167106* |
| Chi-057 | 0.17 | 972122 | *Bemarituzumab* | *0.04* | *330685* |
| Chi-058 | 0.26 | 967216 | *anti-KLH IgG1* | *0.00* | *10806* |
| *Aprutumab* | *0.03* | *393164* | - | - | - |
| *Bemarituzumab* | *0.11* | *1007459* | - | - | - |
| *anti-KLH IgG1* | *0.05* | *8466* | - | - | - |

**Table 4: Binding activity of chimeric antibodies to CHO-Kl-huFGFR2c cells**

| CHO-K1-huFGFR2c FACS binding | Plateau (MFI) | CHO-K1-huFGFR2c FACS binding | Plateau (MFI) |
|---|---|---|---|
| Chi-001 | 14053 | Chi-010 | 14380 |
| Chi-002 | 14705 | Chi-011 | 15272 |
| Chi-003 | 15334 | Chi-012 | 14694 |
| Chi-004 | 15101 | Chi-013 | 15597 |
| Chi-005 | 14770 | Chi-014 | 14220 |
| Chi-006 | 14594 | Chi-015 | 15342 |
| Chi-008 | 14415 | Chi-016 | 13721 |
| Chi-009 | 17019 | Chi-017 | 13525 |
| *Aprutumab* | *812218* | Chi-018 | 25735 |
| *Bemarituzumab* | *13729* | *Aprutumab* | *705664* |
| *anti-KLH IgG1* | *17244* | *Bemarituzumab* | *14909* |
| N/A | N/A | *anti-KLH IgG1* | *17775* |
| Chi-019 | 13561 | Chi-032 | 13022 |
| Chi-020 | 14249 | Chi-033 | 13185 |
| Chi-021 | 14866 | Chi-034 | 13127 |
| Chi-022 | 14751 | Chi-035 | 13175 |
| Chi-023 | 16605 | Chi-036 | 13751 |
| Chi-024 | 15574 | Chi-037 | 20233 |
| Chi-025 | 13721 | Chi-038 | 38037 |
| Chi-026 | 14914 | Chi-039 | 13129 |
| Chi-027 | 172375 | Chi-040 | 14439 |
| *Aprutumab* | *775400* | *Aprutumab* | *632747* |
| *Bemarituzumab* | *13167* | *Bemarituzumab* | *16017* |
| *anti-KLH IgG1* | *17503* | *anti-KLH IgG1* | *13473* |
| Chi-028 | 16649 | Chi-041 | 13447 |
| Chi-029 | 15212 | Chi-042 | 14680 |
| Chi-030 | 15007 | Chi-043 | 13794 |
| Chi-031 | 15804 | Chi-044 | 17973 |
| *Aprutumab* | *762540* | Chi-045 | 13439 |
| *Bemarituzumab* | *15614* | *Aprutumab* | *599621* |
| *anti-KLH IgG1* | *18147* | *Bemarituzumab* | *13132* |
| - | - | *anti-KLH IgG1* | *14120* |

### 6.2 Binding activity of chimeric antibodies to huFGFR1b-Fc, huFGFR3b-his, and huFGFR4-his

Antigens huFGFR1b-Fc (Cat# Sino Biological-16482-H02H), huFGFR3b-His (Cat# Sino Biological-16486-H08H), and huFGFR4-His (Cat# Sino Biological-10538-H08H) were each diluted to 2 µg/mL with DPBS (Gibco), and 50 µL per well of the dilution was added to a 96-well high-binding ELISA plate (Corning) and incubated overnight at 4°C. The next day, the plate was washed three times with wash buffer (0.05% Tween20/DPBS), and blocking buffer (PBS containing 5% non-fat dry milk) was added to block for 1 hour at room temperature. After washing three times with wash buffer, the anti-FGFR2b chimeric antibodies, positive control (Bemarituzumab), and negative control (anti-KLH IgG1) were diluted in ELISA Buffer (PBS containing 1% non-fat dry milk) (starting from 20 µg/mL or 60 µg/mL, 3-fold serial dilution, 8 dilutions), and 100 µL per well of the dilution was added to the 96-well plate and incubated for 1 hour at room temperature. After washing three times with wash buffer, 50 µL per well of 1:5000 diluted secondary antibody Goat anti-human IgG(Fab) HRP (Cat# Sigma-A0293) was added and incubated for 30 minutes at room temperature. After washing three times, 50 µL per well of TMB substrate was added and incubated for 2-3 minutes at room temperature, followed by addition of 50 µL per well of 1N HCl stop solution. OD450 values were measured using a multi-function microplate reader (Thermo Scientific, Multiskan FC). The binding activity results of chimeric antibodies to FGFR1b protein are shown in Table 5, and to FGFR3b and FGFR4 proteins in Table 6. Compared to IgG, bemarituzumab weakly bound to FGFR1b and did not bind to FGFR3b or FGFR4. Chi-006, Chi-018, and Chi-032 weakly bound to FGFR1b but not to FGFR3b or FGFR4, while Chi-042 weakly bound to FGFR1b and FGFR3b at high concentrations but did not bind to FGFR4.

**Table 5: Binding of chimeric antibody to FGFR1b by ELISA**

| Antibody name | FGFR1b binding plateau (OD450) | Antibody name | FGFR1b binding plateau (OD450) |
|---|---|---|---|
| Chi-001 | 2.69 | Chi-020 | 3.38 |
| Chi-005 | 3.19 | Chi-021 | 3.49 |
| Chi-006 | 1.61 | Chi-023 | 3.72 |
| Chi-008 | 2.21 | Chi-024 | 3.56 |
| Chi-010 | 2.21 | Chi-025 | 3.33 |
| Chi-012 | 3.36 | Chi-026 | 3.77 |
| Chi-015 | 3.27 | Chi-028 | 3.77 |
| Chi-016 | 3.04 | Chi-029 | 3.37 |
| *Bemarituzumab* | *0.47* | Chi-031 | 3.55 |
| *IgG* | *0.08* | Chi-034 | 3.46 |
| N/A | N/A | *Bemarituzumab* | *1.47* |
| Chi-002 | 2.86 | *IgG* | *0.15* |
| Chi-003 | 3.13 | Chi-035 | 3.09 |
| Chi-009 | 2.93 | Chi-037 | 3.26 |
| Chi-011 | 3.10 | Chi-040 | 3.44 |
| Chi-014 | 3.22 | Chi-042 | 1.72 |
| Chi-017 | 2.83 | *Bemarituzumab* | *1.96* |
| Chi-018 | 0.96 | *IgG* | *0.09* |
| Chi-022 | 3.06 | Chi-032 | 1.64 |
| Chi-030 | 2.94 | Chi-033 | 2.76 |
| *Bemarituzumab* | *0.94* | Chi-036 | 1.56 |
| *IgG* | *0.06* | Chi-039 | 2.90 |
| - | - | Chi-041 | 3.16 |
| - | - | Chi-043 | 2.99 |
| - | - | *Bemarituzumab* | *1.24* |
| - | - | *IgG* | *0.07* |

**Table 6: Binding of chimeric antibody to FGFR3b and FGFR4 by ELISA**

| Antibody name | FGFR3b binding plateau (OD450) | FGFR4 binding plateau (OD450) |
|---|---|---|
| Chi-001 | 0.36 | 0.07 |
| Chi-002 | 2.41 | 0.10 |
| Chi-003 | 1.46 | 0.10 |
| Chi-005 | 2.59 | 0.10 |
| Chi-006 | 0.32 | 0.06 |
| Chi-008 | 0.92 | 0.16 |
| Chi-009 | 0.24 | 0.07 |
| Chi-010 | 0.29 | 0.06 |
| Chi-011 | 0.62 | 0.06 |
| *Bemarituzumab* | *0.29* | *0.08* |
| *IgG* | *0.30* | *0.07* |
| Chi-012 | 0.41 | 0.11 |
| Chi-014 | 0.82 | 0.11 |
| Chi-015 | 0.37 | 0.09 |
| Chi-016 | 0.45 | 0.08 |
| Chi-017 | 0.49 | 0.11 |
| Chi-018 | 0.31 | 0.09 |
| Chi-020 | 0.28 | 0.09 |
| Chi-021 | 0.55 | 0.10 |
| Chi-022 | 0.30 | 0.08 |
| Chi-023 | 0.69 | 0.10 |
| *Bemarituzumab* | *0.28* | *0.09* |
| *IgG* | *0.29* | *0.09* |
| Chi-024 | 0.27 | 0.08 |
| Chi-025 | 0.25 | 0.06 |
| Chi-026 | 0.24 | 0.06 |
| Chi-028 | 0.72 | 0.09 |
| Chi-029 | 1.54 | 0.06 |
| Chi-030 | 1.68 | 0.28 |
| Chi-031 | 0.40 | 0.11 |
| Chi-032 | 0.20 | 0.07 |
| Chi-033 | 0.29 | 0.07 |
| Chi-034 | 0.71 | 0.09 |
| *Bemarituzumab* | *0.24* | *0.08* |
| *IgG* | *0.26* | *0.08* |
| Chi-035 | 1.09 | 0.10 |
| Chi-036 | 0.31 | 2.61 |
| Chi-037 | 0.41 | 0.08 |
| Chi-040 | 1.92 | 0.18 |
| Chi-041 | 2.14 | 0.16 |
| Chi-042 | 1.80 | 0.29 |
| Chi-043 | 0.32 | 0.12 |
| *Bemarituzumab* | *0.26* | *0.10* |
| *IgG* | *0.26* | *0.07* |

### 6.3 Binding activity of chimeric antibodies to huFGFR1c-His and huFGFR3c-His

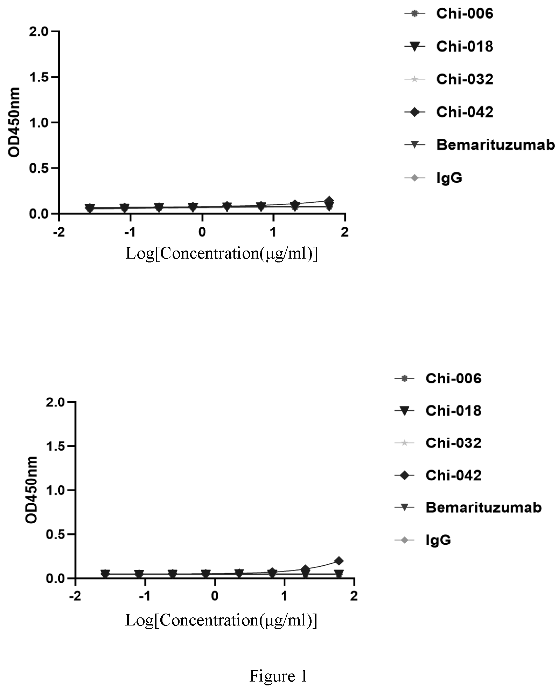

Antigens huFGFR1c-His (Cat# Sino Biological-10616-H08H) and huFGFR3c-His (Cat# Sino Biological-16044-H08H) were each diluted to 2 µg/mL with DPBS (Gibco), and 50 µL per well of the dilution was added to a 96-well high-binding ELISA plate (Corning) and incubated overnight at 4°C. Following the experimental method described in Example 6.2, the binding activity of anti-FGFR2b chimeric antibodies (Chi-006, Chi-018, Chi-032, Chi-042), positive control (Bemarituzumab), and negative control (anti-KLH IgG1) to huFGFR1c-His and huFGFR3c-His was detected. The results are shown in Figure 1. Chi-006, Chi-018, Chi-032, Chi-042, and Bemarituzumab did not bind to huFGFR1c-His or huFGFR3c-His proteins.

### 6.4 Binding activity of chimeric antibodies to cyno FGFR2b-His and mouse FGFR2b-His

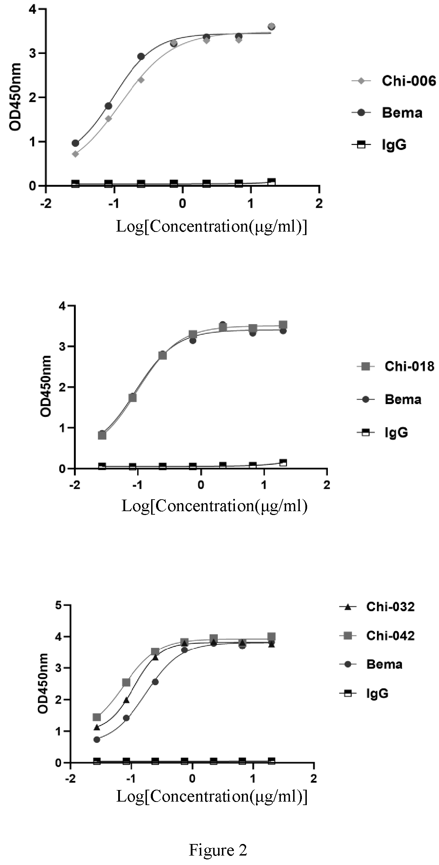

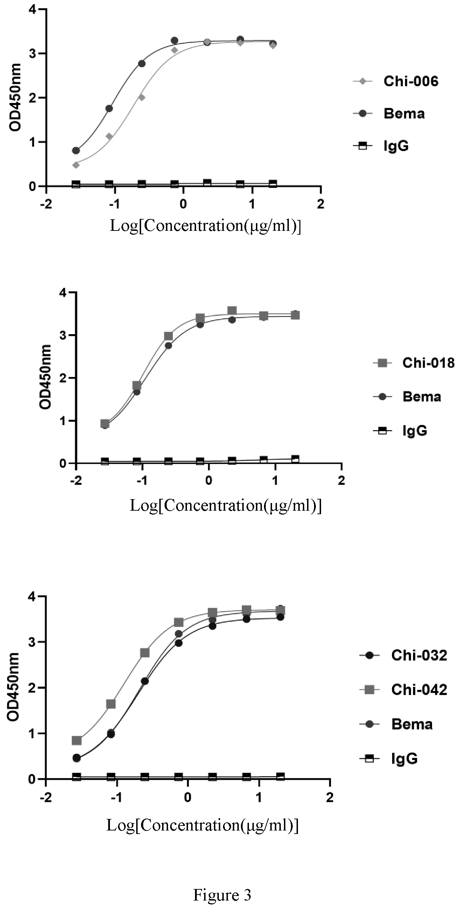

Antigens cyno FGFR2b-His (Cat# Kactus-FGF-CM1BB) and mouse FGFR2b-His (Cat# Acro-FGB-M52H5) were each diluted to 2 µg/mL with DPBS (Gibco), and 50 µL per well of the dilution was added to a 96-well high-binding ELISA plate (Corning) and incubated overnight at 4°C. Following the experimental method described in Example 6.2, the cross-species binding activity of anti-FGFR2b chimeric antibodies (Chi-006, Chi-018, Chi-032, Chi-042) was detected, with Bemarituzumab (Bema) as a positive control. The results are shown in Figures 2 and 3. Chi-006, Chi-018, Chi-032, Chi-042, and Bema all exhibited good cross-binding activity to cyno FGFR2b and mouse FGFR2b.

### 6.5 Ligand-blocking activity of chimeric antibodies

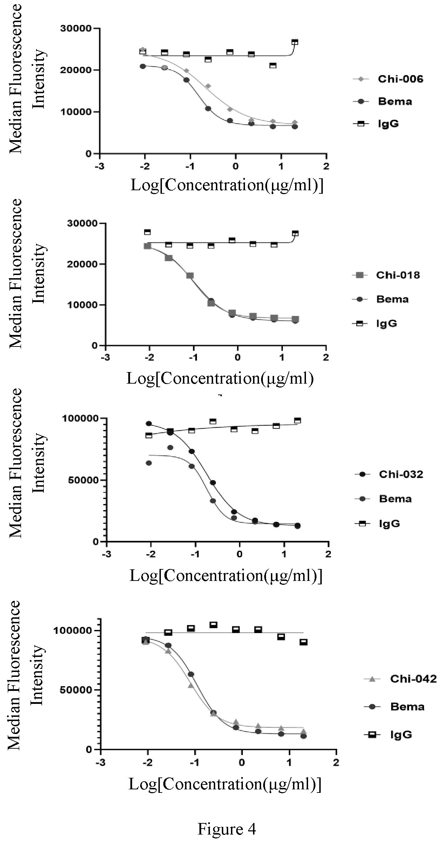

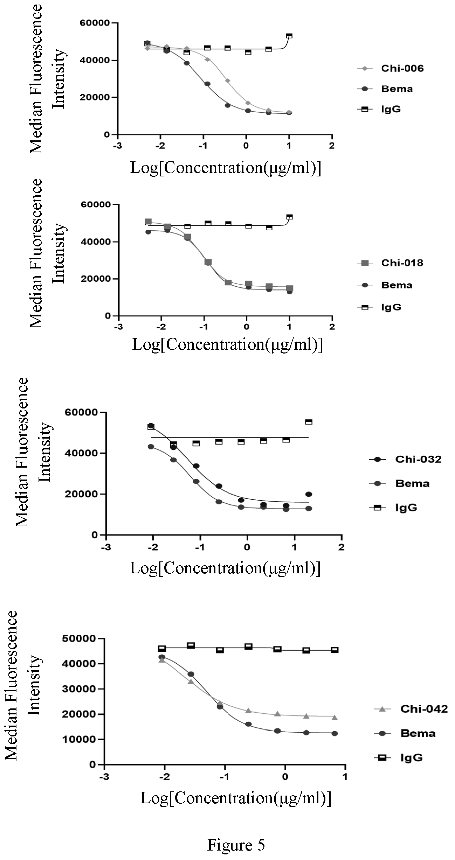

CHO-K1-huFGFR2b (S252W) or CHO-K1-huFGFR2b cells in T75 culture flasks (Corning) were digested and counted, and the cell density was adjusted to 1E6/mL, 100 µL per well of the cells was added to a 96-well U-bottom plate (Corning). The anti-FGFR2b chimeric antibodies (Chi-006, Chi-018, Chi-032, Chi-042), positive control (Bema), and negative control (anti-KLH IgG1) were diluted in FACS Buffer (starting from 10 µg/mL or 20 µg/mL, 3-fold serial dilution). Cells were resuspended in 100 µL per well of diluted antibody and incubated at 4°C for 1 hour. After washing three times with FACS buffer (2% FBS/PBS), 0.2 µg/mL FGF7-biotin (Cat# Kactus-FGF-HE101B) or 0.1 µg/mL FGF10-biotin (Cat# Kactus-FGF-HE010B) was added and incubated at 4°C in the dark for 1 hour. After washing three times with FACS buffer, 1:1000 diluted SA-PE (Cat# BD-554061) was added and incubated at 4°C in the dark for 30 minutes. After washing twice with FACS buffer, cells were resuspended in 50 µL FACS buffer per well, and the median fluorescence intensity (MFI) was measured using an Intellicyt iQue3 (Satorius). Curve fitting analysis was performed using GraphPad Prism 9 software. The results are shown in Figures 4 and 5. All candidate antibodies effectively blocked the interaction of the ligand FGF7 or FGF10 with the receptor FGFR2b.

### 6.6 ADCC activity of chimeric antibodies

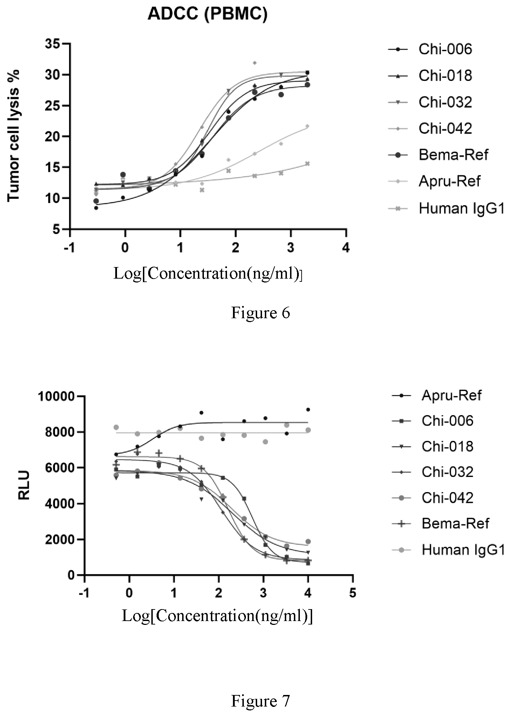

PBMC cells were thawed, adjusted to a density of 2E6-4E6/mL with RPMI 1640 medium (Gibco, Cat# A1049101), placed in T75 culture flasks, and incubated overnight at 37°C in a 5% CO₂ incubator. Target KATO3 cells were collected, washed once with PBS, and labeled with Cell Trace Far Red (Invitrogen, Cat# C34564) according to the manufacturer's instructions. The cells were stained at 37°C for 20 min, then 5 volumes of RPMI 1640 were added and incubated at 37°C for 5 minutes to stop the staining. 3E5 PBMC cells per well (50 µL) and 1.5E4 KATO3 cells per well (50 µL) were added to a 96-well U-bottom plate (Corning). The candidate antibodies were diluted in medium (starting from 60 µg/mL, 5-fold serial dilution), and 20 µL per well of antibody was added to the cells, and incubated for 4 hours in a 37°C incubator. 80 µL per well of buffer (PBS+2% FBS) was added, the plate was centrifuged at 1200 rpm for 5 minutes, and the supernatant was discarded. Cells were washed once with 200 µL per well of buffer, then 100 µL per well of PI (Sigma, Cat# P4864) was added and incubated at 4°C for 5 minutes. Target cell viability was measured using a Fortessa flow cytometer. The results are shown in Figure 6. The results showed that all candidate chimeric antibodies exhibited strong ADCC activity, which was similar to that of the control antibody Bema-Ref, while the other control antibody Apru-Ref showed weaker ADCC activity.

### 6.7 Cell proliferation inhibitory activity of chimeric antibody

FGFR2b/BaF3 cells in the logarithmic growth phase (FGFR2b overexpressed in Baf3 cells, constructed by GenScript, culture medium: RPMI 1640 + 10% FBS + 10 ng/ml IL-NER134 (Sino biological, Cat#51066-MNAH)) were collected and the cells were washed three times with IL₃-free RPMI 1640 medium to remove IL₃ from the original medium. The cells were adjusted to an appropriate density, and 3000 cells in 100 µL per well were added into a 96-well black flat-bottom clear plate (Corning, Cat#3340). 50 µL of a mixture of 100 ng/mL FGF7 (Peprotech, Cat#100-19-10ug) and 40 µg/mL Heparin (Selleck, Cat#S1346) was added to each well. The candidate antibodies were diluted in medium (starting from 40 µg/mL, 3-fold dilution), and 50 µL per well of the antibodies was added to the 96-well plate, and cultured at 37°C for 5 days. After 5 days, the cells in the 96-well plate were mixed, and 70 µL per well of the cells was transferred to a new 96-well plate. 70 µL per well of CellTiter-Glo reagent (Promega, Cat# G7572) was added, and the chemiluminescence signal was measured using a multi-function microplate reader (Molecular Devices, SpectraMax M5). The parameter regression curves were fitted using GraphPad Prism software to calculate EC50 values. The results are shown in Figure 7. All candidate chimeric antibodies effectively inhibited the proliferation of FGFR2b/BaF3 cells in vitro, with inhibitory activity similar to that of the control antibody Bemarituzumab (Bema-Ref), while the other control antibody Aprutumab (Apru-Ref) showed no cell proliferation inhibitory activity.

### 6.8 ERK phosphorylation inhibitory activity of chimeric antibodies

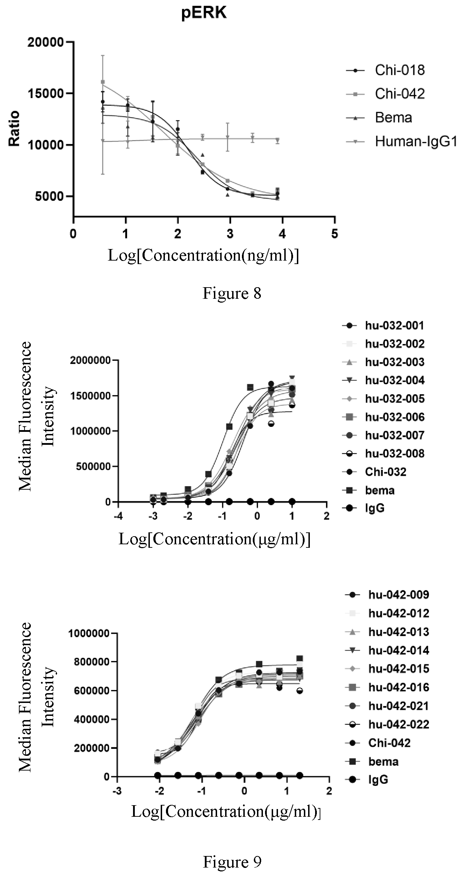

Following antibody treatment, the Advanced phospho-ERK (ThR2 02/TyR2 04) cellular kit (Perkin Elmer, 64AERPEG) was used to detect phosphorylated ERK protein levels in the FGFR2b downstream signaling pathway. FGFR2b/BaF3 cells were collected, washed three times with RPMI 1640 blank medium, resuspended to 2E5/mL, placed in T75 culture flasks, and incubated overnight at 37°C in a 5% CO₂ incubator. The next day, cells were collected, resuspended in RPMI 1640 to 4E6/mL, and 50 µL per well was added to a 96-well U-bottom plate. Candidate antibodies were serially diluted (starting from 50 µg/mL, 3-fold dilution), and 12.5 µL per well of each concentration of antibody was added to the 96-well plate and incubated for 30 minutes at 37°C. 12.5 µL per well of a mixture of FGF7 (final concentration 100 ng/mL) and Heparin (final concentration 10 µg/mL) was added to a 96-well plate and incubated for 15 minutes at 37°C. 25 µL per well of 4× lysis buffer was added, and the plate was incubated on a shaker at 350 rpm for 40 minutes at room temperature. 16 µL of the above lysate supernatant was transferred to a White Opaque 96-well Microplate (Perkin Elmer, Cat#6005680), and 4 µL of pre-mixed Phospho-ERK1/2 d2 antibody and Phospho-ERK1/2 Eu Cryptate antibody mixture was added, and incubated for 4 hours at room temperature. Fluorescence values were read using an Envision (Perkin Elmer). The parameter regression curves were fitted using GraphPad Prism software to calculate EC50 values. The results are shown in Figure 8. The candidate chimeric antibodies inhibited the phosphorylation of ERK protein in the FGF7/FGFR2b signaling pathway in a concentration-dependent manner.

### 6.9 Affinity

The affinity of the candidate chimeric antibodies and control antibodies to human FGFR2b protein was determined using an Octet RED96e molecular interaction instrument. The candidate chimeric antibodies and control antibodies were diluted to 1 µg/mL with PBST (0.05% Tween 20) buffer, then captured onto pre-wetted Protein A biosensors, with a capture amount of 1.3 nm. They were then bound to a gradient-diluted PBST solution of human FGFR2b protein (100 nM, 50 nM, 25 nM, 12.5 nM, 6.25 nM, 3.125 nM, and 1.563 nM) for 180 seconds. Subsequently, the Protein A biosensors were immersed into PBST buffer for dissociation for 360 seconds. The collected data were fitted using the 1:1 binding model in Octet RED96e data Analysis version 12.0 software. The affinity results are shown in Table 7. The results showed that Chi-018, Chi-032, Chi-042, and Bemarituzumab had similar affinities, around 3-5 nM, while Chi-006 had a relatively higher affinity.

**Table 7: Chimeric antibody affinity**

| Samples | ka (1/Ms) | kdis (1/s) | K_{D} (M) |
|---|---|---|---|
| Bemarituzumab | 1.26E+05 | 4.94E-04 | 3.94E-09 |
| Chi-006 | 6.31E+04 | 4.49E-05 | 7.12E-10 |
| Chi-018 | 2.05E+05 | 6.72E-04 | 3.28E-09 |
| Chi-032 | 1.25E+05 | 4.59E-04 | 3.67E-09 |
| Chi-042 | 2.68E+05 | 1.46E-03 | 5.44E-09 |

### Example 7: Humanization of antibody variable regions

For humanization of antibody variable regions, the human immunoglobulin gene database at the NCBI (http://www.ncbi.nlm.nih.gov/igblast/) was first searched for human germline IgG genes homologous to the cDNA sequences of the murine antibodies. The amino acid sequences and precise boundaries of the variable region CDRs were then defined using the Kabat numbering system or the IMGT numbering system. In principle, a human IGHV with high homology to the murine antibody was selected as a humanization template, and humanization of the antibody variable regions was performed by CDR grafting.

Chi-042 and Chi-032 were humanized using the CDR grafting method. For Chi-042 humanization, 5 heavy chains and 9 light chains were generated, which were combined to yield 20 humanized molecules; for Chi-032 humanization, 3 heavy chains and 3 light chains were generated, which were combined to yield 9 humanized molecules. The specific combinations of the humanized antibody heavy and light chains are shown in Tables 1 and 2.

### Example 8: Screening of humanized antibodies

### 8.1 Binding of humanized antibodies to CHO-K1-huFGFR2b cells

CHO-K1-huFGFR2b cells in T75 culture flasks (Corning) were digested and counted, and the cell density was adjusted to 1E6/mL, 100 µL per well of the cells was added to a 96-well U-bottom plate (Corning). The anti-FGFR2b humanized antibodies constructed in Example 7, positive controls (Bema), and negative control (anti-KLH IgG1) were diluted in FACS Buffer (2% FBS/PBS) (starting from 20 µg/mL or 10 µg/mL, 3-fold serial dilution, total of 12 or 8 dilutions). Cells were resuspended in 100 µL per well of the dilution and incubated at 4°C for 1 hour. After washing three times with FACS buffer (2% FBS/PBS), the secondary antibody Alexa fluor 488 F(ab')₂ Fragment Goat Anti-Human IgG, Fcγ fragment specific (Cat# Jackson immunoresearch-109-546-098) or PE F(ab')2 Goat anti-human IgG Fcγ Antibody (Cat# Biolegend-398004) was diluted 1:1000, and cells were incubated at 4°C in the dark for 30 min. After washing twice with FACS buffer, cells were resuspended in 50 µL FACS buffer per well, and the median fluorescence intensity (MFI) was measured using an Intellicyt iQue3 (Satorius). Curve fitting analysis was performed using GraphPad Prism 9 software. The results are shown in Figure 9, demonstrating that the binding activity of the humanized antibodies to CHO-K1-huFGFR2b cells was similar to that of the chimeric antibodies.

### 8.2 ADCC activity of humanized antibodies

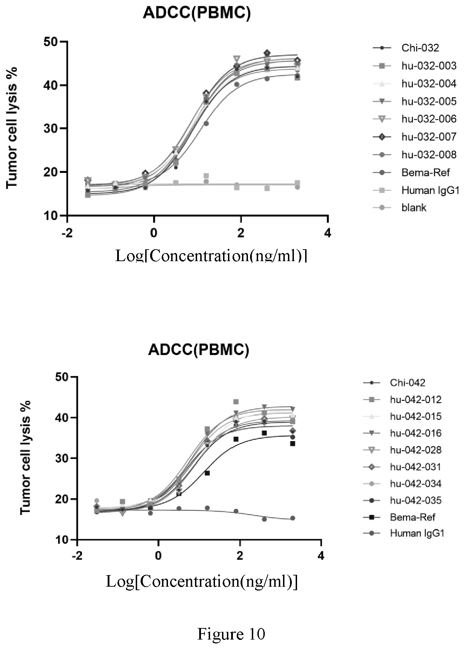

Following the experimental method described in Example 6.6, the ADCC activity of different versions of humanized antibodies hu-042 and hu-032 derived from chimeric antibodies Chi-042 and Chi-032 was detected. The results are shown in Figure 10. The ADCC activity of the humanized molecules was similar to that of the parental chimeric antibodies.

### 8.3 Cell proliferation inhibitory activity of humanized antibody

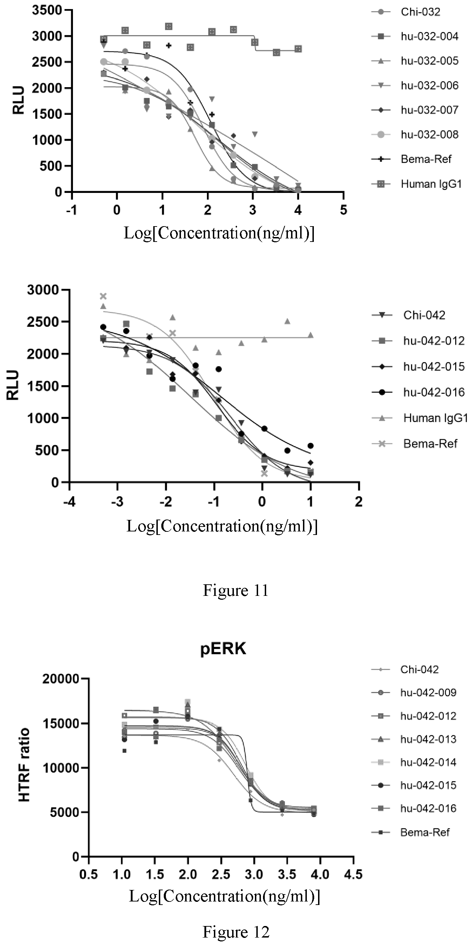

Following the experimental method described in Example 6.7, the cell proliferation inhibition activity of different versions of humanized antibodies hu-042 and hu-032 derived from chimeric antibodies Chi-042 and Chi-032 was detected. The results are shown in Figure 11. The humanized molecules exhibited similar cell proliferation inhibition activity to that of the parental chimeric antibodies.

### 8.4 ERK phosphorylation inhibitory activity of humanized antibodies

Following the experimental method described in Example 6.8, the inhibition of ERK phosphorylation activity of different versions of humanized antibodies hu-042 and hu-032 derived from chimeric antibodies Chi-042 and Chi-032 was detected. The results are shown in Figure 12. The hu-042 humanized molecules inhibited ERK phosphorylation in a concentration-dependent manner, and the inhibitory activity was comparable to that of the parental chimeric antibody.

### 8.5 Affinity of Humanized antibody

The affinity of the humanized antibodies and control antibodies to human FGFR2b protein was determined using an Octet RED96e molecular interaction instrument. The humanized antibodies and control antibodies were diluted to 1 µg/mL with PBST (0.05% Tween 20) buffer, then captured onto pre-wetted Protein A biosensors, with a capture amount of 1.3-1.5 nm. They were then bound to a gradient-diluted PBST solution of human FGFR2b protein (100 nM, 50 nM, 25 nM, 12.5 nM, 6.25 nM, 3.125 nM,; or 100 nM, 25 nM, 5 nM) for 180 seconds. Subsequently, the Protein A biosensors were immersed into PBST buffer for dissociation for 360 seconds. The collected data were fitted using the 1:1 binding model in Octet RED96e data Analysis version 12.0 software. The affinity results are shown in Tables 8 and 9. The affinity of humanized antibody hu-042 was comparable to that of the parental chimeric antibody Chi-042, while the affinity of humanized antibody hu-032 was slightly lower than that of the parental chimeric antibody Chi-032.

**Table 8: Affinity of humanized antibody hu-042**

| Samples | ka (1/Ms) | kdis (1/s) | K_{D} (M) |
|---|---|---|---|
| Bemarituzumab | 2.05E+05 | 5.85E-04 | 2.85E-09 |
| Chi-042 | 3.25E+05 | 1.65E-03 | 5.08E-09 |
| hu-042-009 | 3.78E+05 | 1.97E-03 | 5.22E-09 |
| hu-042-012 | 3.64E+05 | 2.05E-03 | 5.63E-09 |
| hu-042-013 | 3.46E+05 | 1.84E-03 | 5.33E-09 |
| hu-042-014 | 3.17E+05 | 1.89E-03 | 5.98E-09 |
| hu-042-015 | 3.18E+05 | 1.54E-03 | 4.84E-09 |
| hu-042-016 | 3.38E+05 | 1.66E-03 | 4.92E-09 |

**Table 9: Affinity of humanized antibody hu-032**

| Samples | ka (1/Ms) | kdis (1/s) | K_{D} (M) |
|---|---|---|---|
| Bemarituzumab | 1.54E+05 | 6.18E-04 | 4.02E-09 |
| Chi-032 | 1.43E+05 | 2.76E-04 | 1.93E-09 |
| hu-032-003 | 1.78E+05 | 2.59E-03 | 1.45E-08 |
| hu-032-004 | 1.53E+05 | 1.61E-03 | 1.05E-08 |
| hu-032-005 | 1.50E+05 | 1.48E-03 | 9.83E-09 |
| hu-032-006 | 2.44E+05 | 2.65E-03 | 1.09E-08 |
| hu-032-007 | 2.51E+05 | 1.89E-03 | 7.52E-09 |
| hu-032-008 | 2.34E+05 | 1.92E-03 | 8.19E-09 |

### Example 9: Preparation of anti-FGFR2b antibody-drug conjugates

### 9.1 Anti-FGFR2b antibody conjugated to linker-cytotoxin vc-MMAE to prepare conjugate

Antibodies used: reference antibody (Bemarituzumab), hu-032-005, hu-042-012, hu-042-015, hu-042-016.

Linker-payload and its source: vc-MMAE, purchased from Shanghai Haoyuan Medchem Co., Ltd.

The preparation method of the conjugate of anti-FGFR2b antibody coupled to the linker-cytotoxin vc-MMAE to prepare the conjugate was as follows:
Step 1: 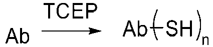
Step 2:

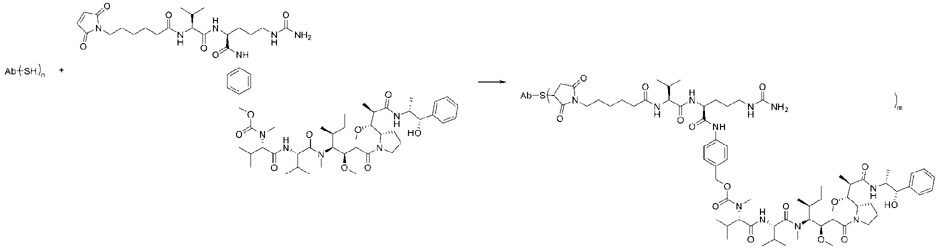

### Preparation of ADC

### ADC-1: Bema

Reduction: 2 mM TCEP (tris(2-carboxyethyl)phosphine, 2.86 eq.) was added to the antibody solution (buffer: pH 7.0, 40 mM PB), and the mixture was incubated at 37°C for 3.5 h to obtain a reduced antibody solution. Conjugation: The reduced antibody solution was placed on ice, and DMA (N,N-dimethylacetamide) and ve-MMAE (8.0 eq.) were added sequentially (total DMA proportion 10%), then the solution was incubated at 25°C for 1h. After the reaction, desalting purification was performed, and the reaction solution was exchanged into buffer solution (20 mM L-His, pH 5.5). The resulting antibody-drug conjugate ADC was obtained by filtration, and the ADC product was stored at -60 to -90°C. Free linker-toxin and other small molecules were detected by reversed-phase chromatography, DAR was detected by HIC chromatography. Purity was determined by size exclusion chromatography.

### ADC-2:hu-032-005

Reduction: 2 mM TCEP (tris(2-carboxyethyl)phosphine, 2.86 eq.) was added to the antibody solution (buffer: pH 7.0, 40 mM PB), and the mixture was incubated at 37°C for 3.5 h to obtain a reduced antibody solution. Conjugation: The reduced antibody solution was placed on ice, and DMA (N,N-dimethylacetamide) and ve-MMAE (8.0 eq.) were added sequentially (total DMA proportion 10%), then the solution was incubated at 25°C for 1h. After the reaction, desalting purification was performed, and the reaction solution was exchanged into buffer solution (20 mM L-His, pH 5.5). The resulting antibody-drug conjugate ADC was obtained by filtration, and the ADC product was stored at -60 to -90°C. Free linker-toxin and other small molecules were detected by reversed-phase chromatography, DAR was detected by HIC chromatography. Purity was determined by size exclusion chromatography.

### ADC-3:hu-042-012

Reduction: 2 mM TCEP (tris(2-carboxyethyl)phosphine, 2.45 eq.) was added to the antibody solution (buffer: pH 7.0, 40 mM PB), and the mixture was incubated at 37°C for 3.5 h to obtain a reduced antibody solution. Conjugation: The reduced antibody solution was placed on ice, and DMA (N,N-dimethylacetamide) and vc-MMAE (8.0 eq.) were added sequentially (total DMA proportion 10%), then the solution was incubated at 25°C for 1h. After the reaction, desalting purification was performed, and the reaction solution was exchanged into buffer solution (20 mM L-His, pH 5.5). The resulting antibody-drug conjugate ADC was obtained by filtration, and the ADC product was stored at -60 to -90°C. Free linker-toxin and other small molecules were detected by reversed-phase chromatography, DAR was detected by HIC chromatography. Purity was determined by size exclusion chromatography.

### ADC-4:hu-042-015

Reduction: 2 mM TCEP (tris(2-carboxyethyl)phosphine, 2.35 eq.) was added to the antibody solution (buffer: pH 7.0, 40 mM PB), and the mixture was incubated at 37°C for 3.5 h to obtain a reduced antibody solution. Conjugation: The reduced antibody solution was placed on ice, and DMA (N,N-dimethylacetamide) and vc-MMAE (8.0 eq.) were added sequentially (total DMA proportion 10%), then the solution was incubated at 25°C for 1h. After the reaction, desalting purification was performed, and the reaction solution was exchanged into buffer solution (20 mM L-His, pH 5.5). The resulting antibody-drug conjugate ADC was obtained by filtration, and the ADC product was stored at -60 to -90°C. Free linker-toxin and other small molecules were detected by reversed-phase chromatography, DAR was detected by HIC chromatography. Purity was determined by size exclusion chromatography.

### ADC-5:hu-042-016

Reduction: 2 mM TCEP (tris(2-carboxyethyl)phosphine, 2.45 eq.) was added to the antibody solution (buffer: pH 7.0, 40 mM PB), and the mixture was incubated at 37°C for 3.5 h to obtain a reduced antibody solution. Conjugation: The reduced antibody solution was placed on ice, and DMA (N,N-dimethylacetamide) and vc-MMAE (8.0 eq.) were added sequentially (total DMA proportion 10%), then the solution was incubated at 25°C for 1h. After the reaction, desalting purification was performed, and the reaction solution was exchanged into buffer solution (20 mM L-His, pH 5.5). The resulting antibody-drug conjugate ADC was obtained by filtration, and the ADC product was stored at -60 to -90°C. Free linker-toxin and other small molecules were detected by reversed-phase chromatography, DAR was detected by HIC chromatography. Purity was determined by size exclusion chromatography. The physicochemical properties of ADC-1 to ADC-5 are summarized in Table 10.

**Table 10: Physicochemical properties of ADC-1 to ADC-5**

| Sample name | Concentration (mg/ml) | SEC | | | DAR | FD (%) |
|---|---|---|---|---|---|---|
| | | Agg (%) | Mono (%) | Frag (%) | | |
| ADC-1 | 2.26 | 1.2 | 98.8 | / | 3.85 | 0.10 |
| ADC-2 | 5.07 | 3.1 | 96.1 | 0.8 | 3.84 | 0.41 |
| ADC-3 | 7.46 | 2.2 | 97.0 | 0.9 | 3.75 | 0.26 |
| ADC-4 | 7.66 | 1.0 | 99.0 | / | 3.86 | 0.21 |
| ADC-5 | 7.32 | 1.7 | 98.3 | 98.3 | 4.09 | 0.16 |

| | | | | | | |
|---|---|---|---|---|---|---|
| FD: free drug ratio | | | | | | |

### 9.2 Anti-FGFR2b antibody conjugated to linker-cytotoxin Deruxtecan to prepare conjugate

### Antibodies: Bemarituzumab, hu-032-005, hu-042-012, hu-042-015, hu-042-016, Isotype IgG1

Linker-payload and its source: Deruxtecan, purchased from Shanghai Haoyuan Medchem Co., Ltd.

The preparation method of the conjugate of anti-FGFR2b antibody coupled to the linker-cytotoxin Deruxtecan to prepare the conjugate was as follows:
Step 1:

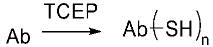

Step 2:

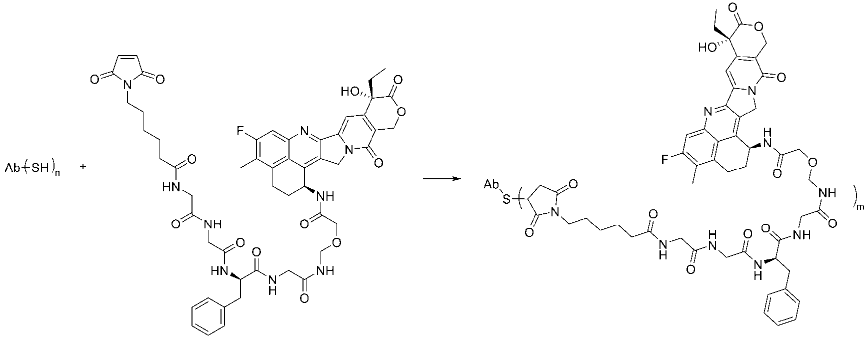

### Preparation of ADC

### ADC-6: Bema

Reduction: 2 mM TCEP (tris (2-carboxyethyl)phosphine, 8.0 eq.) was added to the antibody solution (buffer: pH 7.0, 40 mM PB), and the mixture was incubated at 37°C for 3.5 h to obtain a reduced antibody solution. Conjugation: The reduced antibody solution was placed on ice, and DMA (N,N-dimethylacetamide) and Deruxtecan (14.0 eq.) were added sequentially (total DMA proportion 10%), then the solution was incubated at 25°C for 1h. After the reaction, ultrafiltration purification was performed, and the reaction solution was exchanged into buffer solution (20 mM L-His, pH 5.5). The resulting antibody-drug conjugate ADC was obtained by filtration, and the ADC product was stored at -60 to -90°C. Free linker-toxin and other small molecules were detected by reversed-phase chromatography, DAR was detected by HIC chromatography. Purity was determined by size exclusion chromatography.

### ADC-7:hu-032-005

Reduction: 2 mM TCEP (tris (2-carboxyethyl) phosphine, 8.0 eq.) was added to the antibody solution (buffer: pH 7.0, 40 mM PB), and the mixture was incubated at 37°C for 3.5 h to obtain a reduced antibody solution. Conjugation: The reduced antibody solution was placed on ice, and DMA (N, N-dimethylacetamide) and Deruxtecan (14.0 eq.) were added sequentially (total DMA proportion 10%), then the solution was incubated at 25°C for 1h. After the reaction, ultrafiltration purification was performed, and the reaction solution was exchanged into buffer solution (20 mM L-His, pH 5.5). The resulting antibody-drug conjugate ADC was obtained by filtration, and the ADC product was stored at -60 to -90°C. Free linker-toxin and other small molecules were detected by reversed-phase chromatography, DAR was detected by HIC chromatography. Purity was determined by size exclusion chromatography.

### ADC-8:hu-042-012

Reduction: 2 mM TCEP (tris (2-carboxyethyl) phosphine, 8.0 eq.) was added to the antibody solution (buffer: pH 7.0, 40 mM PB), and the mixture was incubated at 37°C for 3.5 h to obtain a reduced antibody solution. Conjugation: The reduced antibody solution was placed on ice, and DMA (N, N-dimethylacetamide) and Deruxtecan (14.0 eq.) were added sequentially (total DMA proportion 10%), then the solution was incubated at 25°C for 1h. After the reaction, ultrafiltration purification was performed, and the reaction solution was exchanged into buffer solution (20 mM L-His, pH 5.5). The resulting antibody-drug conjugate ADC was obtained by filtration, and the ADC product was stored at -60 to -90°C. Free linker-toxin and other small molecules were detected by reversed-phase chromatography, DAR was detected by HIC chromatography. Purity was determined by size exclusion chromatography.

### ADC-9:hu-042-015

Reduction: 2 mM TCEP (tris (2-carboxyethyl) phosphine, 8.0 eq.) was added to the antibody solution (buffer: pH 7.0, 40 mM PB), and the mixture was incubated at 37°C for 3.5 h to obtain a reduced antibody solution. Conjugation: The reduced antibody solution was placed on ice, and DMA (N, N-dimethylacetamide) and Deruxtecan (14.0 eq.) were added sequentially (total DMA proportion 10%), then the solution was incubated at 25°C for 1h. After the reaction, ultrafiltration purification was performed, and the reaction solution was exchanged into buffer solution (20 mM L-His, pH 5.5). The resulting antibody-drug conjugate ADC was obtained by filtration, and the ADC product was stored at -60 to -90°C. Free linker-toxin and other small molecules were detected by reversed-phase chromatography, DAR was detected by HIC chromatography. Purity was determined by size exclusion chromatography.

### ADC-10:hu-042-016

Reduction: 2 mM TCEP (tris (2-carboxyethyl) phosphine, 8.0 eq.) was added to the antibody solution (buffer: pH 7.0, 40 mM PB), and the mixture was incubated at 37°C for 3.5 h to obtain a reduced antibody solution. Conjugation: The reduced antibody solution was placed on ice, and DMA (N, N-dimethylacetamide) and Deruxtecan (14.0 eq.) were added sequentially (total DMA proportion 10%), then the solution was incubated at 25°C for 1h. After the reaction, ultrafiltration purification was performed, and the reaction solution was exchanged into buffer solution (20 mM L-His, pH 5.5). The resulting antibody-drug conjugate ADC was obtained by filtration, and the ADC product was stored at -60 to -90°C. Free linker-toxin and other small molecules were detected by reversed-phase chromatography, DAR was detected by HIC chromatography. Purity was determined by size exclusion chromatography.

### ADC-11: Isotype IgG1

Reduction: 2 mM TCEP (tris (2-carboxyethyl) phosphine, 8.0 eq.) was added to the antibody solution (buffer: pH 7.0, 40 mM PB), and the mixture was incubated at 37°C for 3.5 h to obtain a reduced antibody solution. Conjugation: The reduced antibody solution was placed on ice, and DMA (N, N-dimethylacetamide) and Deruxtecan (14.0 eq.) were added sequentially (total DMA proportion 10%), then the solution was incubated at 25°C for 1h. After the reaction, ultrafiltration purification was performed, and the reaction solution was exchanged into buffer solution (20 mM L-His, pH 5.5). The resulting antibody-drug conjugate ADC was obtained by filtration, and the ADC product was stored at -60 to -90°C. Free linker-toxin and other small molecules were detected by reversed-phase chromatography, DAR was detected by HIC chromatography. Purity was determined by size exclusion chromatography. The physicochemical properties of ADC-6 to ADC-11 are summarized in Table 11.

**Table 11: Physicochemical properties of ADC-6 to ADC-11**

| Sample name | Concentration (mg/ml) | SEC (%) | DAR | FD (%) |
|---|---|---|---|---|
| ADC-6 | 2.57 | 99.2 | 7.21 | 0.17 |
| ADC-7 | 3.61 | 95.3 | 7.40 | 0.48 |
| ADC-8 | 3.31 | 96.4 | 7.23 | 0.32 |
| ADC-9 | 5.08 | 97.6 | 7.29 | 0.36 |
| ADC-10 | 10.05 | 98.0 | 7.70 | 0.17 |
| ADC-11 | 2.50 | 100.0 | 7.34 | 0.07 |

### 9.3 Anti-FGFR2b antibody conjugated to linker-cytotoxin CPD2 to prepare conjugate

### Antibody: hu-042-016

Linker-payload and its source: CPD2, prepared under commission by Shanghai WuXi AppTec Co., Ltd.

The preparation method of the conjugate of anti-FGFR2b antibody coupled to the linker-cytotoxin CPD2 to prepare the conjugate was as follows:
Step 1: 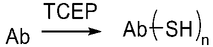
Step 2:

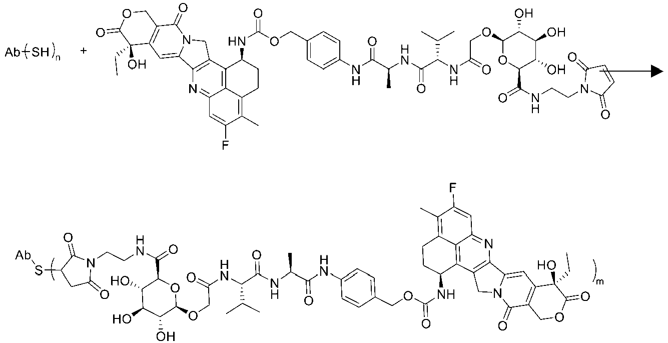

### Preparation of ADC-12

Step 1:TCEP (tris(2-carbonylethyl)phosphine, 7.0 eq.) was added to the antibody in a buffer solution of 50 mM PBS/2 mM EDTA, pH 6.5, and the reaction was stirred at 25°C for 4 h to obtain a reduced antibody solution. Step 2: DMA (N,N-dimethylacetamide) and CPD2 (14.0 eq.) were added to the reduced antibody solution (total DMA proportion 20%), and the reaction was maintained at 25°C for 1 h. The reaction solution was exchanged into the buffer solution (20 mM L-histidine, pH 5.5) and filtered to obtain the antibody-drug conjugate (ADC) of the present invention. The ADC was stored at -60 to -90°C. Free small molecules were detected by reverse-phase high-performance liquid chromatography, and DAR was detected by hydrophobic interaction chromatography. Purity was determined by size exclusion chromatography. The physicochemical properties of ADC-12 are shown in Table 12.

**Table 12: Physicochemical properties of ADC-12**

| Sample name | Concentration (mg/ml) | SEC (%) | DAR | FD (%) |
|---|---|---|---|---|
| ADC-12 | 3.16 | 94.1 | 7.66 | 1.19 |

### 9.4 Anti-FGFR2b antibody conjugated to linker-cytotoxin CPD4 to prepare conjugate

### Antibodies: Bemarituzumab, hu-042-016

Linker-payload and its source: CPD4, prepared under commission by Shanghai WuXi AppTec Co., Ltd.

The preparation method of the conjugate of anti-FGFR2b antibody coupled to the linker-cytotoxin CPD4 to prepare the conjugate was as follows:
Step 1: 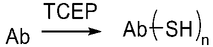
Step 2:

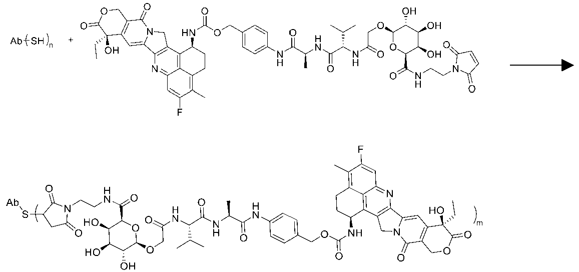

### Preparation of ADC-13 and ADC-14

Step 1: TCEP (tris(2-carbonylethyl)phosphine, 7.0 eq.) was added to the antibody in a buffer solution of 50 mM PBS/2 mM EDTA, pH 6.5, and the reaction was stirred at 25°C for 4 h to obtain a reduced antibody solution. Step 2: DMA (N,N-dimethylacetamide) and CPD4 (14.0 eq.) were added to the reduced antibody solution (total DMA proportion 20%), and the reaction was maintained at 25°C for 1 h. The reaction solution was exchanged into the buffer solution (20 mM L-histidine, pH 5.5) and filtered to obtain the antibody-drug conjugate (ADC) of the present invention. The ADC was stored at -60 to -90°C. Free small molecules were detected by reverse-phase high-performance liquid chromatography, and DAR was detected by hydrophobic interaction chromatography. Purity was determined by size exclusion chromatography. The physicochemical properties of ADC-13 and ADC-14 are shown in Table 13.

**Table 13: Physicochemical properties of ADC-13 to ADC-14**

| Sample name | Antibodies | Concentration (mg/ml) | SEC (%) | DAR | FD (%) |
|---|---|---|---|---|---|
| ADC-13 | Bemarituzumab | 4.18 | 95.4 | 7.49 | 0.65 |
| ADC-14 | hu-042-016 | 4.39 | 93.2 | 7.77 | 0.67 |

### 9.5 Anti-FGFR2b antibody conjugated to linker-cytotoxin CPD5 to prepare conjugate

### Antibody: Bemarituzumab

Linker-payload and its source: CPD5, prepared under commission by Shanghai WuXi AppTec Co., Ltd.

The preparation method of the conjugate of anti-FGFR2b antibody coupled to the linker-cytotoxin CPD5 to prepare the conjugate was as follows:
Step 1: 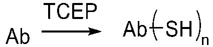
Step 2:

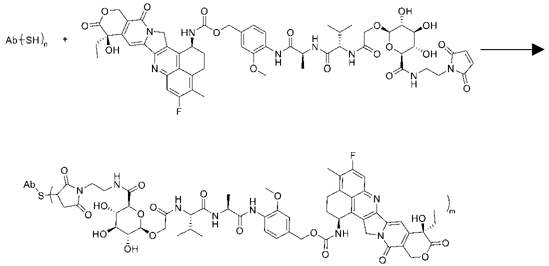

### Preparation of ADC-15

Step 1: TCEP (tris(2-carbonylethyl)phosphine, 7.0 eq.) was added to the antibody in a buffer solution of 50 mM PBS/2 mM EDTA, pH 6.5, and the reaction was stirred at 25°C for 4 h to obtain a reduced antibody solution. Step 2: DMA (N,N-dimethylacetamide) and CPD5 (14.0 eq.) were added to the reduced antibody solution (total DMA proportion 20%), and the reaction was maintained at 25°C for 1 h. The reaction solution was exchanged into the buffer solution (20 mM L-histidine, pH 5.5) and filtered to obtain the antibody-drug conjugate (ADC) of the present invention. The ADC was stored at -60 to -90°C. Free small molecules were detected by reverse-phase high-performance liquid chromatography, and DAR was detected by hydrophobic interaction chromatography. Purity was determined by size exclusion chromatography. The physicochemical properties of ADC-15 are shown in Table 14.

**Table 14: Physicochemical properties of ADC-15**

| Sample name | Concentration (mg/ml) | SEC (%) | DAR | FD (%) |
|---|---|---|---|---|
| ADC-15 | 4.17 | 96.7 | 7.51 | 1.24 |

### 9.6 Anti-FGFR2b antibody conjugated to linker-cytotoxin CPD6 to prepare conjugate

### Antibodies: Bemarituzumab, hu-042-016

Linker-payload and its source: CPD6, prepared under commission by Shanghai WuXi AppTec Co., Ltd.

The preparation method of the conjugate of anti-FGFR2b antibody coupled to the linker-cytotoxin CPD6 to prepare the conjugate was as follows:
Step 1: 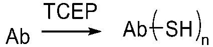
Step 2:

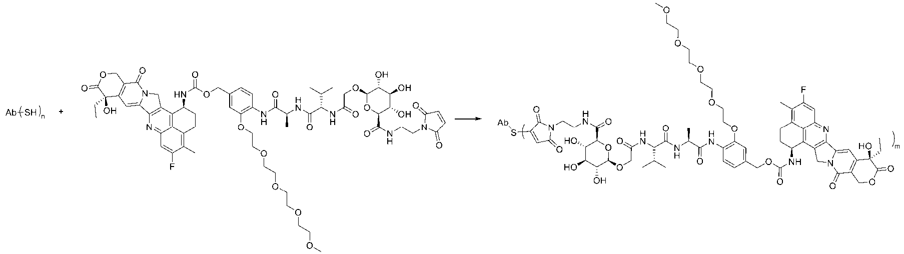

### Preparation of ADC-16 and ADC-17

Step 1: TCEP (tris(2-carbonylethyl)phosphine, 7.0 eq.) was added to the antibody in a buffer solution of 50 mM PBS/2 mM EDTA, pH 6.5, and the reaction was stirred at 25°C for 4 h to obtain a reduced antibody solution. Step 2: DMA (N,N-dimethylacetamide) and CPD6 (14.0 eq.) were added to the reduced antibody solution (total DMA proportion 20%), and the reaction was maintained at 25°C for 1 h. The reaction solution was exchanged into the buffer solution (20 mM L-histidine, pH 5.5) and filtered to obtain the antibody-drug conjugate (ADC) of the present invention. The ADC was stored at -60 to -90°C. Free small molecules were detected by reverse-phase high-performance liquid chromatography, and DAR was detected by hydrophobic interaction chromatography. Purity was determined by size exclusion chromatography. The physicochemical properties of ADC-16 and ADC-17 are shown in Table 15.

**Table 15: Physicochemical properties of ADC-16 to ADC-17**

| Sample name | Antibodies | Concentration (mg/ml) | SEC (%) | DAR | FD (%) |
|---|---|---|---|---|---|
| ADC-16 | Bemarituzumab | 5.08 | 95.9 | 7.46 | 1.76 |
| ADC-17 | hu-042-016 | 3.60 | 94.1 | 7.82 | 0.47 |

### 9.7 Anti-FGFR2b antibody conjugated to linker-cytotoxin SN38-Comp5 to prepare conjugate

### Antibody: hu-042-016

Linker-payload and its source: SN38-Comp5, synthesized by Suzhou Junmeng Biomedical Technology Co., Ltd.

The preparation method of the conjugate of anti-FGFR2b antibody coupled to the linker-cytotoxin SN38-Comp5 to prepare the conjugate was as follows:
Step 1: 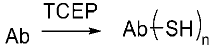
Step 2:

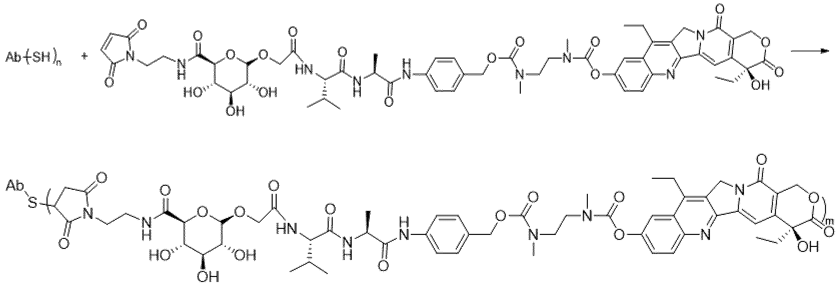

### Preparation of ADC-18

Step 1: TCEP (tris(2-carbonylethyl)phosphine, 7.0 eq.) was added to the antibody in a buffer solution of 50 mM PBS/2 mM EDTA, pH 6.5, and the reaction was stirred at 25°C for 3.5 h to obtain a reduced antibody solution. Step 2: DMA (N,N-dimethylacetamide) and Comp5 (16.0 eq.) were added to the reduced antibody solution (total DMA proportion 20%), and the reaction was maintained at 25°C for 1 h. The reaction solution was exchanged into the buffer solution (50 mM citric acid, pH 6.0) and filtered to obtain the antibody-drug conjugate (ADC) of the present invention. The ADC was stored at -60 to -90°C. Free small molecules were detected by reverse-phase high-performance liquid chromatography, and DAR was detected by PLRP chromatography. Purity was determined by size exclusion chromatography. The physicochemical properties of ADC-18 are shown in Table 16.

**Table 16: Physicochemical properties of ADC-18**

| Sample name | Concentration (mg/ml) | SEC (%) | DAR | FD (%) |
|---|---|---|---|---|
| ADC-18 | 1.01 | 99.1 | 8.0 | <0.52 |

### 9.8 Anti-FGFR2b antibody conjugated to linker-cytotoxin Deruxtecan to prepare conjugate (DAR4)

### Antibody: hu-042-016

Linker-payload and its source: Deruxtecan, purchased from Shanghai Haoyuan Medchem Co., Ltd.

The preparation method of the conjugate of anti-FGFR2b antibody coupled to the linker-cytotoxin to prepare the conjugate was as follows:
Step 1: 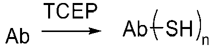
Step 2:

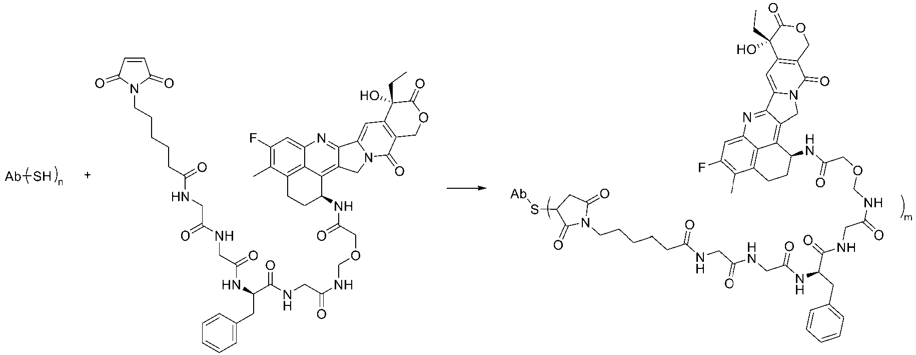

### Preparation of ADC-19

Reduction: 2 mM TCEP (tris (2-carboxyethyl) phosphine, 2.38 eq.) was added to the antibody solution (buffer: pH 6.5, 50 mM PB/ 2 mM EDTA), and the mixture was incubated at 37°C for 3 h to obtain a reduced antibody solution. Conjugation: The reduced antibody solution was placed on ice, and DMA (N, N-dimethylacetamide) and Deruxtecan (7 eq.) were added sequentially (total DMA proportion 15%), then the solution was incubated at 25°C for 1h. After the reaction, ultrafiltration purification was performed, and the reaction solution was exchanged into buffer solution (20 mM L-His, pH 5.5). The resulting antibody-drug conjugate ADC was obtained by filtration, and the ADC product was stored at -60 to -90°C. Free linker-toxin and other small molecules were detected by reversed-phase chromatography, DAR was detected by HIC chromatography. Purity was determined by size exclusion chromatography. The physicochemical properties of ADC-19 are shown in Table 17.

**Table 17: Physicochemical properties of ADC-19**

| Sample name | Concentration (mg/ml) | SEC (%) | DAR | FD (%) |
|---|---|---|---|---|
| ADC-19 | 9.87 | 97.9 | 3.94 | <0.28 |

### 9.9 Anti-FGFR2b antibody conjugated to linker-cytotoxin CPD2 to prepare conjugate (DAR4)

### Antibody: hu-042-016

Linker-payload and its source: CPD2, prepared under commission by Shanghai WuXi AppTec Co., Ltd.

The preparation method of the conjugate of anti-FGFR2b antibody coupled to the linker-cytotoxin CPD2 to prepare the conjugate was as follows:
Step 1: 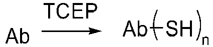
Step 2:

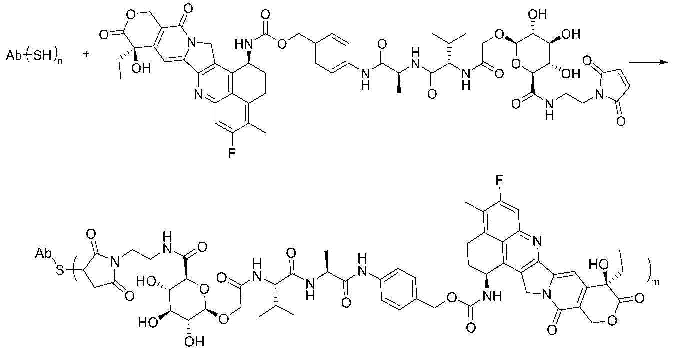

### Preparation of ADC-20

Step 1:TCEP (tris(2-carbonylethyl)phosphine, 2.2 eq.) was added to the antibody in a buffer solution of 50 mM PBS/2 mM EDTA, pH 6.5, and the reaction was stirred at 37°C for 3 h to obtain a reduced antibody solution. Step 2: DMA (N,N-dimethylacetamide) and CPD2 (6.0 eq.) were added to the reduced antibody solution (total DMA proportion 15%), and the reaction was maintained at 25°C for 1 h. The reaction solution was exchanged into the buffer solution (20 mM L-histidine, pH 5.5) and filtered to obtain the antibody-drug conjugate (ADC) of the present invention. The ADC was stored at -60 to -90°C. Free small molecules were detected by reverse-phase high-performance liquid chromatography, and DAR was detected by hydrophobic interaction chromatography. Purity was determined by size exclusion chromatography. The physicochemical properties of ADC-20 are shown in Table 18.

**Table 18: Physicochemical properties of ADC-20**

| Sample name | Concentration (mg/ml) | SEC (%) | DAR | FD (%) |
|---|---|---|---|---|
| ADC-20 | 10.05 | 97.8 | 4.03 | 0.87 |

### 9.10 Anti-FGFR2b antibody conjugated to linker-cytotoxin CPD6 to prepare conjugate (DAR4)

### Antibody: hu-042-016

Linker-payload and its source: CPD6, prepared under commission by Shanghai WuXi AppTec Co., Ltd.

The preparation method of the conjugate of anti-FGFR2b antibody coupled to the linker-cytotoxin CPD6 to prepare the conjugate was as follows:
Step 1: 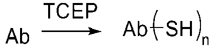
Step 2:

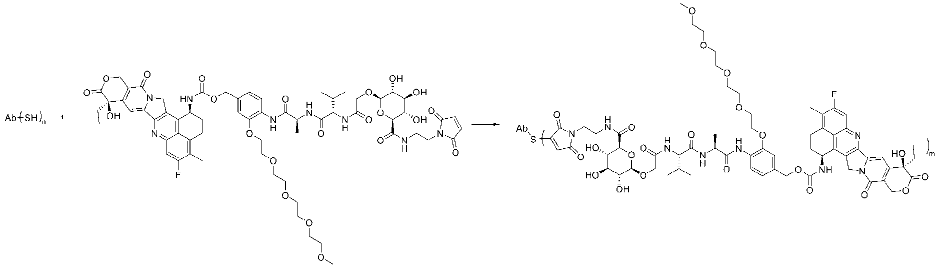

### Preparation of ADC-21

Step 1: TCEP (tris (2-carbonylethyl) phosphine, 2.2 eq.) was added to the antibody in a buffer solution of 50 mM PBS/2 mM EDTA, pH 6.5, and the reaction was stirred at 37°C for 3 h to obtain a reduced antibody solution. Step 2: DMA (N, N-dimethylacetamide) and CPD6 (6.0 eq.) were added to the reduced antibody solution (total DMA proportion 15%), and the reaction was maintained at 25°C for 1 h. The reaction solution was exchanged into the buffer solution (20 mM L-histidine, pH 5.5) and filtered to obtain the antibody-drug conjugate (ADC) of the present invention. The ADC was stored at -60 to -90°C. Free small molecules were detected by reverse-phase high-performance liquid chromatography, and DAR was detected by hydrophobic interaction chromatography. Purity was determined by size exclusion chromatography. The physicochemical properties of ADC-21 are shown in Table 19.

**Table 19: Physicochemical properties of ADC-21**

| Sample name | Concentration (mg/ml) | SEC (%) | DAR | FD (%) |
|---|---|---|---|---|
| ADC-21 | 9.92 | 97.6 | 4.30 | <0.38 |

### Example 10: Internalization activity of anti-FGFR2b antibody drug conjugates

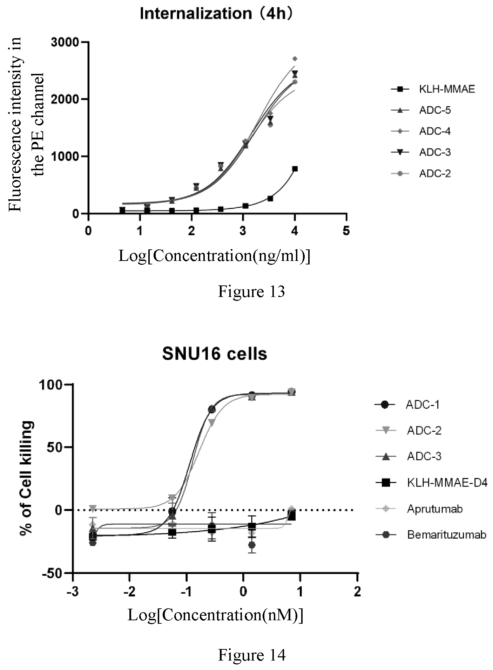

Each ADC sample prepared in Example 9 was labeled according to the instructions for Zenon^{™} pHrodo^{™} iFL IgG Labeling Reagent (Invitrogen, Cat# Z25612). The samples were serially diluted in culture medium, and 50 µL per well of the samples was added to an equal volume of SNU16 cells. The cells were incubated at 37°C for 4 hours, and the fluorescence of the PE channel was measured by flow cytometry to reflect the number of internalized molecules. The results are shown in Figure 13. Compared to the negative control KLH-MMAE, hu-042-MMAE and hu-032-MMAE exhibited good target-dependent internalization activity.

### Example 11: In vitro killing experiment of anti-FGFR2b antibody drug conjugate

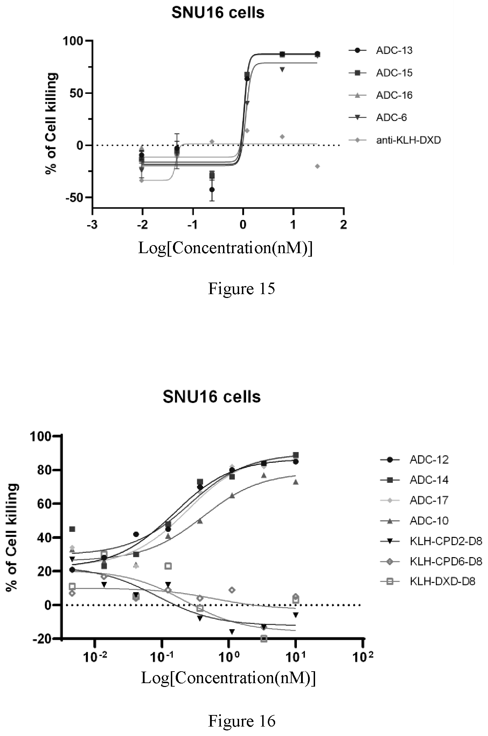

After 6 days of co-culture of each ADC sample with SNU16 cells expressing FGFR2b, viable cell counts were determined using a cell counting lite (Vazyme #DD1102-01). The in vitro cytotoxic activity of the candidate molecules was determined by calculating the proliferation inhibition rate (Proliferation inhibition rate (%) = (number of viable cell count in untreated group - number of viable cell count in treated group) / (number of viable cell count in untreated group - number of viable cell count in solution control group)). Figure 14 shows that Bemarituzumab or Aprutumab monoclonal antibodies did not affect the proliferation activity of SNU16 cells, whereas ADC-1, -2, and -3 exhibited significant cytotoxic activity. Figure 15 shows that ADC samples (ADC-13, -15, -16) constructed with different linkers exhibited significant and similar cytotoxic activity on SNU16 cells, which was comparable to that of ADC-6. Table 20 summarizes the in vitro cytotoxicity EC50 values of ADC-13, -15, -16 samples and ADC-6, and the relative cytotoxic activity (%) of ADC-13, -15, -16 was calculated based on ADC-6.

**Table 20: Proliferation inhibitory activity of anti-FGFR2b ADCs to SNU16 cells**

| Sample | Bema-DXD (ADC-6) | Bema-CPD4 (ADC-13) | Bema-CPD5 (ADC-15) | Bema-CPD6 (ADC-16) | anti-KLH-DXD |
|---|---|---|---|---|---|
| EC₅₀(nM) | ~ 1.141 | ~ 1.067 | ~ 1.040 | ~ 1.052 | N/A |
| Relative activity (%) | ~100.0 | ~106.9 | ~109.7 | ~108.5 | N/A |

| | | | | | |
|---|---|---|---|---|---|
| NA: Not applicable. | | | | | |

Furthermore, Figure 16 compares the cytotoxic activity of hu-042-016 ADC samples constructed with different linkers against SNU16 cells. The results showed that all hu-042-016-CPD ADC samples (ADC-12, -14, -17) significantly inhibited the in vitro proliferation ability of SNU16 cells, with similar activity that was slightly higher than that of hu-042-016-DXD ADC (ADC-10).

### Example 12: In vitro bystander assay of anti-FGFR2b antibody drug conjugate

N87 cells were pre-labeled according to the instructions of CellTrace^{™} Violet (Cat# Invitrogen^{™}-C34557) and stained with PBS at 37°C for 20 minutes. Five volumes of complete medium (RPMI 1640 + 10% FBS) were added and incubated at 37°C for 5 minutes to terminate staining. 5000 SNU16 cells (100 µL) and 15000 N87 cells (100 µL) were added per well to a 24-well U-bottom plate (Corning). Candidate antibodies were diluted to 100 nM in complete medium, and 50 µL per well of the antibodies was added to the 24-well plate. Complete medium was added to bring the volume to 500 µL per well. The plate was gently mixed and incubated at 37°C for 5 days. After 5 days, the supernatant was gently shaken, and cells in the supernatant were collected into flow cytometry tubes. The medium was then washed with 300 µl of PBS per well and the PBS was collected into the corresponding flow cytometry tubes. 150 µL of TrypLE^{™} (Cat# Gibco-12604021) was added, and the plate was incubated at 37°C for 5 minutes for digestion. An equal volume of complete medium was added to stop the digestion, and the digested cells were collected into the corresponding flow cytometry tubes. The tubes were centrifuged at 1000 rpm for 5 minutes, the supernatant was discarded, and 300 µL of buffer containing 250 µL FACS buffer (PBS+2% FBS+0.1% PI) and 50 µL CountBright^{™} Absolute Counting Beads (Cat# Invitrogen-C36950) was added to each tube. The samples were mixed by vortexing and analyzed on a Fortessa flow cytometer, with collection of 5000 beads per well as a stopping gate. FlowJo was used to analyze PI-negative viable SNU16 and N87 cells. The numbers of PI-negative viable SNU16 and N87 cells in the complete buffer volume were calculated based on the CountBright^{™} Absolute Counting Beads. Data were plotted using GraphPad Prism.

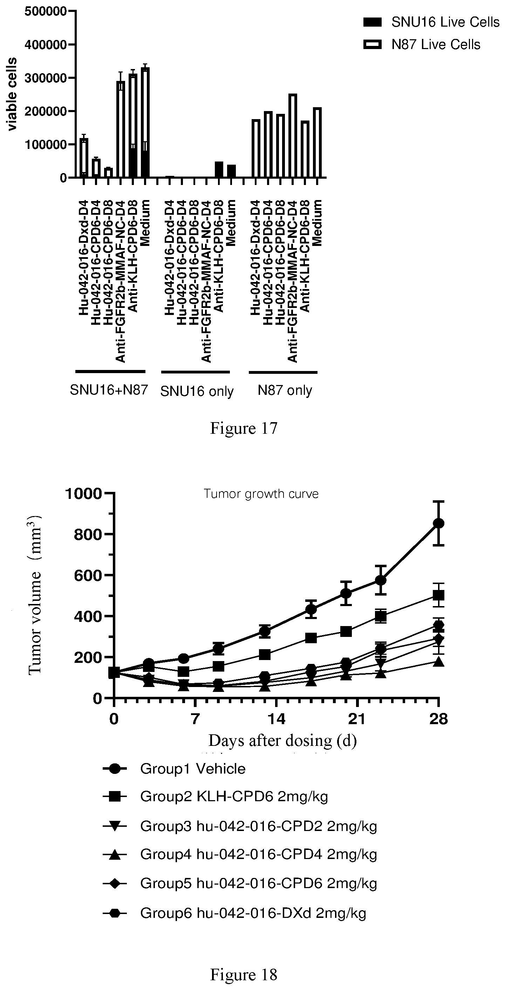

The results are shown in Figure 17. In co-culture of SNU16 and N87 cells, hu-042-016-Dxd-D4, hu-042-016-CPD6-D4, and hu-042-016-CPD6-D8 significantly killed FGFR2b-positive SNU16 cells and exhibited good bystander killing activity against FGFR2b-negative N87 cells. Due to the poor membrane-penetrating ability of MMAF, the anti-FGFR2b MMAF sample lacked bystander-killing activity and only inhibited SNU16 cell proliferation without affecting N87 cell viability. Anti-KLH-CPD6-D8 and blank medium groups showed no effective killing effect on either cell type and served as negative controls. When SNU16 cells were cultured alone, all anti-FGFR2b ADCs effectively killed SNU16 cells; however, when N87 cells were cultured alone, none of the anti-FGFR2b ADCs affected N87 cell proliferation.

### Example 13: In vivo efficacy study of anti-FGFR2b antibody-drug conjugates

Experimental animals: 48 NDG mice, female, 6-8 weeks old, body weight approximately 18-22 g. Purchased from Biocytogen Jiangsu Gene Biotechnology Co., Ltd.

Antibody drugs:
hu-042-016-DXD, Lot#: 20230704-R1; DAR: 7.28;
hu-042-016-CPD2, Lot#: 20230626-R1; DAR: 7.66;
hu-042-016-CPD4, Lot#: 20230616-R1; DAR: 7.88;
hu-042-016-CPD6, Lot#: 20230616-R2; DAR: 7.82;
KLH-CPD6, Lot#: 20230713-R1; DAR: 7.84.

### Test process:

SNU16 cells were cultured in RPMI 1640 supplemented with 10% fetal bovine serum (FBS). SNU16 cells in the logarithmic growth phase were collected, resuspended in PBS to an appropriate concentration for inoculation into NDG mice. SNU16 cells were harvested and inoculated subcutaneously on the right flank of NDG mice at a density of 3.5×10⁶ cells/100 µl. When the tumors grew to approximately 150 mm³, the mice were randomized into groups based on tumor volume, and dosing was initiated. The day of grouping was designated as Day 0. Detailed dosing methods, doses, and routes of administration are shown in Table 21 below.

**Table 21: dosing regimen, doses and route of administration**

| Group | Drug-treated group | N | Dose | Dosing regimen | Route of administration |
|---|---|---|---|---|---|
| 1 | vehicle control | 6 | - | single dose | i.v. |
| 2 | KLH-CPD6 | 6 | 2 mg/kg | single dose | i.v. |
| 3 | hu-042-016-CPD2 | 6 | 2 mg/kg | single dose | i.v. |
| 4 | hu-042-016-CPD4 | 6 | 2 mg/kg | single dose | i.v. |
| 5 | hu-042-016-CPD6 | 6 | 2 mg/kg | single dose | i.v. |
| 6 | hu-042-016-DXD | 6 | 2 mg/kg | single dose | i.v. |

| | | | | | |
|---|---|---|---|---|---|
| Note: N: number of animals used; i.v.: intravenous injection; administration volume: the administration volume was adjusted according to the body weight of the tumor-bearing mice (0.1 mL/10 g). | | | | | |

During the experiment, tumor volume and body weight were measured twice a week. The experimental results are shown in Table 22 and Figure 18.

**Table 22: Average tumor volume of mice in each group (Mean ± SEM)**

| Experimental group | Tumor volume (post-administration) | | TGI% (Day 28) | T/C% (Day 28) | P value |
|---|---|---|---|---|---|
| | Day 0 | Day 28 | | | |
| 1 | 125.69±18.87 | 852.59±261.11 | -- | -- | -- |
| 2 | 125.76±17.82 | 502.81±141.96**** | 48.13 | 51.87 | <0.0001 |
| 3 | 127.60±16.30 | 275.30±147.11**** | 79.68 | 20.32 | <0.0001 |
| 4 | 125.73±16.29 | 180.31±46.06**** | 92.49 | 7.51 | <0.0001 |
| 5 | 124.91±15.03 | 291.96±94.30**** | 77.02 | 22.98 | <0.0001 |
| 6 | 125.08±13.36 | 358.23±83.60**** | 67.92 | 32.08 | <0.0001 |

| | | | | | |
|---|---|---|---|---|---|
| Note: p < 0.05 was considered significant. The antitumor efficacy of the test substance was evaluated using the tumor growth inhibition rate (TGI) (%), which reflects the tumor growth inhibition rate. TGI(%)=[(1- (TV_{Treatment}_DayN - TV_{Treatment}_Day0))/(TV_{Vehicle}_DayN - TV_{Vehicle}_Day0)]×100%. TV_{Treatment}_DayN, TV_{Treatment}_Day0, TV_{Vehicle}_DayN, and TV_{Vehicle}_Day0 are the mean tumor volumes of the treatment group on a given day (Day N) and at baseline (Day 0), and of the vehicle control group on a given day (Day N) and at baseline (Day 0), respectively. T/C (%) is the relative tumor proliferation rate, T/C (%) = (1 - TGI) × 100%. | | | | | |

At the end of the experiment (28 days after administration), compared with the control group, the anti-FGFR2b antibody-drug conjugate (ADC) hu-042-016-CPD2, hu-042-016-CPD4, hu-042-016-CPD6 and hu-042-016-DXD at a dose level of 2 mg/kg significantly inhibited tumor growth, with tumor growth inhibition rates of 79.68% (p < 0.0001), 92.49% (p < 0.0001), 77.02% (p < 0.0001), and 67.92% (p < 0.0001), respectively.

### Example 14: In vivo efficacy study of anti-FGFR2b antibody drug conjugate (DAR4)

Experimental animals: 40 NDG mice, female, 6-8 weeks old, body weight approximately 18-22 g. Purchased from Biocytogen Jiangsu Gene Biotechnology Co., Ltd.

Antibody drugs:
hu-042-016-DXD DAR4, Lot#: 20231001-R12; DAR: 4.05;
hu-042-016-CPD2 DAR4, Lot#: 20231001-R1; DAR: 4.14;
hu-042-016-CPD1 DAR4, Lot#: 20231017-L1; DAR: 4.10;
hu-042-016-CPD6 DAR4, Lot#: 20231011-R1; DAR: 4.10;
hu-042-016-CPD6 DAR8, Lot#: 20231011-R2; DAR: 7.90;
KLH-CPD6 DAR4, Lot#: 20231107-R1; DAR: 4.23.

### Test process:

SNU16 cells were cultured in RPMI 1640 supplemented with 10% fetal bovine serum (FBS). SNU16 cells in the logarithmic growth phase were collected, resuspended in PBS to an appropriate concentration, and inoculated subcutaneously on the right flank of NDG mice at a density of 3.5×10⁶ cells/100 µl. When the tumors grew to approximately 150 mm³, the day of grouping was designated as Day 0. The mice were randomized into groups based on tumor volume, and dosing was initiated. Detailed dosing methods, doses, and routes of administration are shown in Table 23 below.

**Table 23: dosing regimen, doses and route of administration**

| Group | Drug-treated group | N | Dose | Dosing regimen | Route of administration |
|---|---|---|---|---|---|
| 1 | Vehicle control | 5 | | single dose | i.v. |
| 2 | hu-042-016-CPD6 DAR4 | 5 | 0.6 mg/kg | single dose | i.v. |
| 3 | hu-042-016-CPD6 DAR4 | 5 | 2 mg/kg | single dose | i.v. |
| 4 | KLH-CPD6 DAR4 | 5 | 2 mg/kg | single dose | i.v. |
| 5 | hu-042-016-CPD6 DAR8 | 5 | 2 mg/kg | single dose | i.v. |
| 6 | hu-042-016-DXd DAR4 | 5 | 2 mg/kg | single dose | i.v. |
| 7 | hu-042-016-CPD2 DAR4 | 5 | 2 mg/kg | single dose | i.v. |
| 8 | hu-042-016-CPD1 DAR4 | 5 | 2 mg/kg | single dose | i.v. |

| | | | | | |
|---|---|---|---|---|---|
| Note: N: number of animals used; i.v.: intravenous injection; administration volume: the administration volume was adjusted according to the body weight of the tumor-bearing mice (0.1 mL/10 g). | | | | | |

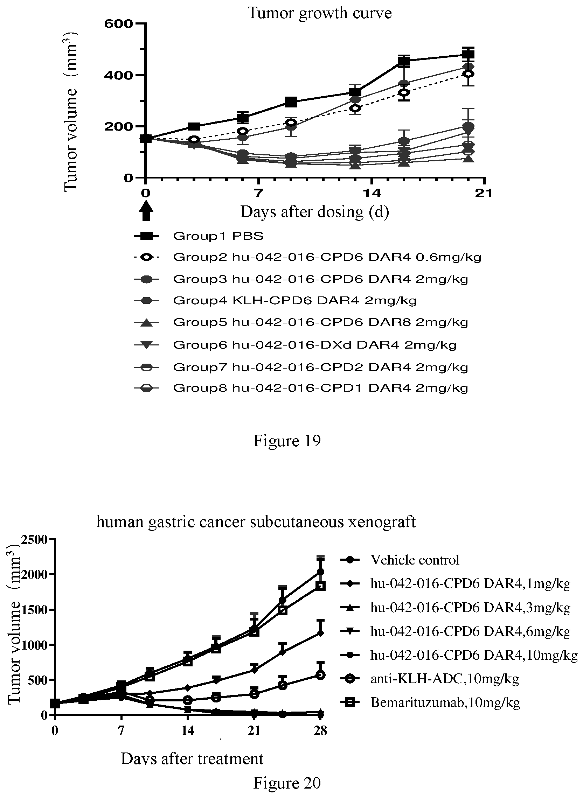

During the experiment, tumor volume and body weight were measured twice a week. The experimental results are shown in Table 24 and Figure 19.

**Table 24: Average tumor volume of mice in each group (Mean ± SEM)**

| Experimental group | Tumor volume (post-administration) | | TGI% (Day 20) | T/C% (Day 20) | P value |
|---|---|---|---|---|---|
| | Day 0 | Day 20 | | | |
| 1 | 151.59±22.93 | 479.01±60.22 | -- | -- | -- |
| 2 | 154.60±21.17 | 405.92±107.85 | 23.24 | 76.76 | 0.1928 |
| 3 | 153.47±19.67 | 202.93±153.10**** | 84.89 | 15.11 | <0.0001 |
| 4 | 153.41±18.53 | 432.91±167.85 | 14.64 | 85.36 | 0.6565 |
| 5 | 152.79±14.46 | 75.42±17.75**** | 123.63 | -23.63 | <0.0001 |
| 6 | 154.28±14.01 | 177.61±107.60**** | 92.88 | 7.12 | <0.0001 |
| 7 | 153.24±11.77 | 102.46±18.83**** | 115.51 | -15.51 | <0.0001 |
| 8 | 152.32±11.40 | 129.19±66.22**** | 107.06 | -7.06 | <0.0001 |

| | | | | | |
|---|---|---|---|---|---|
| Note: p < 0.05 was considered significant. The antitumor efficacy of the test substance was evaluated using the tumor growth inhibition rate (TGI) (%), which reflects the tumor growth inhibition rate. TGI(%)=[(1- (TV_{Treatment}_DayN - TV_{Treatment}_Day0))/(TV_{Vehicle}_DayN - TV_{Vehicle}_Day0)]×100%. TVTreatment_DayN, TVTreatment_Day0, TVVehicle_DayN, and TVVehicle_Day0 are the mean tumor volumes of the treatment group on a given day (Day N) and at baseline (Day 0), and of the vehicle control group on a given day (Day N) and at baseline (Day 0), respectively. T/C (%) is the relative tumor proliferation rate, T/C (%) = (1 - TGI) × 100%. | | | | | |

At the end of the experiment (20 days after administration), compared with the control group, the anti-FGFR2b antibody-drug conjugate (ADC) hu-042-016-CPD6 DAR4, hu-042-016-CPD2 DAR4, hu-042-016-CPD1 DAR4 and hu-042-016-DXd DAR4 at a dose level of 2 mg/kg significantly inhibited tumor growth, with TGI of 84.89% (p < 0.0001), 115.51% (p < 0.0001), 107.06% (p < 0.0001), and 92.88% (p < 0.0001), respectively. In addition, hu-042-016-CPD6 DAR8 showed the greatest tumor inhibition effect, with a TGI of 123.63% (p < 0.0001).

### Example 15: In vivo pharmacodynamic study of anti-FGFR2b antibody-drug conjugates in human gastric cancer subcutaneous transplanted tumors

Experimental Procedure: The successfully revived FP4+2 generation LD1-0017-200652 human gastric cancer in vivo xenograft tumor pieces were cut into small pieces of approximately 3 mm × 3 mm × 3 mm and inoculated subcutaneously into the right dorsal side of NU/NU mice using a tumor inoculation needle (efficacy study passage: FP4+3). Each animal was inoculated with approximately 30-45 mg of tumor tissue supplemented with 10 µL of Matrigel. The mice were observed after inoculation, and tumor growth was monitored. On day 18 after inoculation, when the mean tumor volume of the tumor-bearing mice reached 163.61 mm³, the mice were randomized into groups and administered. The day of grouping and dosing was defined as Day 0. Grouping and dosing information are shown in Table 25 below.

**Table 25: dosing regimen, doses and route of administration**

| Group | Drug-treated group | N | Dose (mg/kg) | Dosing regimen | Route of administration |
|---|---|---|---|---|---|
| 1 | Vehicle | 6 | -- | single dose | IV |
| 2 | hu-042-016-CPD6 DAR4 | 6 | 1 | single dose | IV |
| 3 | hu-042-016-CPD6 DAR4 | 6 | 3 | single dose | IV |
| 4 | hu-042-016-CPD6 DAR4 | 6 | 6 | single dose | IV |
| 5 | hu-042-016-CPD6 DAR4 | 6 | 10 | single dose | IV |
| 6 | anti-KLH-ADC | 6 | 10 | single dose | IV |
| 7 | Bemarituzumab | 6 | 10 | BIW*4W | IV |

| | | | | | |
|---|---|---|---|---|---|
| Note: N: number of animals used; BIW*4W: administration twice a week for 4 consecutive weeks; IV: intravenous injection. | | | | | |

During the experiment, tumor volume was measured twice a week. The experimental results are shown in Table 26 and Figure 20.

**Table 26: Evaluation of the antitumor efficacy of the test drugs in a human gastric cancer subcutaneous xenograft model**

| Group | Treatment group | Tumor volume (Day 0) | Tumor volume (Day 28) | T/C (%) (Day 28) | TGI (%) (Day 28) |
|---|---|---|---|---|---|
| 1 | Vehicle | 163.73±11.68 | 2034.39±175.94 | -- | -- |
| 2 | hu-042-016-CPD6 DAR4 (1 mg/kg) | 163.81±10.38 | 1165.26±183.43 | 57.25 | 46.47 |
| 3 | hu-042-016-CPD6 DAR4 (3 mg/kg) | 163.09±10.11 | 40.10±18.86 | 1.98 | 106.57 |
| 4 | hu-042-016-CPD6 DAR4 (6 mg/kg) | 163.78±10.89 | 0±0 | 0.00 | 108.76 |
| 5 | hu-042-016-CPD6 DAR4 (10 mg/kg) | 163.91±10.39 | 0±0 | 0.00 | 108.76 |
| 6 | anti-KLH-ADC | 163.6±12.75 | 569.80±178.52 | 28.03 | 78.29 |
| 7 | Bemarituzumab | 163.37±12.03 | 1829.27±427.37 | 90.12 | 10.95 |

The experimental results showed that, compared to the Vehicle group, all dosing groups of hu-042-016-CPD6 DAR4 inhibited tumor growth, with inhibitory activity significantly higher than that of the positive control Bemarituzumab (TGI of only 10.95%). Among them, the 3 mg/kg, 6 mg/kg, and 10 mg/kg groups showed more significant inhibition effects, with TGIs of 106.57%, 108.76%, and 108.76%, respectively.

Example 16: In vivo pharmacodynamic study of anti-FGFR2b antibody drug conjugates i subcutaneous human breast cancer xenografts

Experimental Procedure: The successfully revived FP3+3 generation LD1-2009-361825 human breast cancer in vivo xenograft tumor pieces were cut into small pieces of approximately 3 mm × 3 mm × 3 mm and inoculated subcutaneously into the right dorsal side of NCG mice using a tumor inoculation needle (efficacy study passage: FP3+4). Each animal was inoculated with approximately 50-90 mg of tumor tissue supplemented with 30 µL of Matrigel. The mice were observed after inoculation, and tumor growth was monitored. On day 28 after inoculation, when the mean tumor volume of the tumor-bearing mice reached 149.22 mm³, the mice were randomized into groups and dosed. The day of grouping and dosing was defined as Day 0. Specific grouping information is shown in Table 26.

**Table 26: dosing regimen, doses and route of administration**

| Group | Drug-treated group | N | Dose (mg/kg) | Dosing regimen | Route of administration |
|---|---|---|---|---|---|
| 1 | Vehicle | 8 | -- | single dose | IV |
| 2 | hu-042-016-CPD6 DAR4 | 8 | 3 | single dose | IV |
| 3 | hu-042-016-CPD6 DAR4 | 8 | 6 | single dose | IV |
| 4 | hu-042-016-CPD6 DAR4 | 8 | 10 | single dose | IV |
| 5 | anti-KLH-ADC | 8 | 10 | single dose | IV |
| 6 | Bemarituzumab | 8 | 10 | BIW*4W | IV |

| | | | | | |
|---|---|---|---|---|---|
| Note: N: number of animals used; BIW*4W: administration twice a week for 4 consecutive weeks; IV: intravenous injection. | | | | | |

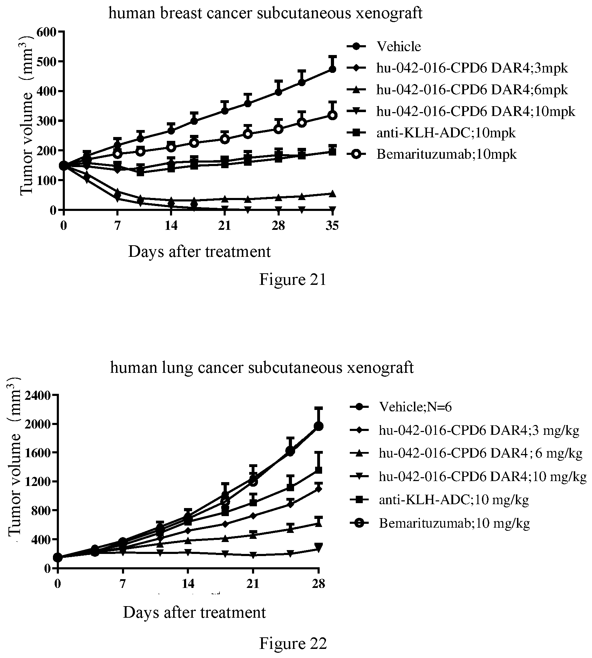

During the experiment, tumor volume was measured twice a week. The experimental results are shown in Table 27 and Figure 21.

**Table 27. Evaluation of the antitumor efficacy of the test drugs in a human breast cancer subcutaneous xenograft model**

| Group | Treatment group | Tumor volume (Day 0) | Tumor volume (Day 35) | T/C(%) (Day 35) | TGI(%) (Day 35) |
|---|---|---|---|---|---|
| 1 | Vehicle | 149.29±10.01 | 473.41±42.18 | -- | -- |
| 2 | hu-042-016-CPD6 DAR4 | 149.02±10.72 | 196.04±19.05 | 41.49 | 85.49 |
| | (3 mg/kg) | | | | |
| 3 | hu-042-016-CPD6 DAR4 (6 mg/kg) | 149.45±9.81 | 55.31±8.97 | 11.67 | 129.04 |
| 4 | hu-042-016-CPD6 DAR4 (10 mg/kg) | 149.15±9.30 | 0±0 | 0.00 | 146.02 |
| 5 | anti-KLH-ADC | 149.18±9.88 | 195.23±21.82 | 41.27 | 85.79 |
| 6 | Bemarituzumab | 149.22±8.62 | 318.53±44.47 | 67.32 | 47.76 |

The experimental results showed that, compared to the Vehicle group, all dose groups of hu-042-016-CPD6 DAR4 significantly inhibited tumor growth (TGIs of 85.49%, 129.04%, and 146.02%, respectively). The inhibitory activity was positively correlated with dose and was superior to that of Bemarituzumab (TGI of 47.76%).

Example 17: In vivo pharmacodynamic study of anti-FGFR2b antibody-drug conjugates on subcutaneous human lung cancer xenografts

Experimental Procedure: The successfully revived FP2+1 generation LD1-0025-200783 human lung cancer in vivo xenograft tumor pieces were cut into small pieces of approximately 3 mm × 3 mm × 3 mm and inoculated subcutaneously into the right dorsal side of NU/NU mice using a tumor inoculation needle (efficacy study passage: FP2+2). Each animal was inoculated with approximately 40-75 mg of tumor tissue supplemented with 15 µL of Matrigel. The mice were observed after inoculation, and tumor growth was monitored. On day 21 after inoculation, when the mean tumor volume of the tumor-bearing mice reached 149.12 mm³, the mice were randomized into groups and dosed. The day of grouping and dosing was defined as Day 0. Specific grouping information is shown in Table 28.

**Table 28: dosing regimen, doses and route of administration**

| Group | Drug-treated group | N | Dose (mg/kg) | Dosing regimen | Route of administration |
|---|---|---|---|---|---|
| 1 | Vehicle | 6 | -- | QW*2 | IV |
| 2 | hu-042-016-CPD6 DAR4 | 6 | 3 | QW*2 | IV |
| 3 | hu-042-016-CPD6 DAR4 | 6 | 6 | QW*2 | IV |
| 4 | hu-042-016-CPD6 DAR4 | 6 | 10 | QW*2 | IV |
| 5 | anti-KLH-ADC | 6 | 10 | QW*2 | IV |
| 6 | Bemarituzumab | 6 | 10 | BIW*4W | IV |

| | | | | | |
|---|---|---|---|---|---|
| Note: N: number of animals; IV: tail vein injection; QW: administration once a week; BIW*4W: administration twice a week for 4 consecutive weeks. | | | | | |

During the experiment, tumor volume was measured twice a week. The experimental results are shown in Table 29 and Figure 22.

**Table 29: Evaluation of the antitumor efficacy of the test drugs in a human lung cancer subcutaneous xenograft tumor model**

| Group | Treatment group | Tumor volume (Day 0) | Tumor volume (Day 28) | T/C(%) (Day 28) | TGI(%) (Day 28) |
|---|---|---|---|---|---|
| 1 | Vehicle | 149.23±12.38 | 1963.93±259.08 | -- | -- |
| 2 | hu-042-016-CPD6 DAR4 (3 mg/kg) | 149.00±9.44 | 1095.93±80.19 | 55.89 | 47.82 |
| 3 | hu-042-016-CPD6 DAR4 (6 mg/kg) | 149.01±9.63 | 619.63±84.17 | 31.60 | 74.07 |
| 4 | hu-042-016-CPD6 DAR4 (10 mg/kg) | 149.36±9.92 | 262.96±70.48 | 13.38 | 93.74 |
| 5 | anti-KLH-ADC | 149.06±10.06 | 1354.36±247.45 | 69.04 | 33.58 |
| 6 | Bemarituzumab | 149.03±12.12 | 1969.71±245.93 | 100.43 | -0.33 |

The experimental results showed that, compared to the Vehicle group, Bemarituzumab had almost no anti-tumor activity, with a TGI of -0.33%. All dose groups of hu-042-016-CPD6 DAR4 significantly inhibited tumor growth, with TGIs of 47.82%, 74.07%, and 93.74%, respectively, and the inhibitory activity was positively correlated with dose.

## Claims

1. An antibody or antigen-binding fragment thereof that specifically binds to FGFR2b, wherein the antibody or antigen-binding fragment thereof comprises:
a HCDR1 having the amino acid sequence as shown in SEQ ID NO: 1, 9, 17 or 25, or having 1, 2 or 3 amino acid differences from the amino acid sequence as shown in SEQ ID NO: 1, 9, 17 or 25;
a HCDR2 having the amino acid sequence as shown in SEQ ID NO: 45, 2, 10, 18, 26, 33, 43, or 44, or having 1, 2 or 3 amino acid differences from the amino acid sequence as shown in SEQ ID NO: 45, 2, 10, 18, 26, 33, 43, or 44;
a HCDR3 having the amino acid sequence as shown in SEQ ID NO: 27, 3, 11, or 19, or having 1, 2 or 3 amino acid differences from the amino acid sequence as shown in SEQ ID NO: 27, 3, 11, or 19;
a LCDR1 having the amino acid sequence as shown in SEQ ID NO: 51, 5, 13, 21, 29, 37, or 38, or having 1, 2 or 3 amino acid differences from the amino acid sequence as shown in SEQ ID NO: 51, 5, 13, 21, 29, 37, or 38;
a LCDR2 having the amino acid sequence as shown in SEQ ID NO: 53, 6, 14, 22, 30, 39, or 52, or having 1, 2 or 3 amino acid differences from the amino acid sequence as shown in SEQ ID NO: 53, 6, 14, 22, 30, 39, or 52;
a LCDR3 having the amino acid sequence as shown in SEQ ID NO: 31, 7, 15, or 23, or having 1, 2 or 3 amino acid differences from the amino acid sequence as shown in SEQ ID NO: 31, 7, 15, or 23.

2. The antibody or antigen-binding fragment thereof according to claim 1, wherein the antibody or antigen-binding fragment thereof comprises a heavy chain variable region and/or a light chain variable region:
the heavy chain variable region comprises:
HCDR1, HCDR2 and HCDR3 having amino acid sequences as shown in SEQ ID NO: 25, SEQ ID NO: 45 and SEQ ID NO: 27, respectively; or HCDR1, HCDR2 and HCDR3 having 1, 2 or 3 amino acid differences from the amino acid sequences as shown in SEQ ID NO: 25, SEQ ID NO: 45 and SEQ ID NO: 27; or
HCDR1, HCDR2 and HCDR3 having amino acid sequences as shown in SEQ ID NO: 1, SEQ ID NO: 2 and SEQ ID NO: 3, respectively; or HCDR1, HCDR2 and HCDR3 having 1, 2 or 3 amino acid differences from the amino acid sequences as shown in SEQ ID NO: 1, SEQ ID NO: 2 and SEQ ID NO: 3; or
HCDR1, HCDR2 and HCDR3 having amino acid sequences as shown in SEQ ID NO: 9, SEQ ID NO: 10 and SEQ ID NO: 11, respectively; or HCDR1, HCDR2 and HCDR3 having 1, 2 or 3 amino acid differences from the amino acid sequences as shown in SEQ ID NO: 9, SEQ ID NO: 10 and SEQ ID NO: 11; or
HCDR1, HCDR2 and HCDR3 having amino acid sequences as shown in SEQ ID NO: 17, SEQ ID NO: 18 and SEQ ID NO: 19, respectively; or HCDR1, HCDR2 and HCDR3 having 1, 2 or 3 amino acid differences from the amino acid sequences as shown in SEQ ID NO: 17, SEQ ID NO: 18 and SEQ ID NO: 19; or
HCDR1, HCDR2 and HCDR3 having amino acid sequences as shown in SEQ ID NO: 25, SEQ ID NO: 26 and SEQ ID NO: 27, respectively; or HCDR1, HCDR2 and HCDR3 having 1, 2 or 3 amino acid differences from the amino acid sequences as shown in SEQ ID NO: 25, SEQ ID NO: 26 and SEQ ID NO: 27; or
HCDR1, HCDR2 and HCDR3 having amino acid sequences as shown in SEQ ID NO: 17, SEQ ID NO: 33 and SEQ ID NO: 19, respectively; or HCDR1, HCDR2 and HCDR3 having 1, 2 or 3 amino acid differences from the amino acid sequences as shown in SEQ ID NO: 17, SEQ ID NO: 33 and SEQ ID NO: 19; or
HCDR1, HCDR2 and HCDR3 having amino acid sequences as shown in SEQ ID NO: 25, SEQ ID NO: 43 and SEQ ID NO: 27, respectively; or HCDR1, HCDR2 and HCDR3 having 1, 2 or 3 amino acid differences from the amino acid sequences as shown in SEQ ID NO: 25, SEQ ID NO: 43 and SEQ ID NO: 27; or
HCDR1, HCDR2 and HCDR3 having amino acid sequences as shown in SEQ ID NO: 25, SEQ ID NO: 44 and SEQ ID NO: 27, respectively; or HCDR1, HCDR2 and HCDR3 having 1, 2 or 3 amino acid differences from the amino acid sequences as shown in SEQ ID NO: 25, SEQ ID NO: 44 and SEQ ID NO: 27;
the light chain variable region comprises:
LCDR1, LCDR2 and LCDR3 having amino acid sequences as shown in SEQ ID NO: 51, SEQ ID NO: 53 and SEQ ID NO: 31, respectively; or LCDR1, LCDR2 and LCDR3 having 1, 2 or 3 amino acid differences from the amino acid sequences as shown in SEQ ID NO: 51, SEQ ID NO: 53 and SEQ ID NO: 31; or
LCDR1, LCDR2 and LCDR3 having amino acid sequences as shown in SEQ ID NO: 5, SEQ ID NO: 6 and SEQ ID NO: 7, respectively; or LCDR1, LCDR2 and LCDR3 having 1, 2 or 3 amino acid differences from the amino acid sequences as shown in SEQ ID NO: 5, SEQ ID NO: 6 and SEQ ID NO: 7; or
LCDR1, LCDR2 and LCDR3 having amino acid sequences as shown in SEQ ID NO: 13, SEQ ID NO: 14 and SEQ ID NO: 15, respectively; or LCDR1, LCDR2 and LCDR3 having 1, 2 or 3 amino acid differences from the amino acid sequences as shown in SEQ ID NO: 13, SEQ ID NO: 14 and SEQ ID NO: 15; or
LCDR1, LCDR2 and LCDR3 having amino acid sequences as shown in SEQ ID NO: 21, SEQ ID NO: 22 and SEQ ID NO: 23, respectively; or LCDR1, LCDR2 and LCDR3 having 1, 2 or 3 amino acid differences from the amino acid sequences as shown in SEQ ID NO: 21, SEQ ID NO: 22 and SEQ ID NO: 23; or
LCDR1, LCDR2 and LCDR3 having amino acid sequences as shown in SEQ ID NO: 29, SEQ ID NO: 30 and SEQ ID NO: 31, respectively; or LCDR1, LCDR2 and LCDR3 having 1, 2 or 3 amino acid differences from the amino acid sequences as shown in SEQ ID NO: 29, SEQ ID NO: 30 and SEQ ID NO: 31; or
LCDR1, LCDR2 and LCDR3 having amino acid sequences as shown in SEQ ID NO: 21, SEQ ID NO: 39 and SEQ ID NO: 23, respectively; or LCDR1, LCDR2 and LCDR3 having 1, 2 or 3 amino acid differences from the amino acid sequences as shown in SEQ ID NO: 21, SEQ ID NO: 39 and SEQ ID NO: 23; or
LCDR1, LCDR2 and LCDR3 having amino acid sequences as shown in SEQ ID NO: 37, SEQ ID NO: 39 and SEQ ID NO: 23, respectively; or LCDR1, LCDR2 and LCDR3 having 1, 2 or 3 amino acid differences from the amino acid sequences as shown in SEQ ID NO: 37, SEQ ID NO: 39 and SEQ ID NO: 23; or
LCDR1, LCDR2 and LCDR3 having amino acid sequences as shown in SEQ ID NO: 38, SEQ ID NO: 39 and SEQ ID NO: 23, respectively; or LCDR1, LCDR2 and LCDR3 having 1, 2 or 3 amino acid differences from the amino acid sequences as shown in SEQ ID NO: 38, SEQ ID NO: 39 and SEQ ID NO: 23; or
LCDR1, LCDR2 and LCDR3 having amino acid sequences as shown in SEQ ID NO: 51, SEQ ID NO: 52 and SEQ ID NO: 31, respectively; or LCDR1, LCDR2 and LCDR3 having 1, 2 or 3 amino acid differences from the amino acid sequences as shown in SEQ ID NO: 51, SEQ ID NO: 52 and SEQ ID NO: 31; or
preferably, the antibody or antigen-binding fragment thereof comprises:
a heavy chain variable region comprising HCDR1, HCDR2, and HCDR3 as shown in SEQ ID NO: 25, SEQ ID NO: 45, and SEQ ID NO: 27, respectively; and a light chain variable region comprising LCDR1, LCDR2, and LCDR3 as shown in SEQ ID NO: 51, SEQ ID NO: 53, and SEQ ID NO: 31, respectively; or
a heavy chain variable region comprising HCDR1, HCDR2, and HCDR3 as shown in SEQ ID NO: 1, SEQ ID NO: 2, and SEQ ID NO: 3, respectively; and a light chain variable region comprising LCDR1, LCDR2, and LCDR3 as shown in SEQ ID NO: 5, SEQ ID NO: 6, and SEQ ID NO: 7, respectively; or
a heavy chain variable region comprising HCDR1, HCDR2, and HCDR3 as shown in SEQ ID NO: 9, SEQ ID NO: 10, and SEQ ID NO: 11, respectively; and a light chain variable region comprising LCDR1, LCDR2, and LCDR3 as shown in SEQ ID NO: 13, SEQ ID NO: 14, and SEQ ID NO: 15, respectively; or
a heavy chain variable region comprising HCDR1, HCDR2, and HCDR3 as shown in SEQ ID NO: 17, SEQ ID NO: 18, and SEQ ID NO: 19, respectively; and a light chain variable region comprising LCDR1, LCDR2, and LCDR3 as shown in SEQ ID NO: 21, SEQ ID NO: 22, and SEQ ID NO: 23, respectively; or
a heavy chain variable region comprising HCDR1, HCDR2, and HCDR3 as shown in SEQ ID NO: 25, SEQ ID NO: 26, and SEQ ID NO: 27, respectively; and a light chain variable region comprising LCDR1, LCDR2, and LCDR3 as shown in SEQ ID NO: 29, SEQ ID NO: 30, and SEQ ID NO: 31, respectively; or
a heavy chain variable region comprising HCDR1, HCDR2, and HCDR3 as shown in SEQ ID NO: 17, SEQ ID NO: 33, and SEQ ID NO: 19, respectively; and a light chain variable region comprising LCDR1, LCDR2, and LCDR3 as shown in SEQ ID NO: 37, SEQ ID NO: 39, and SEQ ID NO: 23, respectively; or
a heavy chain variable region comprising HCDR1, HCDR2, and HCDR3 as shown in SEQ ID NO: 17, SEQ ID NO: 18, and SEQ ID NO: 19, respectively; and a light chain variable region comprising LCDR1, LCDR2, and LCDR3 as shown in SEQ ID NO: 37, SEQ ID NO: 39, and SEQ ID NO: 23, respectively; or
a heavy chain variable region comprising HCDR1, HCDR2, and HCDR3 as shown in SEQ ID NO: 17, SEQ ID NO: 33, and SEQ ID NO: 19, respectively; and a light chain variable region comprising LCDR1, LCDR2, and LCDR3 as shown in SEQ ID NO: 38, SEQ ID NO: 39, and SEQ ID NO: 23, respectively; or
a heavy chain variable region comprising HCDR1, HCDR2, and HCDR3 as shown in SEQ ID NO: 17, SEQ ID NO: 18, and SEQ ID NO: 19, respectively; and a light chain variable region comprising LCDR1, LCDR2, and LCDR3 as shown in SEQ ID NO: 38, SEQ ID NO: 39, and SEQ ID NO: 23, respectively; or
a heavy chain variable region comprising HCDR1, HCDR2, and HCDR3 as shown in SEQ ID NO: 17, SEQ ID NO: 33, and SEQ ID NO: 19, respectively; and a light chain variable region comprising LCDR1, LCDR2, and LCDR3 as shown in SEQ ID NO: 21, SEQ ID NO: 39, and SEQ ID NO: 23, respectively; or
a heavy chain variable region comprising HCDR1, HCDR2, and HCDR3 as shown in SEQ ID NO: 17, SEQ ID NO: 18, and SEQ ID NO: 19, respectively; and a light chain variable region comprising LCDR1, LCDR2, and LCDR3 as shown in SEQ ID NO: 21, SEQ ID NO: 39, and SEQ ID NO: 23, respectively; or
a heavy chain variable region comprising HCDR1, HCDR2, and HCDR3 as shown in SEQ ID NO: 25, SEQ ID NO: 43, and SEQ ID NO: 27, respectively; and a light chain variable region comprising LCDR1, LCDR2, and LCDR3 as shown in SEQ ID NO: 51, SEQ ID NO: 52, and SEQ ID NO: 31, respectively; or
a heavy chain variable region comprising HCDR1, HCDR2, and HCDR3 as shown in SEQ ID NO: 25, SEQ ID NO: 44, and SEQ ID NO: 27, respectively; and a light chain variable region comprising LCDR1, LCDR2, and LCDR3 as shown in SEQ ID NO: 51, SEQ ID NO: 52, and SEQ ID NO: 31, respectively; or
a heavy chain variable region comprising HCDR1, HCDR2, and HCDR3 as shown in SEQ ID NO: 25, SEQ ID NO: 44, and SEQ ID NO: 27, respectively; and a light chain variable region comprising LCDR1, LCDR2, and LCDR3 as shown in SEQ ID NO: 51, SEQ ID NO: 53, and SEQ ID NO: 31, respectively; or
a heavy chain variable region comprising HCDR1, HCDR2, and HCDR3 as shown in SEQ ID NO: 25, SEQ ID NO: 45, and SEQ ID NO: 27, respectively; and a light chain variable region comprising LCDR1, LCDR2, and LCDR3 as shown in SEQ ID NO: 51, SEQ ID NO: 52, and SEQ ID NO: 31, respectively; or
a heavy chain variable region comprising HCDR1, HCDR2, and HCDR3 as shown in SEQ ID NO: 25, SEQ ID NO: 43, and SEQ ID NO: 27, respectively; and a light chain variable region comprising LCDR1, LCDR2, and LCDR3 as shown in SEQ ID NO: 51, SEQ ID NO: 53, and SEQ ID NO: 31, respectively.

3. The antibody or antigen-binding fragment thereof according to claim 1 or 2, wherein the antibody or antigen-binding fragment thereof comprises a heavy chain variable region and a light chain variable region, wherein:
the heavy chain variable region comprises an amino acid sequence as shown in SEQ ID NO: 49, 4, 12, 20, 28, 34, 35, 36, 46, 47, 48, or 50, or comprises an amino acid sequence having at least 95%, 96%, 97%, 98%, or 99% sequence identity to the amino acid sequence as shown in SEQ ID NO: 49, 4, 12, 20, 28, 34, 35, 36, 46, 47, 48, or 50;
the light chain variable region comprises an amino acid sequence as shown in SEQ ID NO: 62, 8, 16, 24, 32, 40, 41, 42, 54, 55, 56, 57, 58, 59, 60, or 61, or comprises an amino acid sequence having at least 95%, 96%, 97%, 98%, or 99% sequence identity to the amino acid sequence as shown in SEQ ID NO: 62, 8, 16, 24, 32, 40, 41, 42, 54, 55, 56, 57, 58, 59, 60, or 61;
preferably, the heavy chain variable region of the antibody or antigen-binding fragment thereof comprises an amino acid sequence having at least 95%, 96%, 97%, 98%, or 99% sequence identity to the amino acid sequence as shown in SEQ ID NO: 49, 34, 35, 36, 46, 47, 48, or 49; and the light chain variable region comprises an amino acid sequence having at least 95%, 96%, 97%, 98%, or 99% sequence identity to the amino acid sequence as shown in SEQ ID NO: 62, 40, 41, 42, 54, 55, 56, 57, 58, 58, 60, or 61;
more preferably, the heavy chain variable region of the antibody or antigen-binding fragment thereof comprises the amino acid sequence as shown in SEQ ID NO: 49, 34, 35, 36, 46, 47, 48, or 50; and the light chain variable region comprises the amino acid sequence as shown in SEQ ID NO: 62, 40, 41, 42, 54, 55, 56, 57, 58, 58, 60, or 61.

4. The antibody or antigen-binding fragment thereof according to claim 3, wherein:
the heavy chain variable region comprises the amino acid sequence as shown in SEQ ID NO:49, or an amino acid sequence has at least 95%, 96%, 97%, 98% or 99% sequence identity to the amino acid sequence as shown in SEQ ID NO:49; and the light chain variable region comprises the amino acid sequence as shown in SEQ ID NO:62, or an amino acid sequence has at least 95%, 96%, 97%, 98% or 99% sequence identity to the amino acid sequence as shown in SEQ ID NO:62; or
the heavy chain variable region comprises the amino acid sequence as shown in SEQ ID NO:4, or an amino acid sequence has at least 95%, 96%, 97%, 98% or 99% sequence identity to the amino acid sequence as shown in SEQ ID NO:4; and the light chain variable region comprises the amino acid sequence as shown in SEQ ID NO:8, or an amino acid sequence has at least 95%, 96%, 97%, 98% or 99% sequence identity to the amino acid sequence as shown in SEQ ID NO:8; or
the heavy chain variable region comprises the amino acid sequence as shown in SEQ ID NO:12, or an amino acid sequence has at least 95%, 96%, 97%, 98% or 99% sequence identity to the amino acid sequence as shown in SEQ ID NO:12; and the light chain variable region comprises the amino acid sequence as shown in SEQ ID NO:16, or an amino acid sequence has at least 95%, 96%, 97%, 98% or 99% sequence identity to the amino acid sequence as shown in SEQ ID NO:16; or
the heavy chain variable region comprises the amino acid sequence as shown in SEQ ID NO:20, or an amino acid sequence that has at least 95%, 96%, 97%, 98% or 99% sequence identity to the amino acid sequence as shown in SEQ ID NO:20; and the light chain variable region comprises the amino acid sequence as shown in SEQ ID NO:24, or an amino acid sequence that has at least 95%, 96%, 97%, 98% or 99% sequence identity to the amino acid sequence as shown in SEQ ID NO:24; or
the heavy chain variable region comprises the amino acid sequence as shown in SEQ ID NO:28, or an amino acid sequence has at least 95%, 96%, 97%, 98% or 99% sequence identity to the amino acid sequence as shown in SEQ ID NO:28; and the light chain variable region comprises the amino acid sequence as shown in SEQ ID NO:32, or an amino acid sequence has at least 95%, 96%, 97%, 98% or 99% sequence identity to the amino acid sequence as shown in SEQ ID NO:32; or
the heavy chain variable region comprises the amino acid sequence as shown in SEQ ID NO:34, or an amino acid sequence that has at least 95%, 96%, 97%, 98% or 99% sequence identity to the amino acid sequence as shown in SEQ ID NO:34; and the light chain variable region comprises the amino acid sequence as shown in SEQ ID NO: 40, 41, or 42, or an amino acid sequence that has at least 95%, 96%, 97%, 98% or 99% sequence identity to the amino acid sequence as shown in SEQ ID NO: 40, 41, or 42; or
the heavy chain variable region comprises the amino acid sequence as shown in SEQ ID NO:35, or an amino acid sequence that has at least 95%, 96%, 97%, 98% or 99% sequence identity to the amino acid sequence as shown in SEQ ID NO:35; and the light chain variable region comprises the amino acid sequence as shown in SEQ ID NO: 40, 41, or 42, or an amino acid sequence that has at least 95%, 96%, 97%, 98% or 99% sequence identity to the amino acid sequence as shown in SEQ ID NO: 40, 41, or 42; or
the heavy chain variable region comprises the amino acid sequence as shown in SEQ ID NO:36, or an amino acid sequence that has at least 95%, 96%, 97%, 98% or 99% sequence identity to the amino acid sequence as shown in SEQ ID NO:36; and the light chain variable region comprises the amino acid sequence as shown in SEQ ID NO: 40, 41, or 42, or an amino acid sequence that has at least 95%, 96%, 97%, 98% or 99% sequence identity to the amino acid sequence as shown in SEQ ID NO: 40, 41, or 42; or
the heavy chain variable region comprises the amino acid sequence as shown in SEQ ID NO:46, 47, or 48, or an amino acid sequence that has at least 95%, 96%, 97%, 98% or 99% sequence identity to the amino acid sequence as shown in SEQ ID NO:46, 47, or 48; and the light chain variable region comprises the amino acid sequence as shown in SEQ ID NO:54, or an amino acid sequence that has at least 95%, 96%, 97%, 98% or 99% sequence identity to the amino acid sequence as shown in SEQ ID NO:54; or
the heavy chain variable region comprises the amino acid sequence as shown in SEQ ID NO:46, 47, or 48, or an amino acid sequence that has at least 95%, 96%, 97%, 98% or 99% sequence identity to the amino acid sequence as shown in SEQ ID NO:46, 47, or 48; and the light chain variable region comprises the amino acid sequence as shown in SEQ ID NO:55, or an amino acid sequence that has at least 95%, 96%, 97%, 98% or 99% sequence identity to the amino acid sequence as shown in SEQ ID NO:55; or
the heavy chain variable region comprises the amino acid sequence as shown in SEQ ID NO:48 or 49, or an amino acid sequence has at least 95%, 96%, 97%, 98% or 99% sequence identity to the amino acid sequence as shown in SEQ ID NO:48 or 49; and the light chain variable region comprises the amino acid sequence as shown in SEQ ID NO:56, or an amino acid sequence has at least 95%, 96%, 97%, 98% or 99% sequence identity to the amino acid sequence as shown in SEQ ID NO:56; or
the heavy chain variable region comprises the amino acid sequence as shown in SEQ ID NO:48 or 49, or an amino acid sequence has at least 95%, 96%, 97%, 98% or 99% sequence identity to the amino acid sequence as shown in SEQ ID NO:48 or 49; and the light chain variable region comprises the amino acid sequence as shown in SEQ ID NO:57, or an amino acid sequence has at least 95%, 96%, 97%, 98% or 99% sequence identity to the amino acid sequence as shown in SEQ ID NO:57; or
the heavy chain variable region comprises the amino acid sequence as shown in SEQ ID NO:48 or 49, or an amino acid sequence has at least 95%, 96%, 97%, 98% or 99% sequence identity to the amino acid sequence as shown in SEQ ID NO:48 or 49; and the light chain variable region comprises the amino acid sequence as shown in SEQ ID NO:58, or an amino acid sequence has at least 95%, 96%, 97%, 98% or 99% sequence identity to the amino acid sequence as shown in SEQ ID NO:58; or
the heavy chain variable region comprises the amino acid sequence as shown in SEQ ID NO:49 or 50, or an amino acid sequence has at least 95%, 96%, 97%, 98% or 99% sequence identity to the amino acid sequence as shown in SEQ ID NO:49 or 50; and the light chain variable region comprises the amino acid sequence as shown in SEQ ID NO:59, or an amino acid sequence has at least 95%, 96%, 97%, 98% or 99% sequence identity to the amino acid sequence as shown in SEQ ID NO:59; or
the heavy chain variable region comprises the amino acid sequence as shown in SEQ ID NO:49 or 50, or an amino acid sequence has at least 95%, 96%, 97%, 98% or 99% sequence identity to the amino acid sequence as shown in SEQ ID NO:49 or 50; and the light chain variable region comprises the amino acid sequence as shown in SEQ ID NO:60, or an amino acid sequence has at least 95%, 96%, 97%, 98% or 99% sequence identity to the amino acid sequence as shown in SEQ ID NO:60; or
the heavy chain variable region comprises the amino acid sequence as shown in SEQ ID NO:49 or 50, or an amino acid sequence has at least 95%, 96%, 97%, 98% or 99% sequence identity to the amino acid sequence as shown in SEQ ID NO:49 or 50; and the light chain variable region comprises the amino acid sequence as shown in SEQ ID NO:61, or an amino acid sequence has at least 95%, 96%, 97%, 98% or 99% sequence identity to the amino acid sequence as shown in SEQ ID NO:61; or
the heavy chain variable region comprises the amino acid sequence as shown in SEQ ID NO:50, or an amino acid sequence that has at least 95%, 96%, 97%, 98% or 99% sequence identity to the amino acid sequence as shown in SEQ ID NO:50; and the light chain variable region comprises the amino acid sequence as shown in SEQ ID NO:62, or an amino acid sequence that has at least 95%, 96%, 97%, 98% or 99% sequence identity to the amino acid sequence as shown in SEQ ID NO:62.

5. The antibody or antigen-binding fragment thereof according to claim 4, wherein the antibody or antigen-binding fragment thereof comprises:
the heavy chain variable region having the amino acid sequence shown in SEQ ID NO: 49 and the light chain variable region having the amino acid sequence shown in SEQ ID NO: 62; or
the heavy chain variable region having the amino acid sequence shown in SEQ ID NO: 34 and the light chain variable region having the amino acid sequence shown in SEQ ID NO: 40, 41 or 42; or
the heavy chain variable region having the amino acid sequence shown in SEQ ID NO: 35 and the light chain variable region having the amino acid sequence shown in SEQ ID NO: 40, 41 or 42; or
the heavy chain variable region having the amino acid sequence shown in SEQ ID NO: 36 and the light chain variable region having the amino acid sequence shown in SEQ ID NO: 40, 41 or 42; or
the heavy chain variable region having the amino acid sequence shown in SEQ ID NO: 46, 47, or 48 and the light chain variable region having the amino acid sequence shown in SEQ ID NO: 54; or
the heavy chain variable region having the amino acid sequence shown in SEQ ID NO: 46, 47, or 48 and the light chain variable region having the amino acid sequence shown in SEQ ID NO: 55; or
the heavy chain variable region having the amino acid sequence shown in SEQ ID NO: 48 or 49 and the light chain variable region having the amino acid sequence shown in SEQ ID NO: 56; or
the heavy chain variable region having the amino acid sequence shown in SEQ ID NO: 48 or 49 and the light chain variable region having the amino acid sequence shown in SEQ ID NO: 57; or
the heavy chain variable region having the amino acid sequence shown in SEQ ID NO: 48 or 49 and the light chain variable region having the amino acid sequence shown in SEQ ID NO: 58; or
the heavy chain variable region having the amino acid sequence shown in SEQ ID NO: 49 or 50 and the light chain variable region having the amino acid sequence shown in SEQ ID NO: 59; or
the heavy chain variable region having the amino acid sequence shown in SEQ ID NO: 49 or 50 and the light chain variable region having the amino acid sequence shown in SEQ ID NO: 60; or
the heavy chain variable region having the amino acid sequence shown in SEQ ID NO: 49 or 50 and the light chain variable region having the amino acid sequence shown in SEQ ID NO: 61; or
the heavy chain variable region having the amino acid sequence shown in SEQ ID NO: 50 and the light chain variable region having the amino acid sequence shown in SEQ ID NO: 62.

6. The antibody or antigen-binding fragment thereof according to claim 1, wherein the antibody comprises:
the heavy chain comprising the amino acid sequence as shown in SEQ ID NO: 63 or an amino acid sequence having at least 95%, 96%, 97%, 98%, or 99% sequence identity to the amino acid sequence as shown in SEQ ID NO: 63; and
the light chain comprising the amino acid sequence as shown in SEQ ID NO: 64, 65, or 66 or an amino acid sequence having at least 95%, 96%, 97%, 98%, or 99% sequence identity to the amino acid sequence as shown in SEQ ID NO: 64, 65, or 66;
preferably, the antibody comprises: the heavy chain comprising the amino acid sequence as shown in SEQ ID NO: 63 and the light chain comprising the amino acid sequence as shown in SEQ ID NO: 64, or the heavy chain comprising the amino acid sequence as shown in SEQ ID NO: 63 and the light chain comprising the amino acid sequence as shown in SEQ ID NO: 65, or the heavy chain comprising the amino acid sequence as shown in SEQ ID NO: 63 and the light chain comprising the amino acid sequence as shown in SEQ ID NO: 66.

7. The antibody or antigen-binding fragment thereof according to any one of claims 1 to 6, wherein the antibody is selected from a murine antibody, a chimeric antibody, a humanized antibody or a fully human antibody, and the antigen-binding fragment is selected from Fab, Fab', F(ab')2, Fv, scFv or sdAb.

8. The antibody or antigen-binding fragment thereof according to any one of claims 1 to 8 , wherein the antibody or antigen-binding fragment thereof is of any IgG subtype, such as IgG1, IgG2, IgG3, or IgG4, preferably of the IgG1 subtype.

9. An isolated anti-FGFR2b antibody or antigen-binding fragment thereof having at least one of the following characteristics:
(I) it binds to an epitope on human FGFR2b protein that is the same as, completely overlapping with, or partially overlapping with the epitope bound by the antibody or antigen-binding fragment according to any one of claims 1 to 8;
(II) it competes with the antibody or antigen-binding fragment thereof according to any one of claims 1 to 8 for binding to an epitope on human FGFR2b protein.

10. A polynucleotide molecule encoding the antibody or antigen-binding fragment thereof according to any one of claims 1 to 9.

11. An expression vector comprising the polynucleotide molecule of claim 10; preferably, the vector is a eukaryotic expression vector.

12. A host cell comprising the polynucleotide molecule of claim 10 or the expression vector of claim 11; preferably, the host cell is a eukaryotic cell, more preferably a mammalian cell.

13. A method for preparing the anti-FGFR2b antibody or antigen-binding fragment thereof according to any one of claims 1-9, wherein the method comprises expressing the antibody or antigen-binding fragment thereof in the host cell of claim 12 under conditions suitable for the expression of the antibody or antigen-binding fragment thereof, and recovering the expressed antibody or antigen-binding fragment thereof from the host cell.

14. An immunoconjugate comprising the antibody or antigen-binding fragment thereof according to any one of claims 1 to 9 and a conjugation moiety.

15. The immunoconjugate according to claim 14, wherein the conjugation moiety is selected from: an anti-tumor agent, an immunomodulator, a cytotoxic drug, a fluorescent substance, a luminescent substance, an enzyme, a small nucleic acid molecule, and any combination thereof; preferably, the small nucleic acid is siRNA or an antisense oligonucleotide (ASO).

16. The immunoconjugate according to claim 14, wherein the immunoconjugate is an antibody-drug conjugate having the structure Ab-(L-D)m, wherein Ab is the FGFR2b-binding antibody or antigen-binding fragment thereof according to any one of claims 1-9; L is a linker; D is a therapeutically active substance or active pharmaceutical ingredient; m represents the average number of L-D units conjugated to Ab, and m ranges from 1 to 8, preferably m is 4.

17. The antibody-drug conjugate according to claim 16, wherein the structure of L is shown in formula (A): 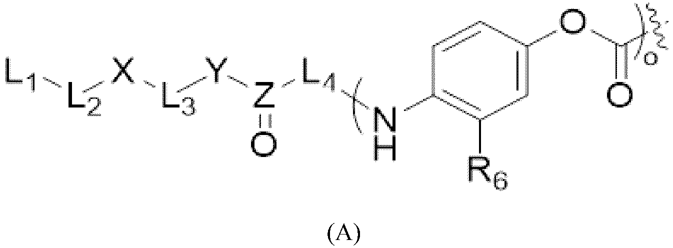 wherein:
L₁ represents a group or bond formed after a functional group capable of reacting with a thiol group reacts with a thiol group;
L₂ and Y are each independently selected from -(CH₂)₂p-, -(OCH₂CH₂)ₚ-, and -(CH₂CH₂O)ₚ-;
L₃ is a hydrophilic group;
L₄ is an amino acid group;
R₆ is selected from H, C₁-C₆ alkoxy, -(OCH₂CH₂)ₚO-C₁-C₆ alkyl, and -(CH₂CH₂O)ₚ-C₁-C₆ alkyl;
X is selected from -NH-, -NH(CH₂)ₙC(=O)-, -C(=O)(CH₂)ₙNH-, and -(CH₂)ₙC(=O)-;
Z is selected from C and S;
o is selected from 0 and 1;
n is selected from 0, 1 and 2;
p is an integer selected from 1 to 10.

18. The immunoconjugate according to claim 17, wherein the functional group capable of reacting with a thiol group in L₁ is selected from maleimide, halogen, halogen-substituted functional groups (such as halogen-substituted aldehyde groups, halogen-substituted -S(O)₂ -), aldehyde groups, alkenyl groups, alkynyl groups, alkanoyl groups, sulfonyl groups, silane groups, isocyanate groups and norbornenyl groups;
preferably, the functional group capable of reacting with a thiol group for L₁ is selected from: 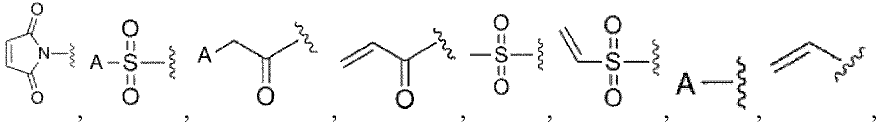 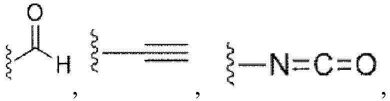 and their derivatives;
wherein: A is halogen;
the wavy lines indicate the position where L₁ connects to L₂;
preferably, the functional group described in L₁ is:

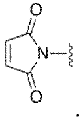

19. The immunoconjugate according to any one of claims 17 to 18, wherein:
L₂ is -(CH₂)ₚ-, and p is an integer from 1 to 4; preferably, L₂ is -CH₂CH₂- or -CH₂CH₂CH₂-; and/or
X is -NH- or -NH(CH₂)ₙC(=O)-; preferably, X is -NH- or -NHC(=O)-; and/or
Y is -(CH₂)ₚ-, with p being 1, 2, or 3; preferably, Y is methylene; and/or
Z is C.

20. The immunoconjugate according to any one of claims 17 to 19, wherein L₃ is a divalent hydrophilic group with two monovalent radical centers generated by removing two hydrogen atoms from a compound selected from a monosaccharide, a disaccharide, a five- or six-membered saturated heterocyclic ring containing 1-2 hydrogen atoms and a derivative thereof;
preferably, the monosaccharide is selected from trioses, tetroses, pentoses, hexoses, and heptoses;
preferably, the disaccharide is selected from maltose, sucrose, and lactose;
preferably, the five-membered or six-membered saturated heterocycle containing 1-2 nitrogen atoms or a derivative thereof is selected from piperazinyl, piperidinyl, and pyrrolidinyl;
preferably, L₃ is a divalent hydrophilic group with two monovalent radical centers generated by removing two hydrogen atoms from a compound selected from glucose, galactose, mannose, glucuronic acid, galactonic acid, mannuronic acid, N-acetylglucosamine, N-acetylgalactosamine, N-acetylmannosamine and N-acetylmuramic acid;
more preferably, L₃ is a structure represented by formula L₃-1: 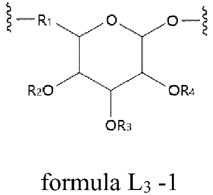
wherein:
R₁ is selected from -O(CH₂)ₚ- and -C(=O)-, p is an integer from 1 to 6, preferably 1, 2, 3 or 4;
R₂, R₃ and R₄ are independently selected from H, C₁-C₆ alkyl, -(CH₂CH₂O)ₜ-C₁-C₆ alkyl, sulfonic acid group and phosphoric acid group;
t is an integer from 1 to 20;
the wavy lines indicate the position where L₃ connects to X and Y.

21. The immunoconjugate according to any one of claims 17 to 20, wherein:
L₄ is selected from the following amino acid residues: valine, citrulline, alanine, glycine, phenylalanine, asparagine, glutamic acid, lysine, serine, threonine, cysteine and tyrosine; the amino acid group is optionally substituted by one or more -OR₂₀, wherein R₂₀ is selected from H, C₁-C₆ alkyl, -(CH₂CH₂O)ₜ-C₁-C₆ alkyl, sulfonic acid group and phosphate group, wherein t is an integer from 1 to 20; or
L₄ is selected from the following peptide segments: valine-citrulline (VC), valine-alanine (VA), glycine-glycine-phenylalanine-glycine (GGFG), glycine-glycine-phenylalanine-glycine-glycine, glycine-glycine-phenylalanine-glycine-glycine-glycine, citrulline-valine, alanine-valine, alanine-alanine, citrulline-alanine, asparagine-citrulline, citrulline-asparagine, citrulline-citrulline, phenylalanine-lysine and lysine-phenylalanine, preferably selected from the following peptide segments: valine-citrulline (VC), valine-alanine (VA) and glycine-Glycine-phenylalanine-glycine (GGFG); each peptide segment is optionally substituted by one or more substituents selected from the following group: -OR₂₀ and -(OCH₂CH₂)ₚO-C₁-C₆ alkyl, wherein R₂₀ is selected from H, C₁-C₆ alkyl, -(CH₂CH₂O)ₜ-C₁-C₆ alkyl, sulfonic acid group and phosphate group, t is an integer from 1 to 20, and p is an integer from 1 to 6.

22. The immunoconjugate according to claim 21, wherein L4 is selected from: 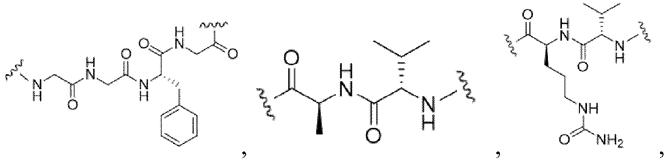 wherein the wavy line indicates the position of attachment of L4 to Z and NH.

23. The immunoconjugate according to any one of claims 14 to 22, wherein R₆ is H, C1-C3 alkoxy, or -(OCH₂CH₂)ₚO-C1-C3 alkyl, wherein p is an integer from 1 to 6; preferably, R₆ is H, methoxy, or -(OCH₂CH₂)ₚOCH₃, wherein p is an integer from 1 to 6 or 1 to 4.

24. The immunoconjugate according to claim 16, wherein the structure of the linker L is as shown in any one of the following structures: 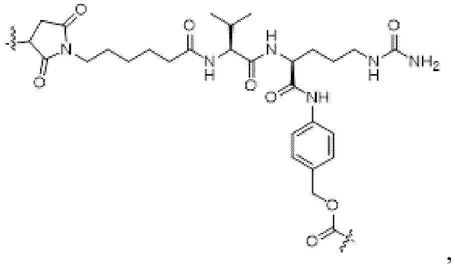 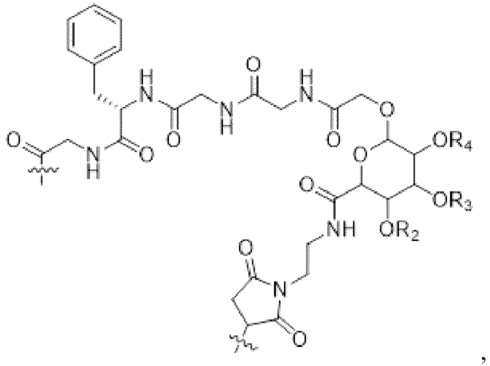 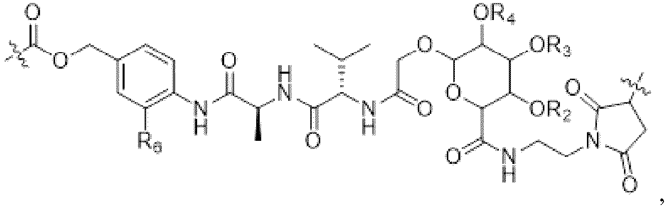 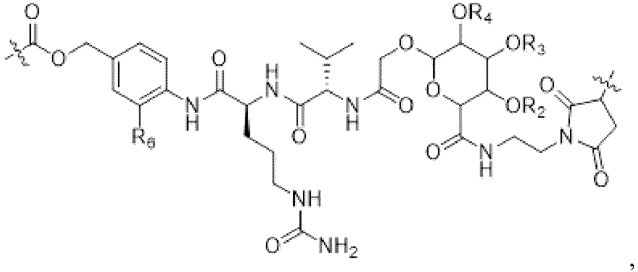 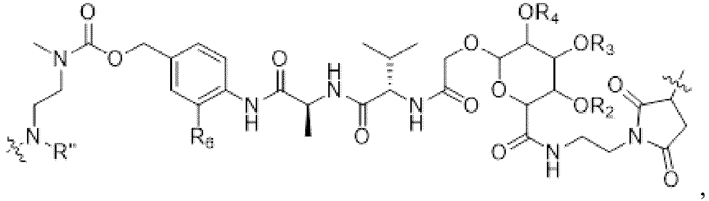 in each formula, R₂, R₃, and R₄ are as defined in claim 20, R₆ is as defined in claim 17 or 22, and R" is H or C1-C6 alkyl.

25. The immunoconjugate according to any one of claims 14 to 24, wherein the drug moiety D comprises a microtubule inhibitor, a DNA damaging agent, a vitamin A precursor, or folic acid;
preferably, the microtubule inhibitors include dolastatins and auristatins, and maytansines; the DNA damaging agents include calicheamicins, duocarmycins, the anthramycin derivative PBD (pyrrolobenzodiazepine), and topoisomerase I inhibitors;
more preferably, the auristatin drugs include monomethyl auristatin E (MMAE), monomethyl auristatin F (MMAF), and auristatin F (AF) or derivatives thereof; the maytansine drugs include but are not limited to DM1, DM3, DM4 or derivatives thereof; the topoisomerase I inhibitors include but are not limited to exatecan, topotecan, irinotecan, 9-nitrocamptothecin, and the camptothecin derivative SN-38.

26. The immunoconjugate according to any one of claims 14 to 25, wherein the linker-drug (L-D) moiety is a structure represented by the following formulas of VCMMAE, CPD2, CPD4, CPD5, CPD6, or SN-38Comp5:

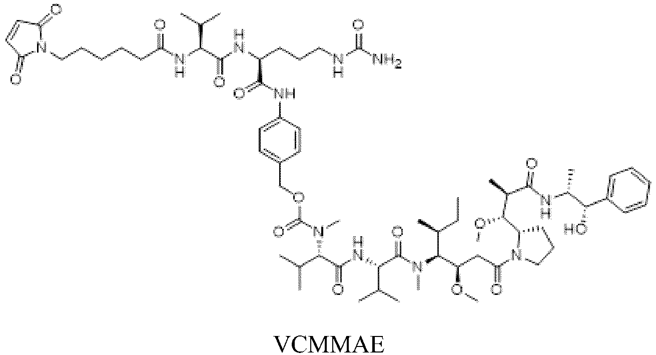

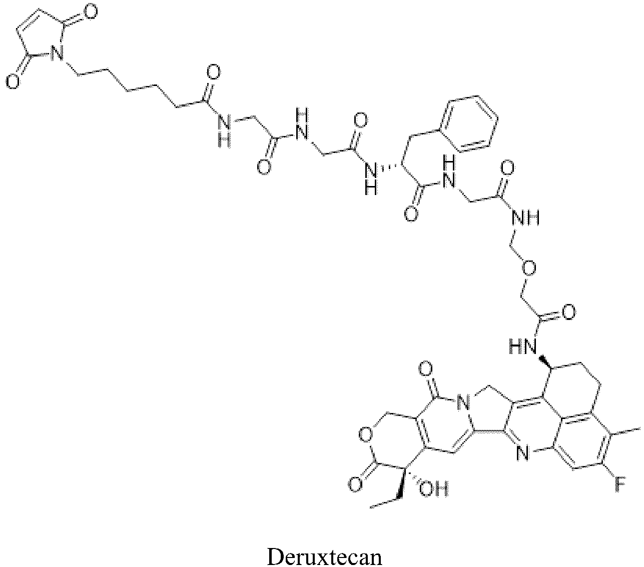

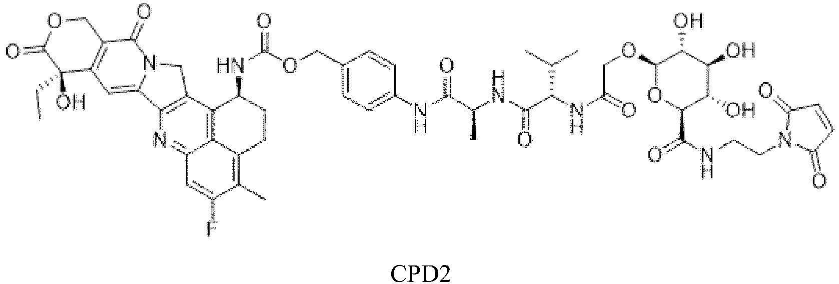

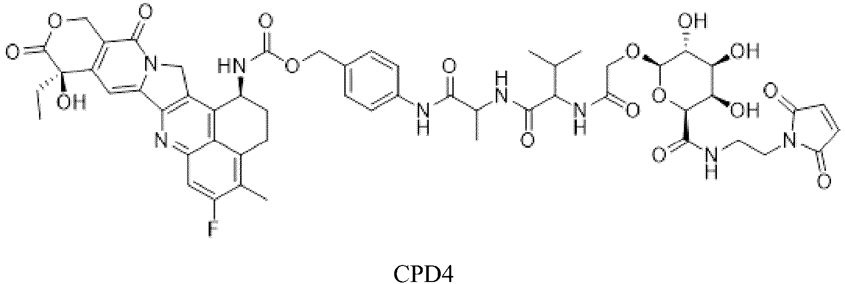

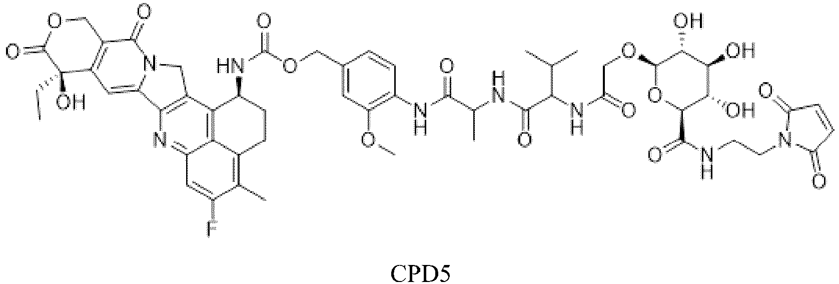

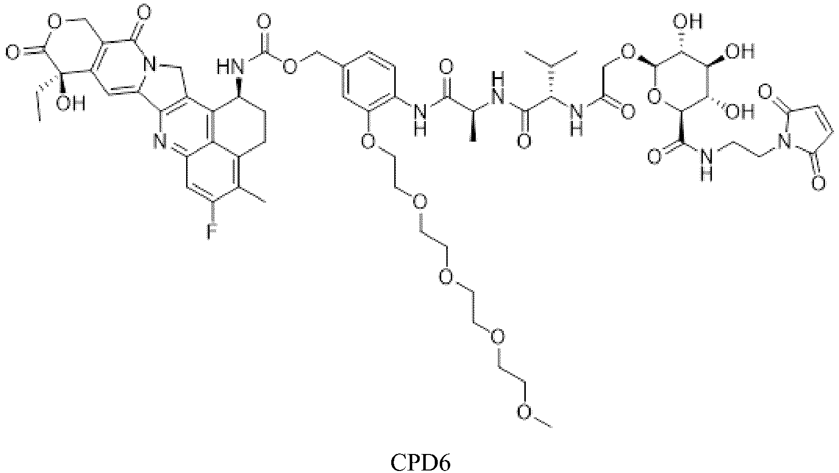

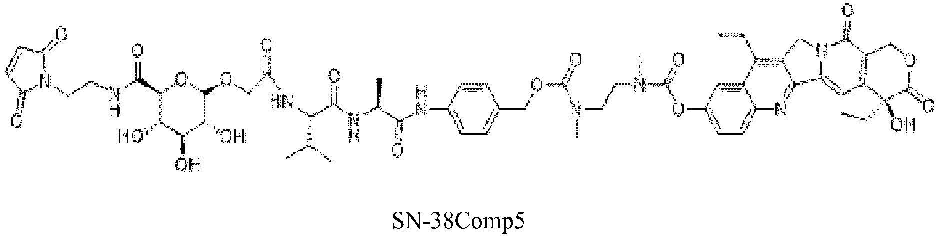

27. The immunoconjugate according to any one of claims 14 to 26, wherein the immunoconjugate is selected from the structures represented by formulas of ADC-I, ADC-II, ADC-III, ADC-IV, ADC-V, ADC-VI, or ADC-VII: 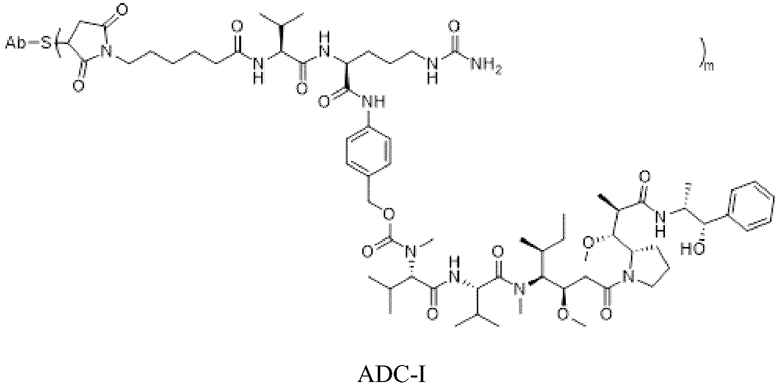 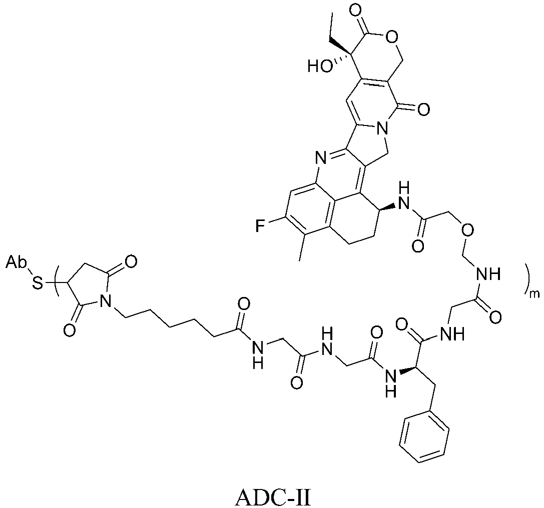 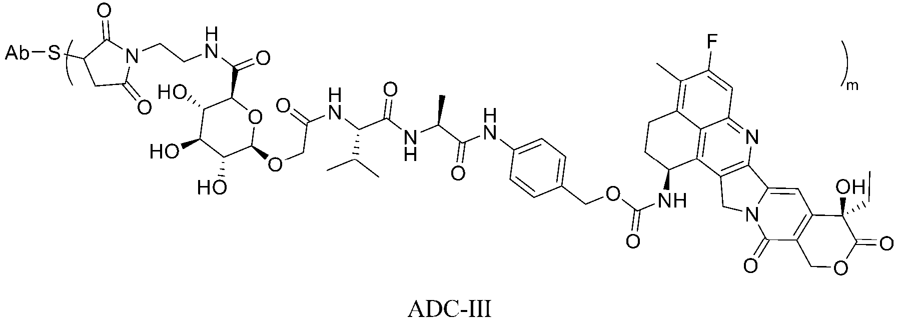 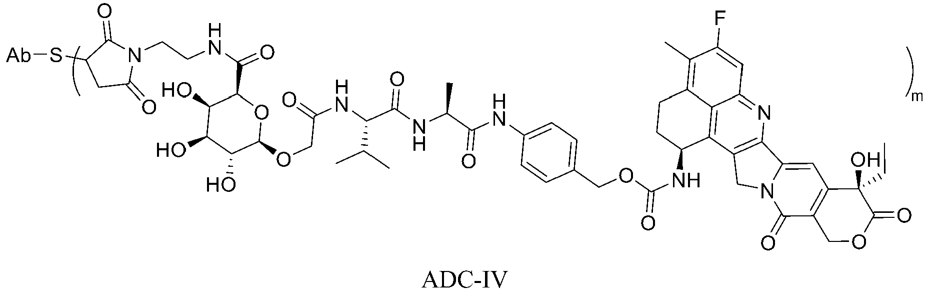 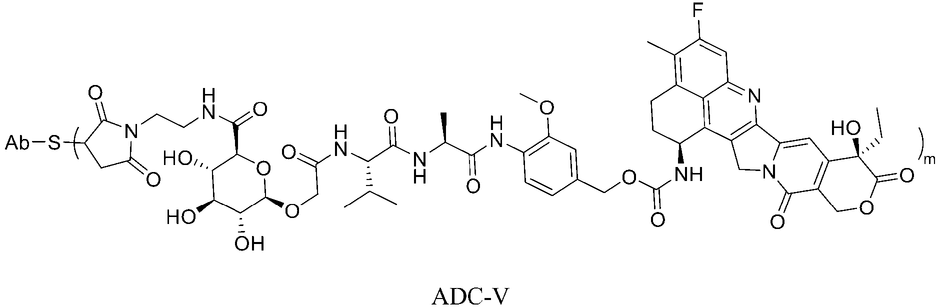 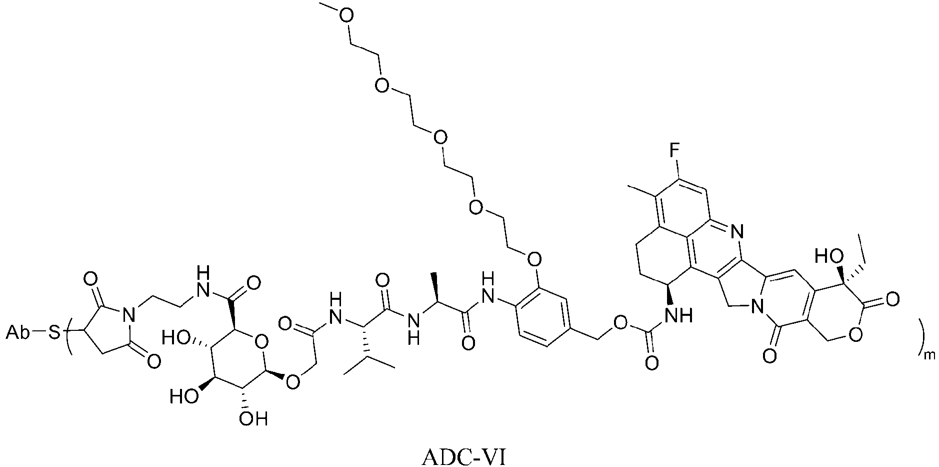 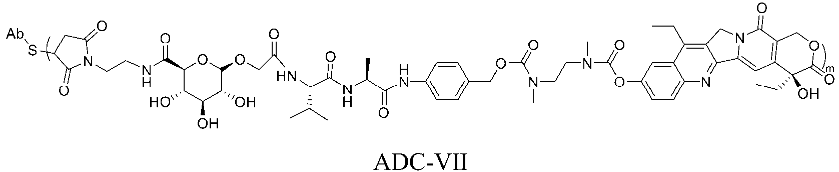 wherein m represents the average number of linker-drug (L-D) units conjugated to Ab, and m ranges from 1 to 8, preferably m is 4.

28. A pharmaceutical composition comprising the antibody or antigen-binding fragment thereof according to any one of claims 1 to 9, the polynucleotide molecule of claim 10, the expression vector of claim 11, the host cell of claim 12, or the immunoconjugate according to any one of claims 14 to 27, and a pharmaceutically acceptable carrier or excipient.

29. Use of the antibody or antigen-binding fragment thereof according to any one of claims 1 to 9, the polynucleotide molecule of claim 10, the expression vector of claim 11, the host cell of claim 12, the immunoconjugate according to any one of claims 14 to 27, or the pharmaceutical composition of claim 28 in the manufacture of a medicament for treating and/or preventing an FGFR2b-mediated disease or disorder, preferably the disease or disorder is cancer; more preferably, the cancer is selected from endometrial cancer, ovarian cancer, breast cancer, lung cancer, gastric cancer, esophageal cancer, bladder cancer, and bile duct cancer.

30. An article comprising a first container, the first container containing a composition comprising the antibody or antigen-binding fragment thereof according to any one of claims 1 to 9, the polynucleotide molecule of claim 10, the expression vector of claim 11, the host cell of claim 12, the immunoconjugate according to any one of claims 14 to 27, or the pharmaceutical composition of claim 28.

31. The article according to claim 31, wherein:
the article further comprises a second container containing a pharmaceutically acceptable buffer; or
the article further comprises a container containing a diluent, buffer, or control antibody for detection purposes.

32. A method for detecting the presence of FGFR2b in a sample using the antibody or antigen-binding fragment thereof according to any one of claims 1 to 9 or a detection composition comprising the antibody or antigen-binding fragment thereof, comprising the step of contacting the antibody or antigen-binding fragment thereof or the detection composition comprising the antibody or antigen-binding fragment thereof with the sample.
